# EUROPEAN PATENT SPECIFICATION

(11) **EP 4 136 098 B1**
(45) Date of publication and mention of the grant of the patent: **17.12.2025**
(21) Application number: 21724444.1
(22) Date of filing: 16.04.2021
(51) Int. Cl.: C07K 14/47

(54) **PEPTIDE-RECEPTIVE MHC-I COMPLEX COMPOSITIONS AND METHODS FOR THEIR GENERATION**
ZUSAMMENSETZUNGEN VON PEPTID-EMPFÄNGLICHEN MHC-I KOMPLEXEN SOWIE METHODEN ZU DEREN HERSTELLUNG
COMPOSITIONS DE COMPLEXE MAJEUR D'HISTOCOMPATIBILITÉ (CMH-I) RÉCEPTIVE AUX PEPTIDES ET PROCÉDÉS DE LEUR PRODUCTION

(30) Priority: 16.04.2020 US 202063011221 P; 02.07.2020 US 202063047812 P; 10.09.2020 US 202063076601 P
(43) Date of publication of application: 22.02.2023
(73) Proprietor: The Regents of the University of California, Oakland, CA 94607 (US)
(72) Inventor: SGOURAKIS, Nikolaos G., Philadelphia, PA 19104-4318 (US)
(74) Representative: Grund, Martin
(86) International application number: PCT/US2021/027841
(87) International publication number: WO 2021/212085

(56) References cited:
- WO-A1-2020/010261
- CLEMENS HERMANN ET AL: "TAPBPR alters MHC class I peptide presentation by functioning as a peptide exchange catalyst", ELIFE, vol. 4, 6 October 2015 (2015-10-06), XP055567332, DOI: 10.7554/eLife.09617
- SARA M O'ROURKE ET AL: "Production of soluble pMHC-I molecules in mammalian cells using the molecular chaperone TAPBPR", PROTEIN ENGINEERING, DESIGN AND SELECTION, vol. 32, no. 12, 31 December 2019 (2019-12-31), GB, pages 525 - 532, XP055758393, ISSN: 1741-0126, DOI: 10.1093/protein/gzaa015
- JOLIEN J. LUIMSTRA ET AL: "A flexible MHC class I multimer loading system for large-scale detection of antigen-specific T cells", JOURNAL OF EXPERIMENTAL MEDICINE, vol. 215, no. 5, 17 April 2018 (2018-04-17), US, pages 1493 - 1504, XP055540611, ISSN: 0022-1007, DOI: 10.1084/jem.20180156
- MCSHAN ANDREW C ET AL: "Peptide exchange on MHC-I by TAPBPR is driven by a negative allostery release cycle", NATURE CHEMICAL BIOLOGY, NATURE PUB. GROUP, NEW YORK, vol. 14, no. 8, 9 July 2018 (2018-07-09), pages 811 - 820, XP036928228, ISSN: 1552-4450, [retrieved on 20180709], DOI: 10.1038/S41589-018-0096-2
- SARAH A. OVERALL ET AL: "High throughput pMHC-I tetramer library production using chaperone-mediated peptide exchange", NATURE COMMUNICATIONS, vol. 11, no. 1, 20 April 2020 (2020-04-20), XP055758394, DOI: 10.1038/s41467-020-15710-1

## Description

### BACKGROUND

Cytotoxic T cell activation occurs when a T cell, via its T cell receptor (TCR), recognizes an antigen peptide-MHC class I complex on an antigen presenting cell. Such TCR/peptide-MHC class I interactions are important, for example, for adaptive immunity against pathogens, for the recognition and elimination of tumor cells, as well as the pathogenesis of particular diseases (e.g., autoimmune diseases).

Antigen peptide-MHC class I multimer complexes are useful for the identification of antigen specific T cells, and antigens that activate such T cells. The identification of antigen specific T cells and corresponding antigens can then be used, for example, for the understanding of disease development, as well as in the development of T-cell based therapies (e.g., cancers), in both preclinical and clinical settings.

High-throughput screening strategies using large arrays of peptide-MHC class I multimers can advantageously allow for the large scale identification of antigen specific T-cells and/or antigens that bind to particular MHC class I. Such high-throughput screens, however, are limited by the ability to produce large collections of peptide-MHC class I multimers. The instability of peptide-deficient MHC class I molecules, for example, makes large scale production of peptide-MHC class I multimers using such peptide-deficient MHC class I molecules difficult to achieve.

To circumvent the problem of unstable peptide-deficient MHC class I molecules, conditional MHC class I ligands are used. *See, e.g.,* Rodenko et al., Nature Protocols 1(3): 1120-1132 (2006). Such conditional ligand bound to MHC class I can be cleaved by exposure to UV light, or to increased temperature (*See, e.g.,* Luimstra et al., J. Exp. Med, 2018). Upon cleavage and in the presence of a peptide of interest, a net exchange occurs wherein the cleaved conditional ligand dissociates with the MHC class I and the peptide of interest associates with the MHC class I, thereby forming the desired peptide-MHC class I complex. Such conditional ligands, however, also have limitations. Conditional ligands almost always result in some sample aggregation and precipitation during the photolysis/peptide exchange step, due to the unstable nature of the transient peptide-deficient MHC. Moreover, using suboptimal peptides can lead to high background levels of exchange. As such, there remains a need for new methods of making peptide-MHC class I complex libraries.

### SUMMARY

The invention is defined by the appended claims.

Compositions that include stable peptide-deficient MHC class I/chaperone complexes and methods of making and using such complexes are provided. In particular embodiments, such peptide-deficient MHC class I/chaperone complexes are used to form peptide MHC class I (pMHC-I) multimers useful for high throughput applications, such as, for the detection of antigen specific T cells and characterization of T cell profiles in subjects.

In a first aspect, provided herein is a method of making a plurality of peptide receptive MHC-I complexes each of which can accept a peptide of interest, each peptide receptive MHC-I complex comprising an MHC class I heavy chain and an β2-microglobulin, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides have the sequence YPLFGWCF or LFGPVYV, under conditions wherein the plurality of MHC class I heavy chains, the β2-microglobulins and the plurality placeholder peptides form a plurality of placeholder peptide-MHC class I (p*MHC-I ) complexes; and
b) contacting the p*MHC-I complexes with a plurality of dipeptides and chaperones, thereby creating the plurality of peptide receptive MHC-I complexes.

In another aspect, provided herein is a method of making a plurality of peptide-MHC class I (pMHC-I) complexes, each complex comprising an MHC Class I heavy chain, a β2-microglobulin, and a peptide of interest, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides having the sequence YPLFGWCF or LFGPVYV, under conditions wherein the plurality of MHC class I heavy chains, the plurality β2-microglobulins and the plurality placeholder peptides form a plurality of placeholder peptide-MHC class I (p*MHC-I ) complexes;
b) forming a plurality of peptide receptive MHC-I complexes by contacting the plurality of p*MHC-I complexes with a plurality of dipeptides and chaperones; and
c) contacting the plurality of peptide receptive MHC-I complexes with a plurality of peptides of interest, thereby forming the plurality of pMHC-I complexes.

In another aspect, provided herein is a method of making a plurality of peptide-MHC class I (pMHC-I) multimer complexes, each complex comprising an MHC class I multimer and a peptide of interest, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides having the sequence YPLFGWCF or LFGPVYV, under conditions wherein the plurality of MHC class I heavy chains, the β2-microglobulins and the plurality of placeholder peptides form a plurality of placeholder peptide-MHC class I (p*MHC-I ) complexes;
b) contacting the plurality of p*MHC-I complexes with a plurality of dipeptides and chaperones, thereby forming a plurality of peptide receptive MHC-I complexes;
c) attaching the plurality of peptide receptive MHC-I complexes to multimer backbones, thereby forming a plurality of peptide receptive MHC-I multimers; and
d) contacting the plurality of peptide receptive MHC- I multimers with a plurality of peptides of interest, thereby forming a plurality of pMHC-I multimer complexes.

In one aspect, provided herein is a method of making a plurality of peptide-MHC class I (pMHC-I) multimer complexes, each complex comprising an MHC class I multimer and a peptide of interest, the method comprising:
a) providing a plurality of placeholder peptide-MHC class I (p*MHC-I) complexes, wherein at least a subset of the plurality of p*MHC-I complex comprises an MHC class I heavy chain comprising one of HLA-A*24:02 and HLA-A*68:02, an β2-microglobulin, and a placeholder peptides have the sequence YPLFGWCF or LFGPVYV;
b) contacting the plurality of p*MHC-I complexes with a plurality of chaperones, dipeptides, multimer backbones, and peptides of interest under conditions to form a plurality of peptide-MHC class I multimer complexes, thereby forming a plurality of peptide-MHC class I multimer complexes; and
c) recovering the plurality of peptide-MHC class I multimer complexes.

In another aspect, provided herein is a composition comprising:
a) a plurality of MHC class I heavy chains comprising one of HLA-A*24:02 and HLA-A*68:02;
b) a plurality of β2-microglobulins;
c) a plurality of placeholder peptides comprising placeholder peptides have the sequence YPLFGWCF or LFGPVYV; and
d) a plurality of chaperones..

In one aspect, provided herein is a composition comprising a plurality of peptide receptive MHC-I complexes configured to accept a peptide of interest, wherein at least a subset of the plurality of peptide receptive MHC-I complexes comprises:
a) a plurality of MHC class I heavy chains comprising one of HLA-A*24:02 and HLA-A*68:02;
b) a plurality of β2-microglobulins; and
c) a plurality of placeholder peptides comprising placeholder peptides have the sequence YPLFGWCF or LFGPVYV.

### BRIEF DESCRIPTION OF THE DRAWINGS

High-throughput screening strategies using large arrays of peptide-MHC class I multimers can allow for the large scale identification of antigen specific T cells and/or antigens that bind to particular MHC class Is (e.g., tumor antigens). Identification of such antigen specific T cells and/or antigens can in turn lead to the understanding of the pathogenesis of particular diseases, as well as the development of novel therapies.

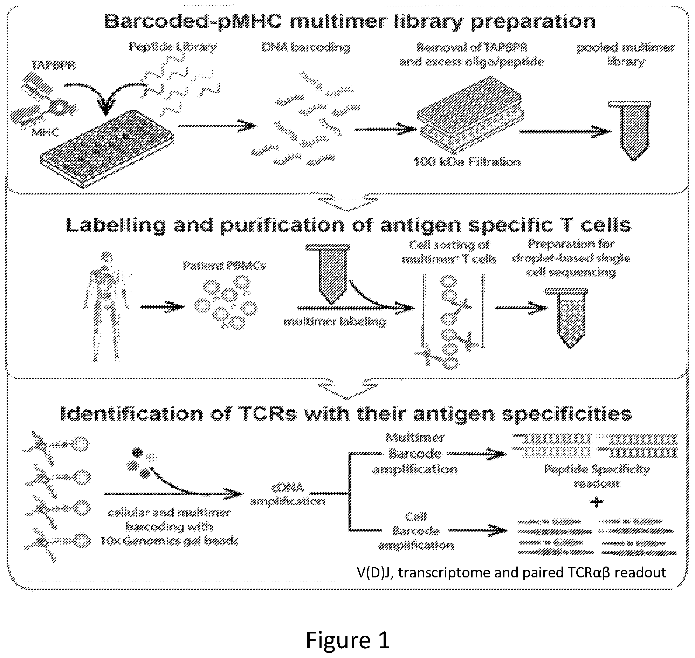

Figure 1 provides a schematic of a method for linking peptide specificities with T cell transcriptomes as described herein. Fluorophore-labeled, empty MHC-I/TAPBPR multimers are loaded with peptides of interest on a 96-well plate format and individually barcoded with DNA oligos designed for 10x Genomics and Illumina compatibility. TAPBPR and excess peptide, along with free oligo, are removed by centrifugation and the multimers are pooled together. A single patient sample can be stained with the pooled multimer library, and collected by fluorescence-activated cell sorting. Tetramer associated oligos and cellular mRNA from individual cells are then barcoded using 10x Genomics gel beads, followed by cDNA synthesis, library preparation and library sequencing. This workflow enables the transcriptome and paired αβ TCR sequences to be linked with pMHC specificities in a single experiment.

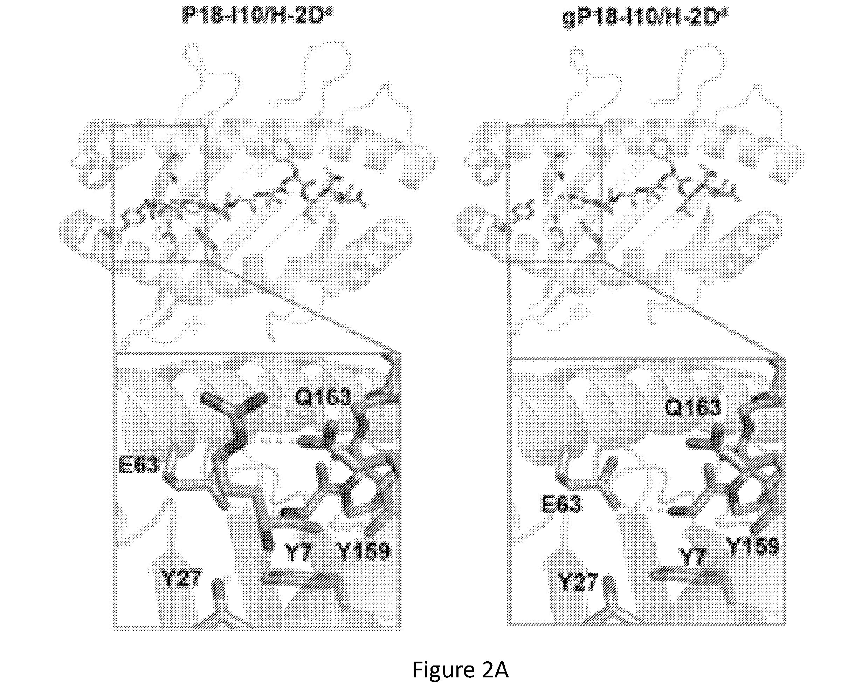

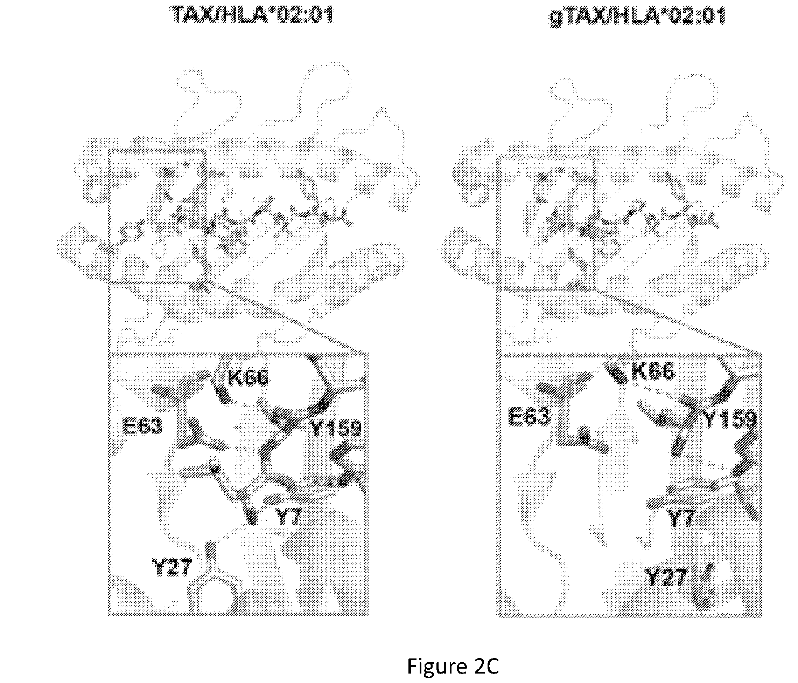

Figures 2A-2C depict the structure-guided design of placeholder peptides for murine MHC-I molecules. Crystal structures showing stabilizing contacts in the A-pocket of the MHC-I peptide-binding groove for: (A) P18-110/H-2D^{d} (PDB 3ECB¹) with the bound peptide shown in light red. (B) and (C) Structure of p29/H-2L (PDB 1LD9²), and QL9/H-2L (PDB 3TF7³), with bound peptides shown in yellow. Insets focus on the A-pocket of the MHC-I peptide binding groove, with polar contacts between the N-terminal residue of the peptide and the indicated MHC-I residues shown as dotted yellow lines. Full peptide sequences are provided, with the first residue indicated in green.

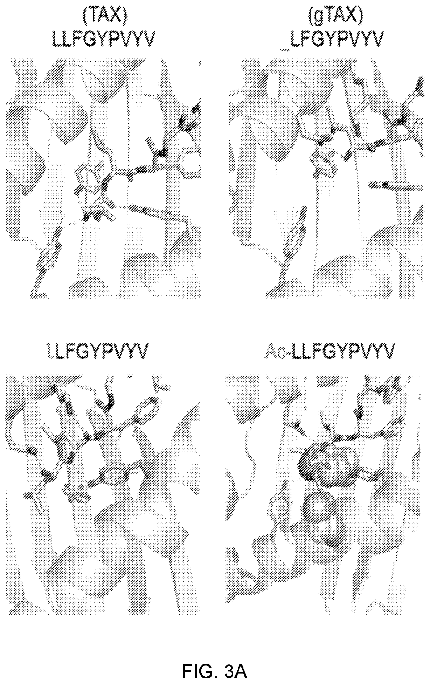

Figures 3A-3G depict the capturing empty HLA-A*02:01/TAPBPR complexes for peptide exchange. (A) Structure-based design of goldilocks peptides: comparison of polar contacts between HLA-A*02:01 and the N-terminal region of LLFGYPVYV (TAX) peptide (upper left). _LFGYPVPYV (gTAX) (upper right), Ac-LLFGYPVYV (bottom right) and ILFGYPVYV where I = D-Leucine (bottom left). Structures were modeled using PDB ID IDUZ[26]. (B) Peptide complex thermal stabilities of HLA-A*02:01 bound to TAX, ILFGYPVYV, Ac-LLFGYPVYV and gTAX. (C) SEC TAPBPR binding assays of TAX/HLA-A02:01 (left), gTAX/HLA-A02:01 (right). (D) Native gel electrophoresis of HLA-A*02:01/TAPBPR complex incubated with a 10-fold molar excess of a nonspecific peptide (p29, YPNVNIHNF) or varying molar excess of a specific, high-affinity peptide (TAX). 12% polyacrylamide native gels were run at 90 V for 5 h at 4 °C before visualization with InstantBlue (Expedeon). Data shown are representative of triplicate gel assays. (E) Competitive binding of TAMRA-TAX to purified HLA-A*02:01/TAPBPR complexes from (C) as a function of increasing peptide concentration, measured by fluorescence polarization. (F) Conceptual diagram of TAPBPR-mediated capture and peptide loading on empty MHC-I molecules. (G) Bio-Layer Interferometry analysis of TAPBPR dissociation from HLA-A*02:01 in the presence of peptides of different affinities. Data shown in (E) and (G) are representative of triplicate assays (n = 3) and error-bars are standard deviations from the mean.

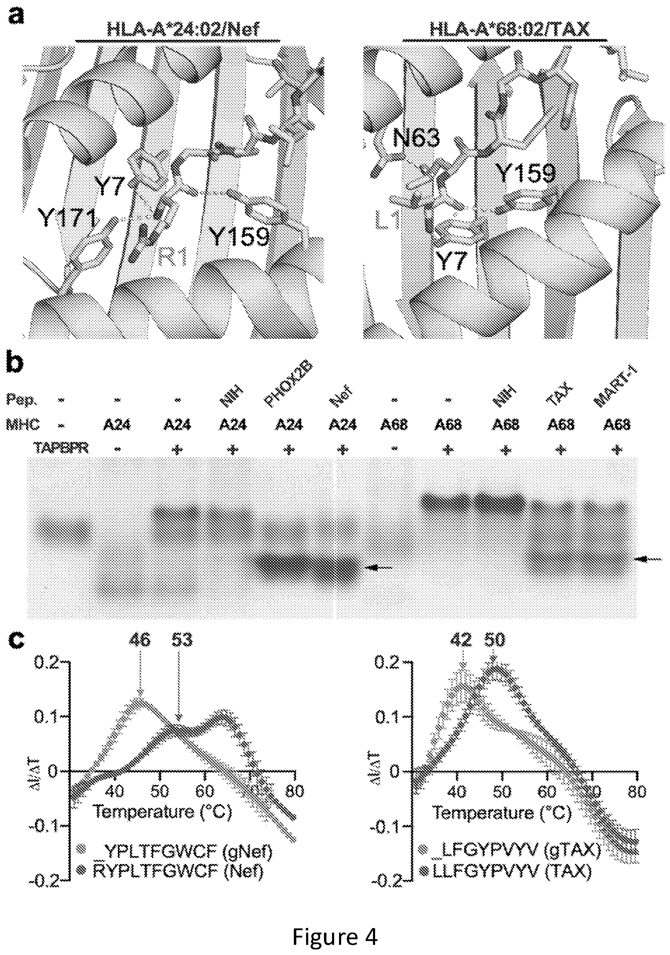

Figure 4a-4c depict TAPBPR-mediated peptide exchange on HLA-A*24:02 and HLA-A*68:02. (a) Structure-based design of goldilocks peptides: analysis of A-pocket hydrogen bonds observed in the X-ray structure of HLA-A*24:02/Nef₁₃₄₋₁₀ (RYPLTFGWCF) and homology-based model of HLA-A*28:02/TAX (LLFGYPVYV). Peptide residues are labelled in red, A-pocket MHC residues in black. The structure of HLA-A*24:02/Nef₁₃₄₋₁₀ was obtained from PDB ID 3QZW (Shi, et al., Mol Immunol 48:2198-2202 (2011)). The structure of HLA-A*68:02/TAX was modeled using a related structure (PDB ID 4HX1 (Niu et al., Mol Immunol 55:381-392 (2013)) as input. (b) Native gel electrophoresis analysis showing MHC-I/TAPBPR complex dissociation in the presence of 10-fold molar excess of relevant, high-affinity peptides PHOX2B (QYNPIRTTF), Nef₁₃₄₋₁₀ (lanes 5 and 6) and TAX, MART-1 (lanes 10 and 11) for complexes prepared using refolded HLA-A*24:02 and HLA-A*68:02 with gNEF (lane 2) and gTAX (lane 7) goldilocks peptides, respectively (protein yields of approximately 6 and 8 mg from a 1 L refolding reaction). The exchanged pMHC molecular species on lanes 5, 6, 10 and 11 are indicated with arrows, showing electrophoretic mobilities that depend on the charge of the bound peptide. Both complexes remain bound in the presence of 10-fold molar excess of the irrelevant peptide p29 (YPNVNIHNF) (lanes 4 and 9). 12% polyacrylamide native gels were run at 90 V for 5 h at 4 °C before visualization with InstantBlue (Expedeon). Data shown are representative of triplicate gel assays. All protein samples used in (a) and (b) were derived from the same peptide exchange experiment, and the gels were processed in parallel. (c) Overlaid Differential Scanning Fluorimetry temperature profiles of: goldilocks/MHC-I (red) and high-affinity peptide/MHC-I (blue) prepared using chaperone-mediated exchange of the goldilocks for high-affinity peptides, followed by purification of the pMHC peak by SEC. Thermal stabilities are shown: HLA-A*24:02 refolded with gNEF (YPLTFGWCF) or exchanged with NEF (RYPLTFGWCF) (left), and HLA-A*68:02 refolded with gTAX (_LFGYPVYV) or exchanged with TAX (LLFGYPVYV) (right). The peaks at approximately 63 °C correspond to the thermal melt of the β₂m light chain (Hellman et al., J Immunol Methods 432:95-101 (2016)). Data shown are representative of triplicate assays and error-bars are standard deviation from the mean.

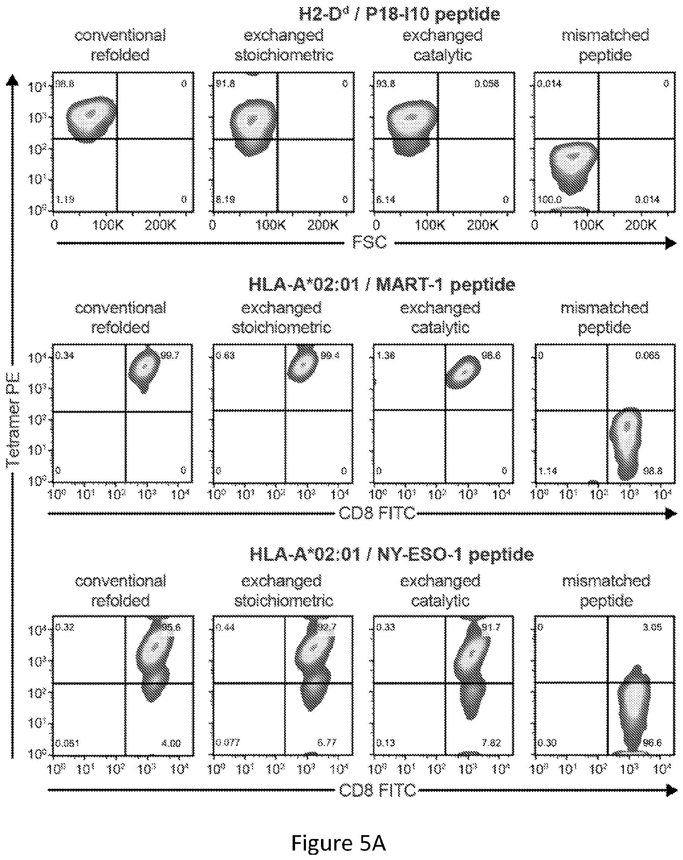

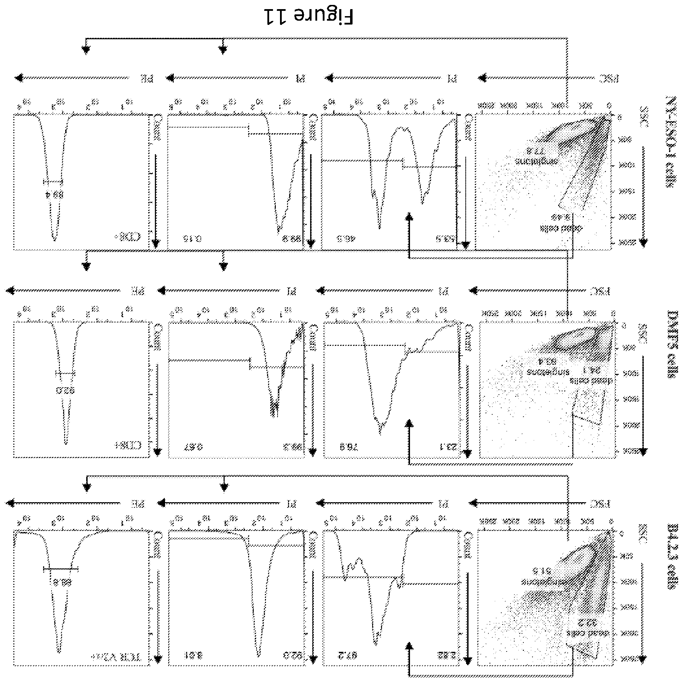

Figures 5A-5D depict flow cytometry analysis using TAPBPR-exchanged pMHC-I tetramers. (A) Representative flow cytometric analysis of top row; murine cells expressing the B4.3.2 TCR, middle row; DMF5 human T cells expressing the MART-1 TCR, bottom row; NY-ESO-1 human T cells expressing the NY-ESO-1 TCR. Columns depict staining with a fixed excess (1 µg/mL) of P18-I10/H2-D^{d} , MART-1/HLA-A*02:01 and NY-ESO-1/HLA-A*02:01 tetramers prepared either by conventional refolding, exchange of peptide on stoichiometric MHC-I/TAPBPR complexes, TAPBPR-mediated peptide exchange using a catalytic (1:100) TAPBPR to MHC-I molar ratio or by stoichiometric exchange of a mismatched peptide, not recognized by the respective TCRs. (B) Histogram plots of tetramer staining. (C) Tetramer titration of P18-110/H-2D^{d} (top), MART- 1/HLA-A*02:01(middle) and NY- ESO-1/HLA-A*02:01 (bottom), prepared by exchange of each peptide on the corresponding stoichiometric MHC-I/TAPBPR complexes. Percentage of cells staining positive with tetramer over a serial two-fold dilution series were plotted and EC₅₀ values calculated by curve fitting to a sigmoidal line (with *R²* values in the 0.97-0.99 range), using Graph Pad Prism version 8 (GraphPad Software, La Jolla California USA). Data shown are representative of triplicate assays (n=3) and error-bars are standard deviations from the mean. Gating strategies used for sorting tetramer-positive cells are outlined in Figure 11.

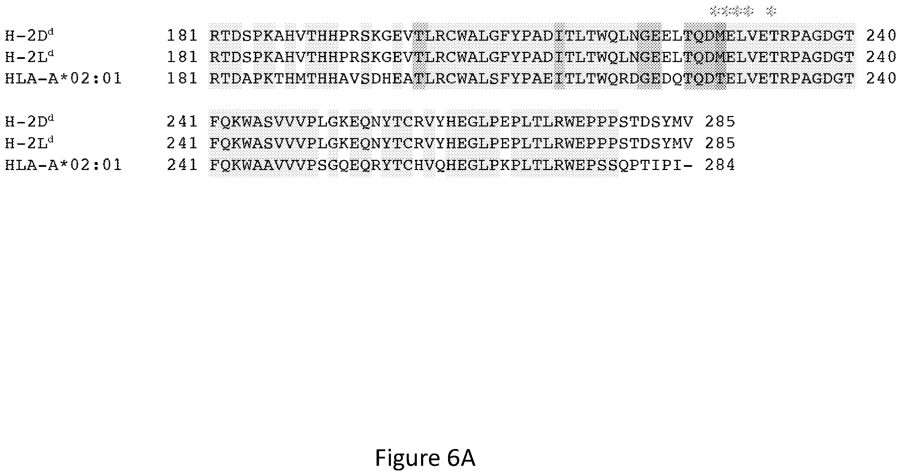

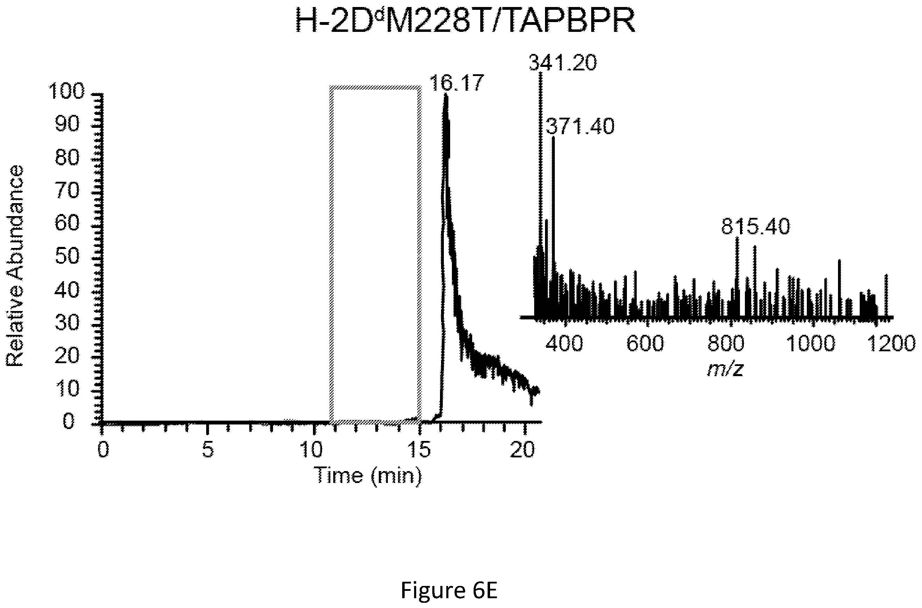

Figures 6A-6E depict the fine-tuning MHC-I/TAPBPR interactions through α3 domain mutants. (A) Alignment of the α₃ domain sequences from murine H-2D^{d}, H-2Ld and human HLA-A*02:01. Conserved residues are highlighted in yellow. The M228T mutation site is highlighted in blue. * indicates residues directly participating in TAPBPR interactions, as shown in published mutagenesis studies and crystal structures. (B) TAPBPR/H-2Dd α₃ domain interface from PDB ID 5WER[5] (left panel) and with the M228T mutation modeled (right panel). (C) Size exclusion chromatograms of H-2D^{d} M228T refolded with either high-affinity P18-I10 or goldilocks gP18-I10 peptides, with and without TAPBPR. (D) and (E) LC/MS analysis of the peptide region from SEC-purified gP 18/H-2D^{d}M228T and H-2D^{d}M228T/TAPBPR peaks from (C). Data shown are representative of triplicate SEC and LC/MS experiments.

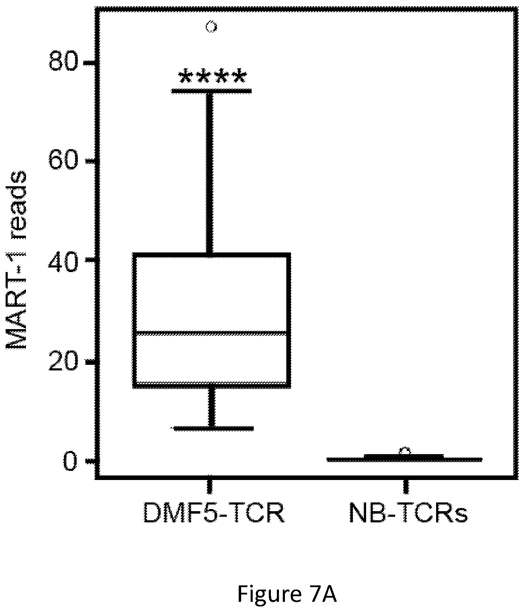

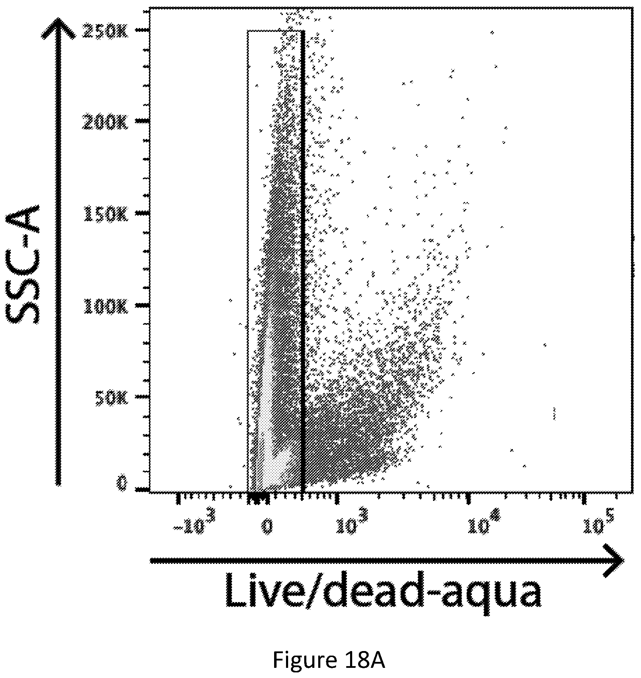

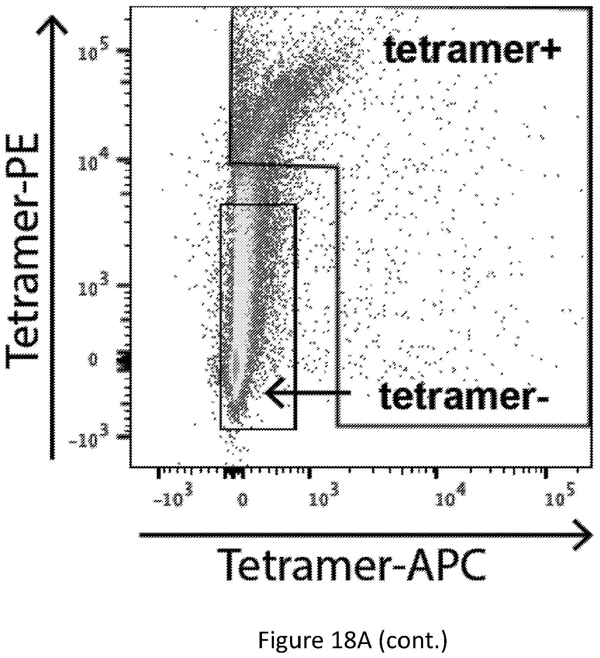

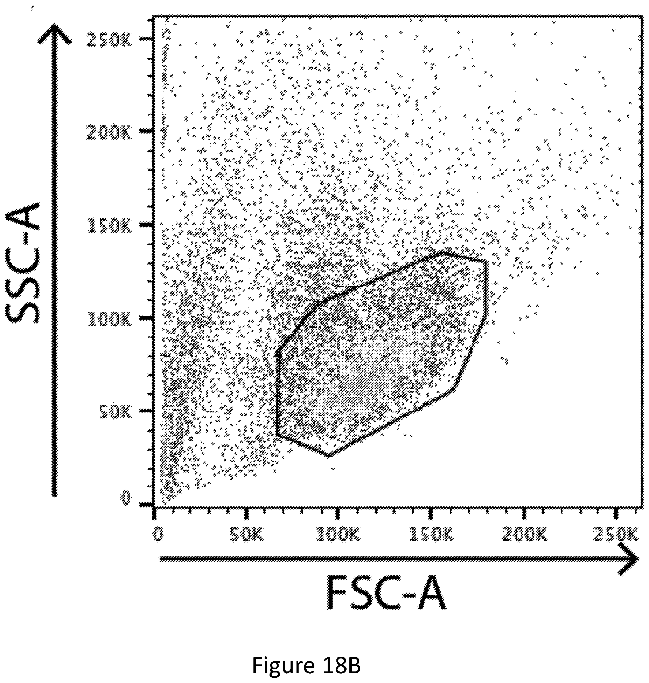

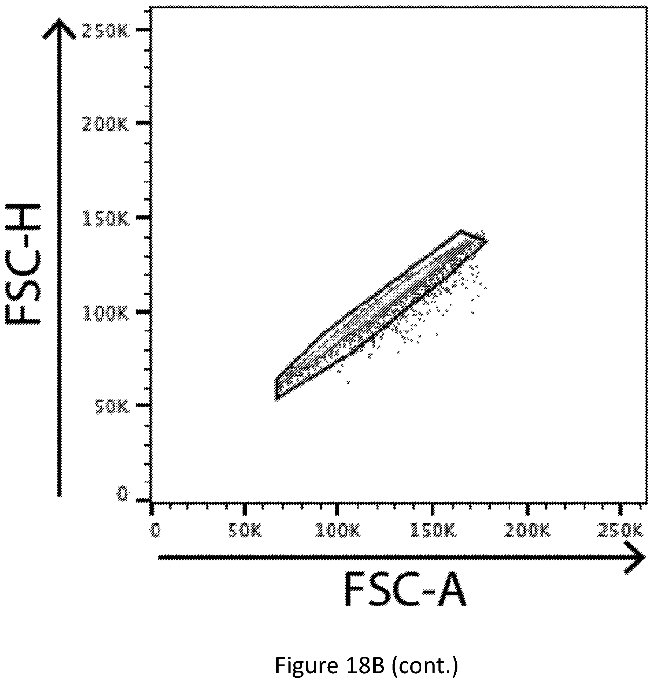

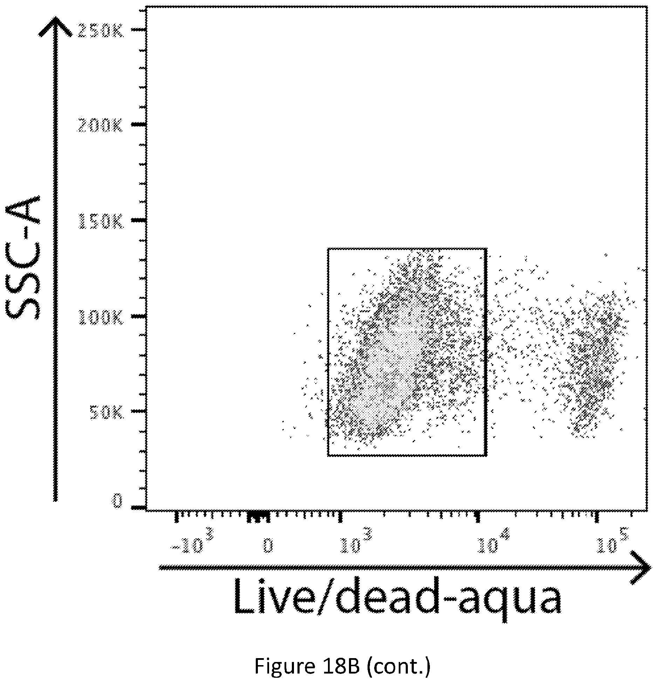

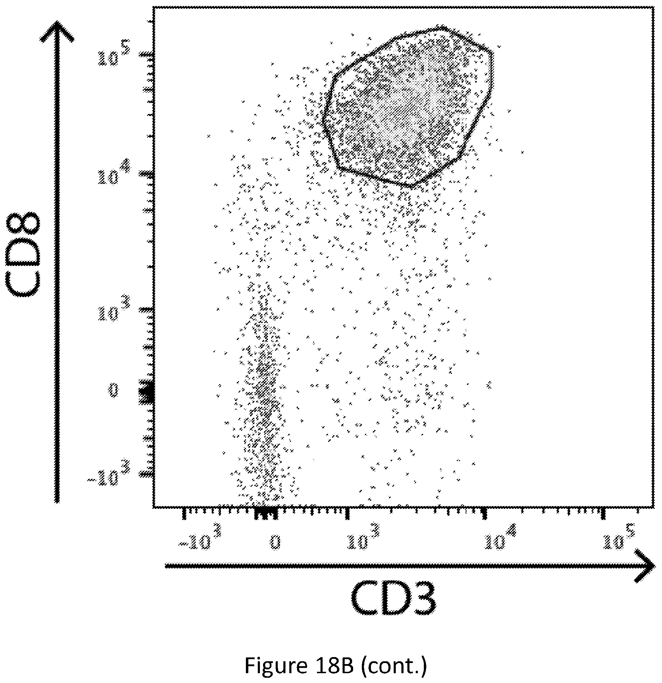

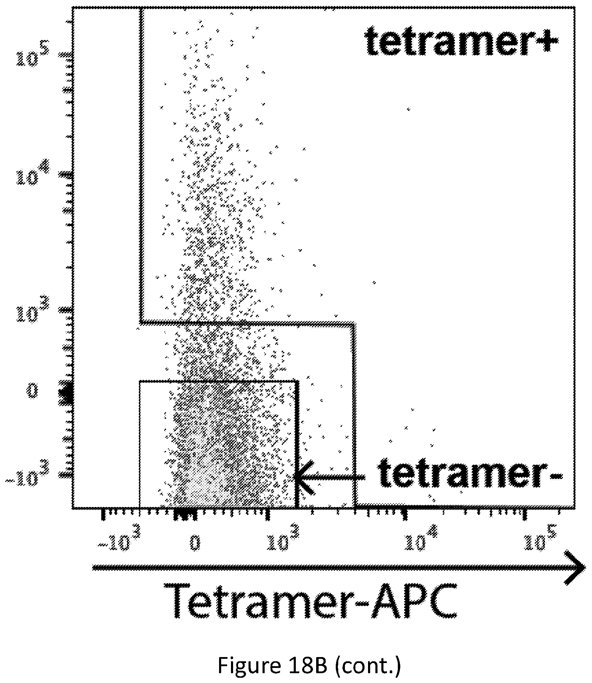

Figures 7A-7E depict the identification of paired αβ TCR sequences with their antigen specificities. (A) Recovery of MART-1 tetramer barcodes on DMF5 cells from PBMC-DC co-cultures spiked with 1% DMF5 cells. Number of MART-1 tetramer reads among DMF5 positive (DMF5-TCR) and negative (NB-TCR) cells. Cells are classified as positive/negative according to sequencing reads of the DMF5 TCR, where >10 DMF5 reads was used as a cutoff to classify DMF5+ cells. Box graphs display the distribution of MART-1 tetramer reads from DMF5+ (>10 DMF5 reads *n* = 76) and DMF5- (≤10 DMF5 reads, *n* = 927) cells. Upper/lower bars and box boundaries indicate the 95th/5th and 75th/25th percentiles, respectively, horizontal box lines indicate medians. Statistical significance was assessed using a two-tailed Mann-Whitney test (*p*-value < 0.0001). (B) Distribution of antigen specificities identified from tetramer + /CD8+ T cells from human splenocytes and the number of tetramer-barcode read per cell. Each dot represents a single cell, n = 102 in total, error bars correspond to one standard deviation from the mean. (C) and (D) V(D)J usage of cells identified as specific for the EBV-BRLF1 (YVLDHLIVV) and NY-ESO-1 (SLLMWITQA) antigens. All TRAVJ (*n* = 11/35) and TRBVJ (*n* = 14/35) chains identified are represented. (E) TCR CDR3 sequences identified for antigen-specific T cells. Consensus BRLF-1 TRA and NY-ESO-1 TRB chains are highlighted in orange and red, respectively. Gating strategies used for sorting tetramer+ cells are outlined in Figure 18.

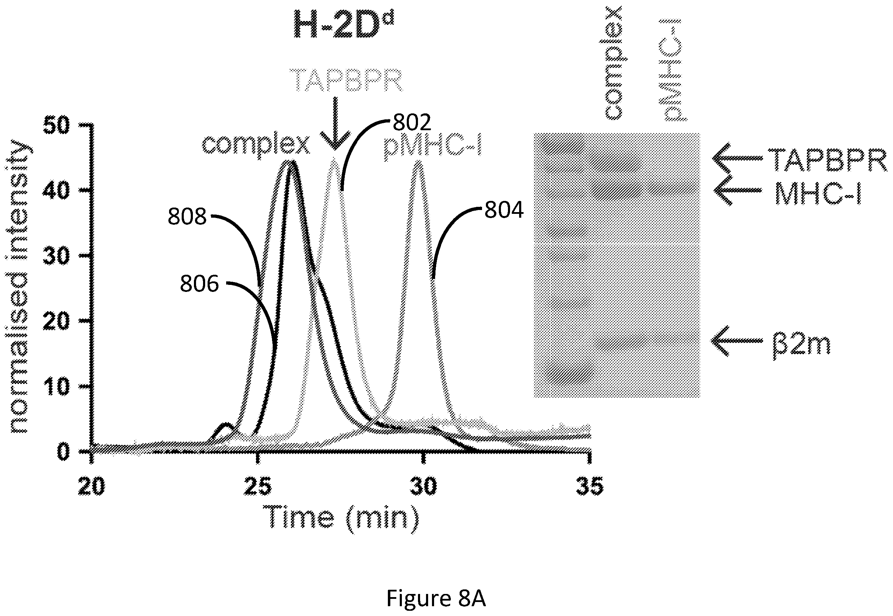

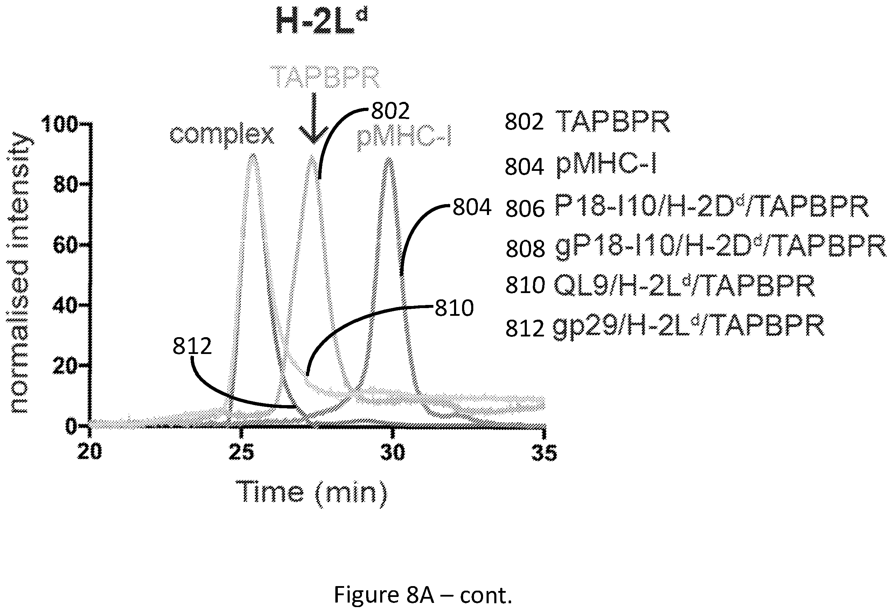

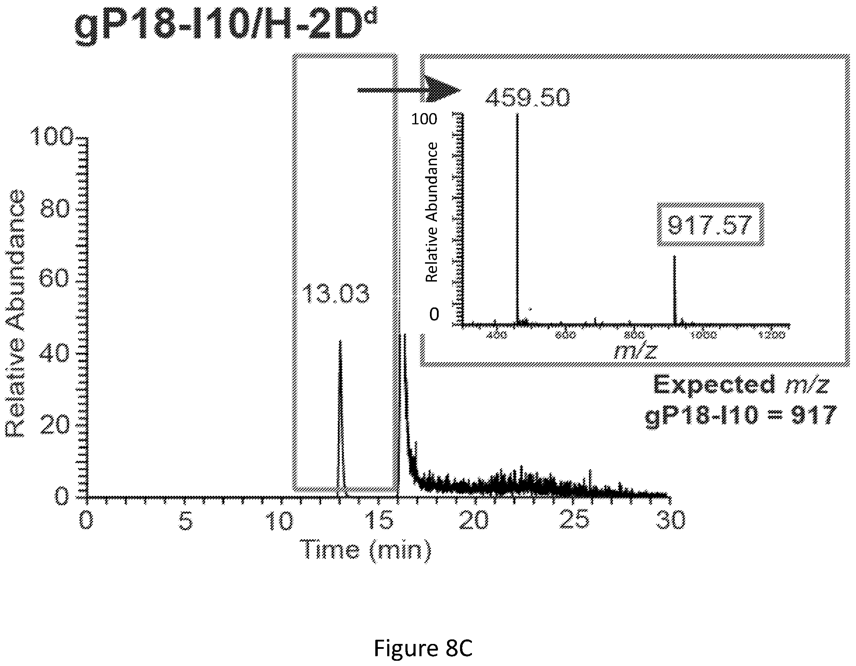

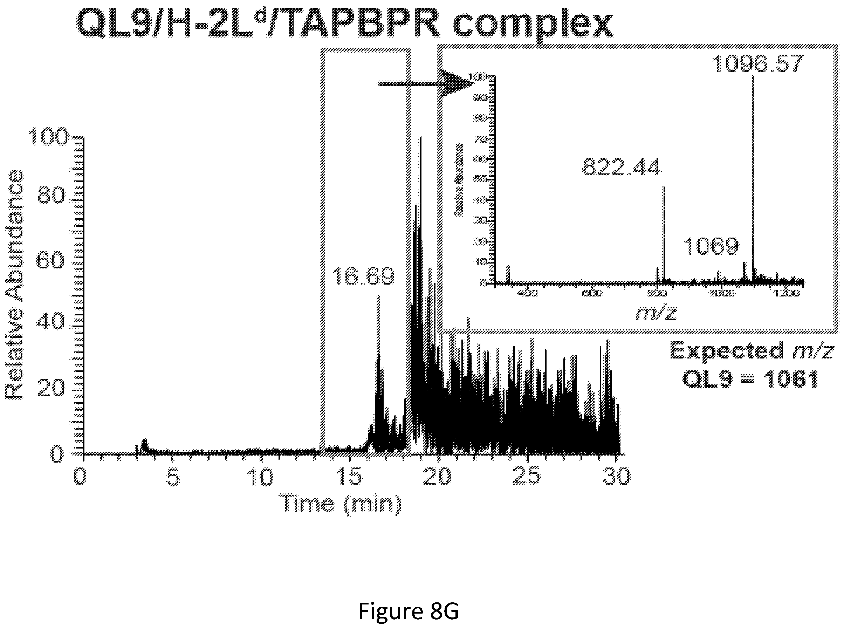

Figures 8A-8G depict a study, showing that destabilizing "goldilocks" peptides promote isolation of empty murine MHC-I/TAPBPR complexes. (A) Size exclusion chromatography (SEC) elution profiles of H-2D^{d} bound to RGPGRAFVTI (P18-I10) or GPGRAFVTI (gP18-I10) (left panel) and H-2L^{d} bound to _PNVNIHNF (gp29) or QLSPFPFDL (QL9) (right panel) in the presence or absence of TAPBPR. SEC of H-2L^{d} molecules shown was performed in the presence of 10 mM GF dipeptide. Inset shows SDS polyacrylamide (12%) electrophoresis analysis of elution fractions at 26 min / 18 mL (pMHC-I/TAPBPR complex) and 30 min / 15 mL (pMHC-I alone). Size markers correspond to sizes of 10, 15, 20, 25, 30, 40, and 50 kDa from bottom to top, respectively. (B-G) Analysis of MHC-I peptide occupancy by LC-MS. Chromatograms shown are filtered to only display peaks containing *m*/*z* ions of interest. Insets show MS analysis of the region indicated by the red box. (B) P18-110/H-2D^{d}/ TAPBPR complex, (C) gP18-I10/H-2D^{d}, (D) gP18-I10/H-2D^{d}/TAPBPR complex, (E) p29/H-2L^{d}/TAPBPR complex, (F) QL9/H-2L^{d}/TAPBPR complex, (G) QL9/H-2L^{d}/TAPBPR complex. Data show are representative of triplicate assays.

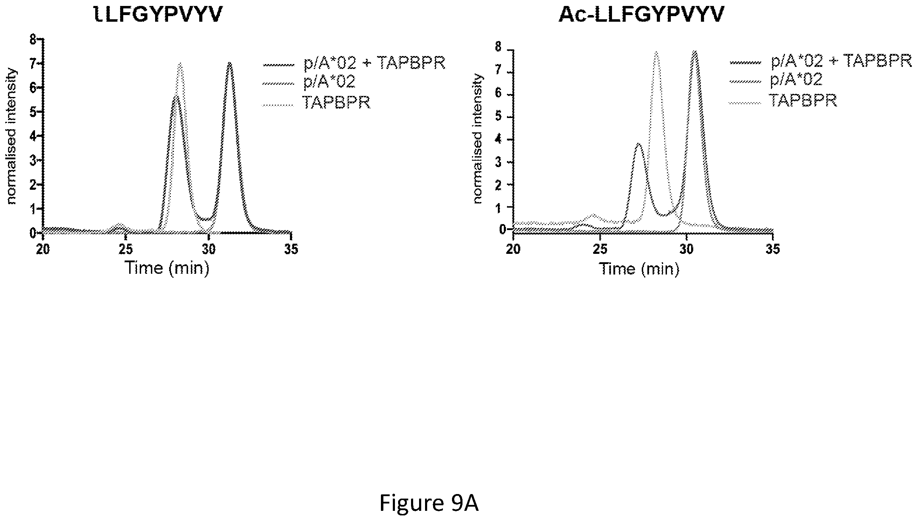

Figures 9A-9D provide an assessment of peptide occupancy of HLA-A*02:01/TAPBPR complexes. (A) SEC elution profile of ┐ LFGYPVYV/HLA-A*02:01, where ┐ denotes a D-Leucine residue (left), and Ac-LLFGYPVYV/HLA-A*02:01, where Ac-L denotes an acetylated N-terminal Leucine residue (right), in the presence or absence of TAPBPR at an equimolar concentration. All binding experiments were performed in the presence of 10 mM GM dipeptide. (B) Analysis of peptide occupancy of gTAX/HLA-A*02:01 complex (left) and HLAA*02:01/TAPBPR complex (right) by LC-MS. Chromatograms shown are filtered to display ions of interest. I nset: MS analysis of the region indicated. (C) SEC analysis of HLA-A*02:01/TAPBPR complex dissociation in the presence of high affinity (TAX) peptide. (D) MS analysis of TAX/HLA-A*02:01 isolated from HLA-A*02:01/TAPBPR complexes loaded with TAX. All MS analysis is done on SEC purified HLA-A*02:01. Data show are representative of triplicate assays.

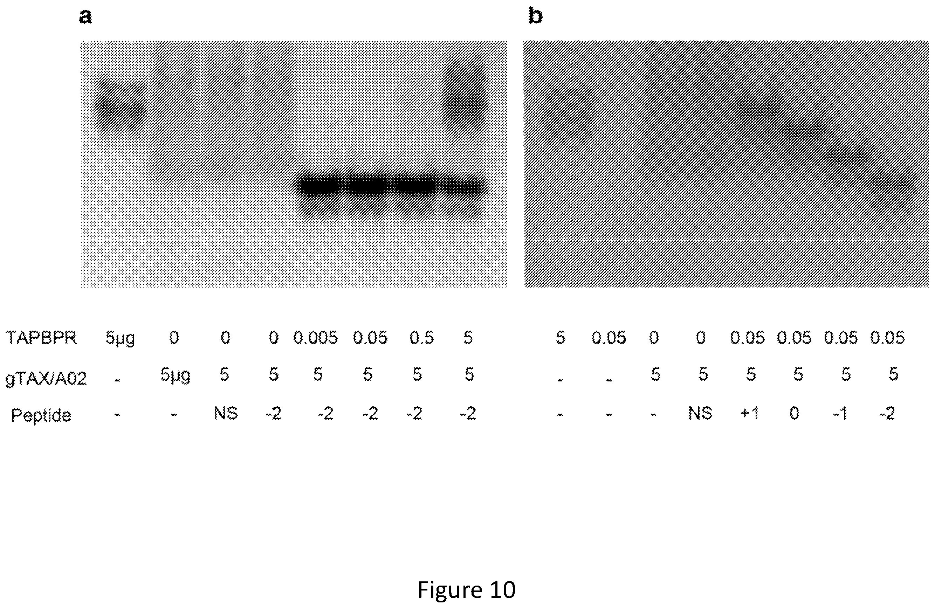

Figures 10a and 10b provide a demonstration of peptide exchange using catalytic amounts of TAPBPR. (a) Native gel electrophoresis analysis of peptide exchange using varying concentrations of TAPBPR (0.005 µg to 5 µg), monitoring the formation of exchanged pMHC molecules of different electrophoretic mobilities. Each indicated reaction was incubated overnight at 4 °C in the presence of 10-fold molar excess of a high affinity peptide (sequence SLLDDAFAL, net charge of -2 at neutral pH), and a fixed concentration of gTAX/HLA-A*2:01. (b) Native gel electrophoresis analysis of TAPBPR exchange on gTAX/HLA-A*02:01 of four different high affinity peptides with net charges at neutral pH ranging from +1 to -2 (sequences RVADYIVKV, ALFPERITV, AIADISYSV, SLLDDAFAL), as indicated. 12% polyacrylamide native gels were run at 90V for 5 hrs at 4°C before visualization with InstantBlue (Expedeon). NS: non-specific gp29 peptide (sequence YPNVNIHNF), used as a negative control. Data shown is representative of triplicate gel assays. All protein samples used in (a) and (b) were derived from the same peptide exchange experiment, and the gels were processed in parallel.

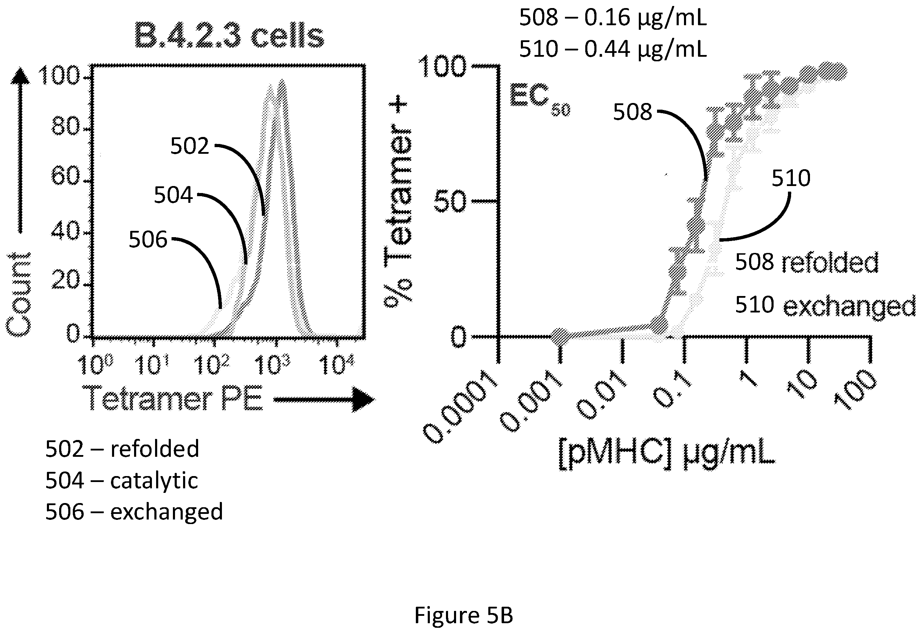

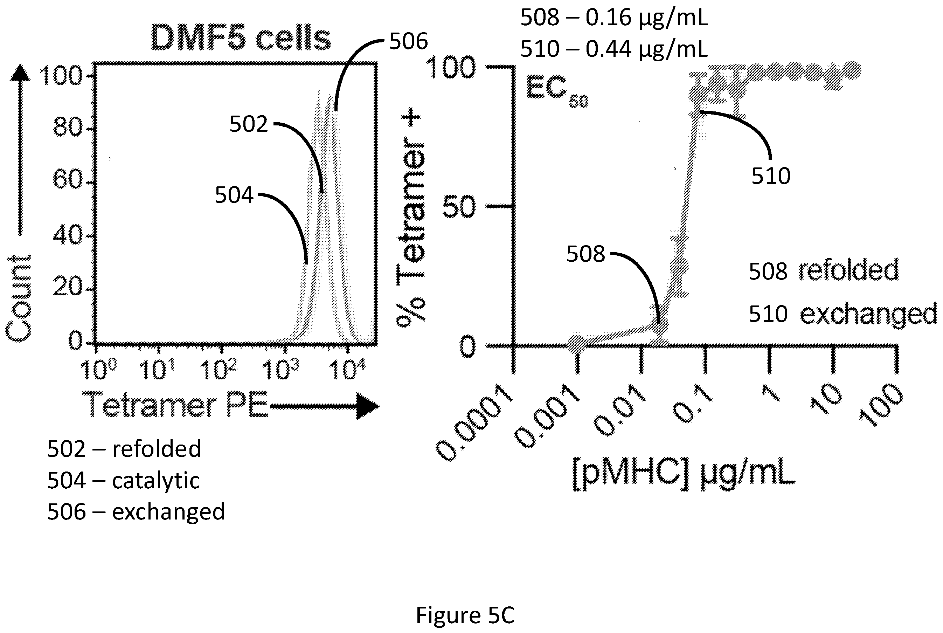

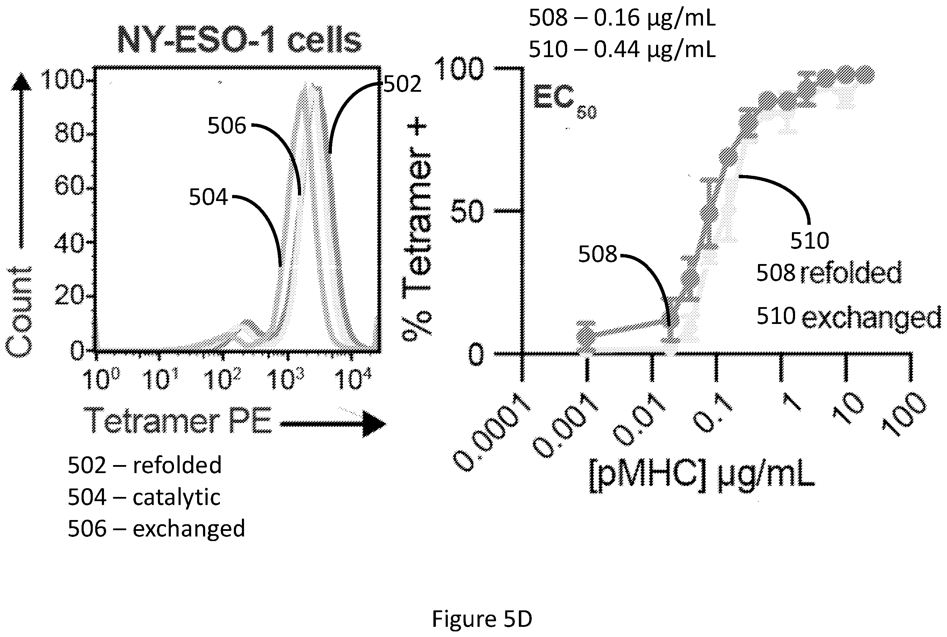

Figure 11 provides a flow cytometry gating strategy for Figure 5. Acquisition and analysis was performed on an LSRII using FACSDiva software (BD, Franklin Lakes, NJ). Cells were sorted by side and forward scatter (SSC-A and FSC-A) and by propidium iodide exclusion (PE-Cy5) for viability and exclusion of multimers. Gating for single live-cells was determined by comparison of unstained and fluorescently labelled cells compensated for spectral overlap of PE, FITC and PI. Murine 58α⁻β⁻ cells expressing the B4.2.3 TCR which recognizes P18-I10 bound to H-2D^{d}, were stained with BD PE-Rat Anti-Mouse Vα2 TCR Ab to confirm TCR receptor expression. The human T cell lines DMF5 and NY-ESO-1, were stained with BD FITC Mouse anti-human CD8 Ab.

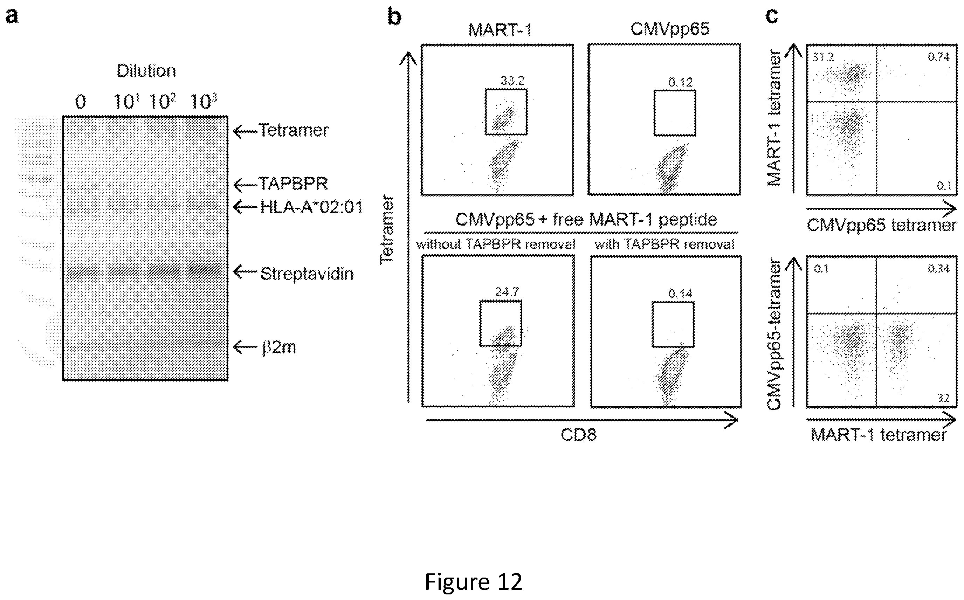

Figures 12A-12C provide a study showing that removal of TAPBPR abrogates the exchange of peptides on and between tetramers for library production. (a) SDS polyacrylamide (12%) electrophoresis of tetramers washed using a 100 kDa spin filter at 10, 100 and 1,000-fold dilutions to remove TAPBPR. Data shown is representative of triplicate gel assays. Size markers correspond to sizes of 10, 15, 20, 25, 30, 40, and 50 kDa from bottom to top, respectively. (b) Representative plots showing tetramer staining of DMF5 T cells with HLA-A*02:01 tetramers prepared by TAPBPR exchange of either the MART-1 epitope specific to the DMF5 TCR, or the irrelevant epitope CMVpp65 (top panels). A very low level (0.12%) of CMVpp65-tetramer positive DMF5 T cells can be detected, likely due to non-specific staining. DMF5 T cells were independently stained with CMVpp65-tetramers incubated with free MART1 peptide at 10-fold molar excess (relative to pMHC-I) without TAPBPR removal (bottom left) or upon complete removal of TAPBPR, as shown in (a) (bottom right), showing recovery of a low (0.14%) level of background staining in the absence of TAPBPR. (c) Staining of DMF5 T cells using a 1:1 mixture of PE-CMVpp65:APCMART-1 tetramers (top panel), or PE-MART-1:APC-CMVpp65 tetramers (bottom panel). In each plot, both tetramer samples were prepared individually using TAPBPR exchange, followed by complete removal of TAPBPR and excess peptide, mixing of the two tetramers and overnight incubation at 4 °C. The absence of a significant PE-tetramer positive population (0.1% - x-axis) in the top panel, or an APC-tetramer positive population in the bottom pane (0.1% - y-axis) is indicative of a negligible background of cross-exchange of peptides between tetramers in the absence TAPBPR, allowing their incorporation into stable tetramer libraries. Numbers in the plots indicate the percentage of total cells. Data show are representative of triplicate, independent staining and flow cytometry experiments.

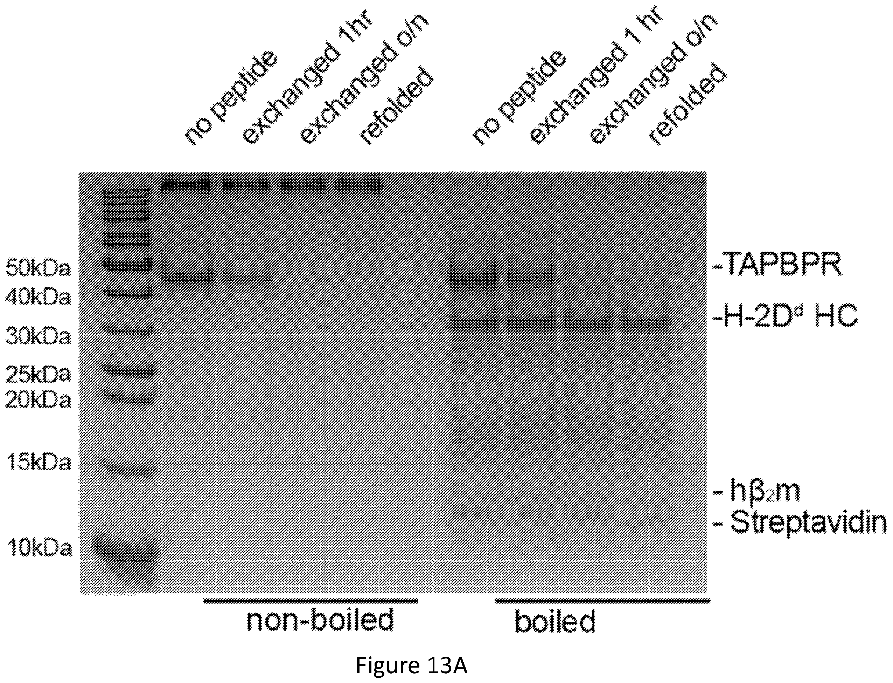

Figures 13A-13B depict a study, showing efficiency of TAPBPR-mediated loading of a high affinity peptide on H-2D^{d}. Tetramers produced by either 1 hr or overnight incubation of stoichiometric TAPBPR:H-2D^{d} complex with 10-fold molar excess of P18-I10 peptide were examined by SDS PAGE and flow cytometric staining of cells expressing B4.2.3, a TCR which recognizes P18-I10 in the context of MHC-I H-2D^{d}: (A) SDS polyacrylamide (12%) electrophoresis of tetramers assembled from pMHC-I using either protocol. Tetramers were solubilized in SDS running buffer with DTT, then boiled (as indicated) prior to electrophoresis. Refolded H-2D^{d} / P18-I10 and empty H-2D^{d} PE-tetramers were included as positive and negative controls, respectively. HC: heavy-chain. Data shown is representative of triplicate gel assays. (B) Titration curve of H-2D^{d} / P18-I10 tetramer binding to B4.2.3 cells. Tetramers were prepared using stoichiometric TAPBPR peptide loaded MHC-I following overnight (red circles) one hour (blue circles) exchange. EC₅₀ was calculated using a sigmoidal 4 point-plot where X equals log of concentration, using GraphPad Prism v8.0 for Mac. Error bars represent standard deviation from the mean. Data represent three independent experimental observations.

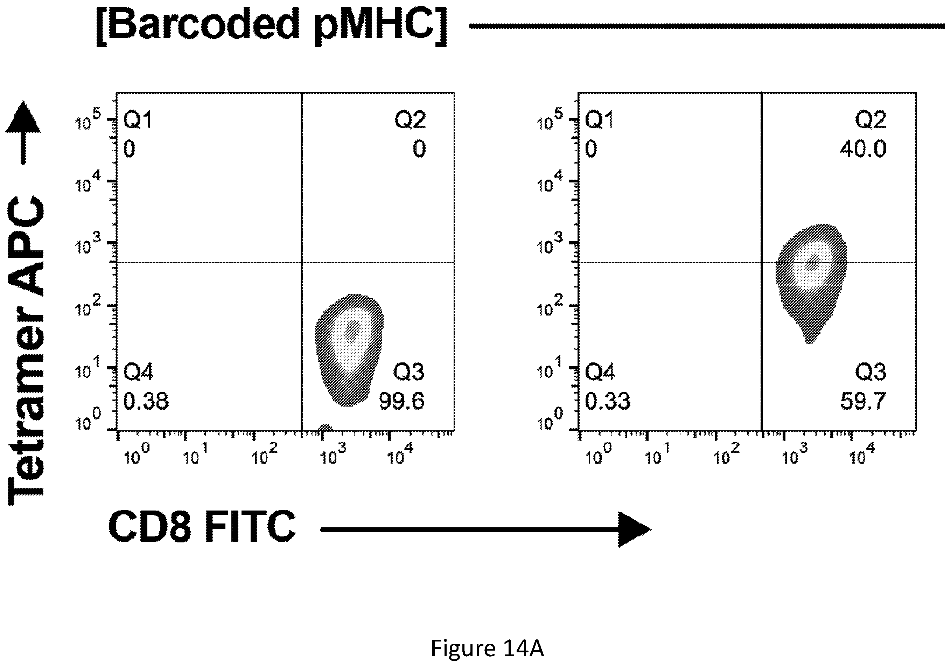

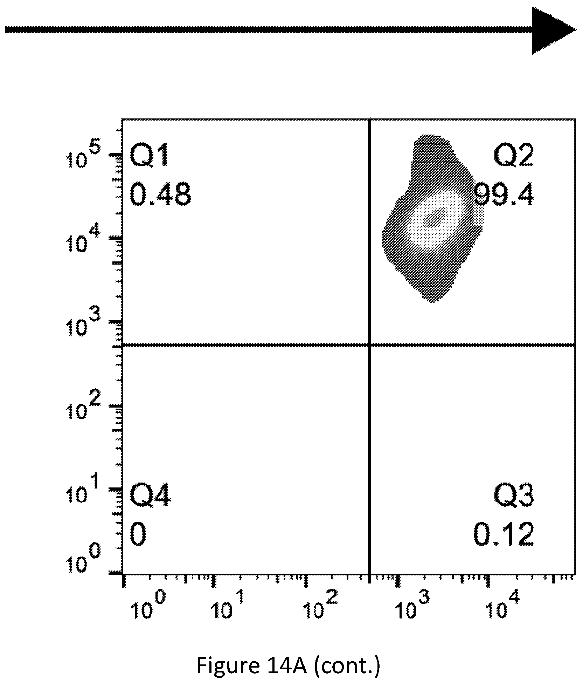

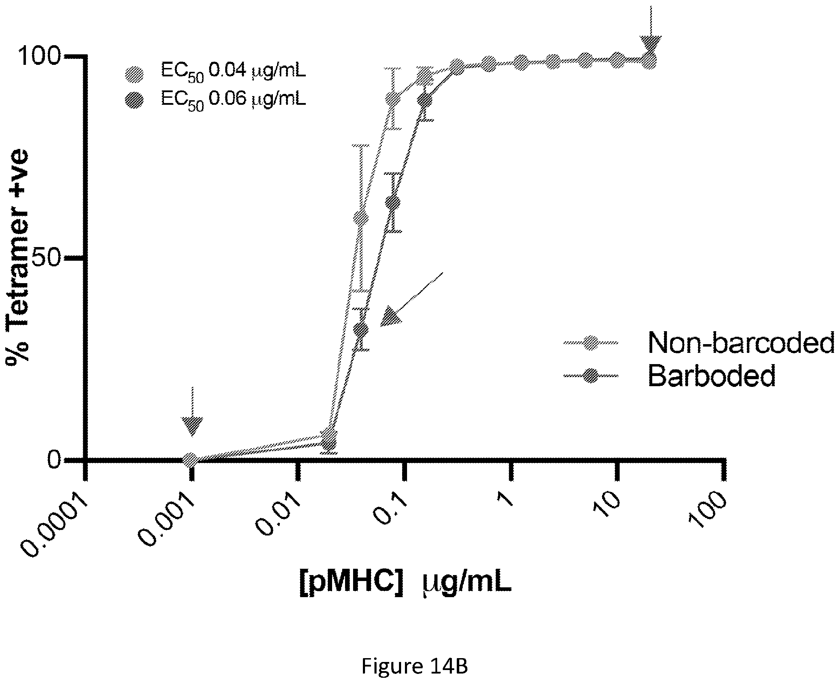

Figures 14A and 14B provide a comparison of barcoded and non-barcoded tetramer binding to cognate TCR receptor. (a) Increasing amounts of MART-1-APC barcoded tetramers produced by catalytic exchange were analyzed by flow cytometric staining of cells expressing DMF5, a TCR which recognizes MART-1 in the context of HLA-A*02:01 restriction. Data shown is representative of triplicate staining assays. (b) Titration of bar-coded and non-barcoded tetramers. Data represents three individual flow cytometry experiments. Blue arrows indicate the concentration of pMHC-I for the three plots shown in panel (a). Percentage of cells staining positive with tetramer over a serial two-fold dilution series were plotted and _{EC50} values calculated by curve fitting to a sigmoidal line (with R2 values in the 0.97-0.99 range), using Graph Pad Prism version 8 for Mac (GraphPad Software, La Jolla California USA). Data shown is representative of triplicate assays and error-bars are standard deviation from the mean. Gating strategies used for sorting tetramer-positive cells are outlined in Figure 11.

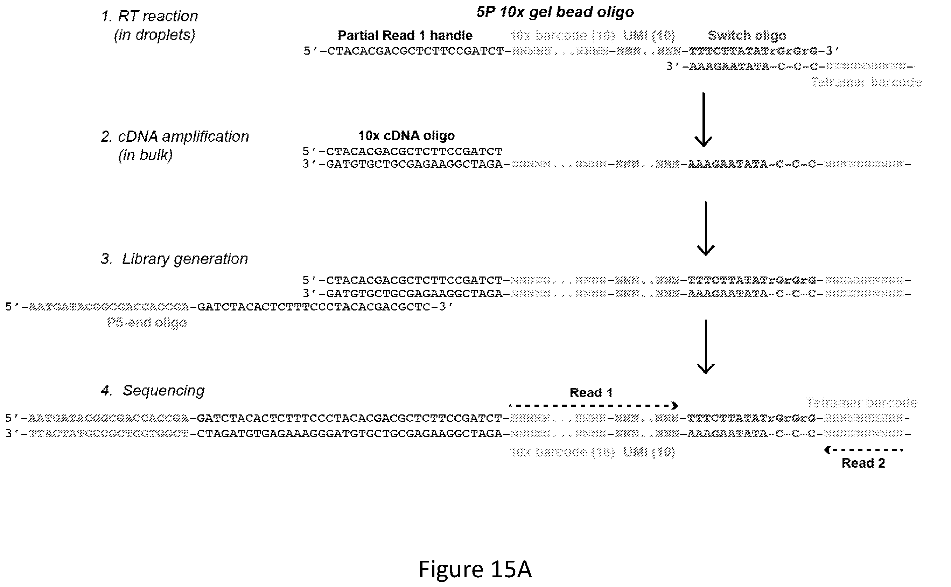

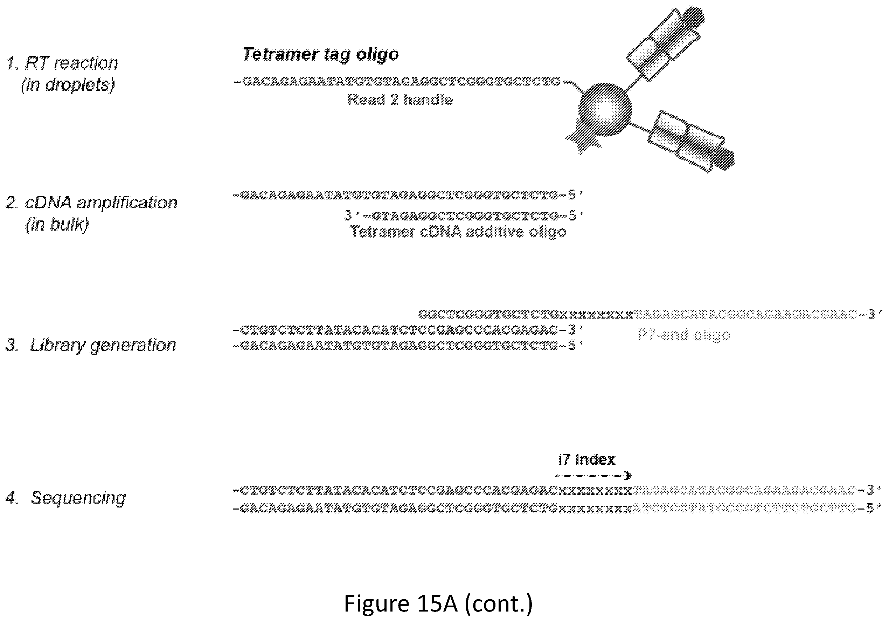

Figures 15A-15C show ECCITE-seq adapted to capturing barcoded pMHC-I tetramers. (A) Biotinylated 5P ECCITE-seq oligos were conjugated to fluorophore-labelled (red star) streptavidin tetramers. The oligos contain a unique tetramer barcode, a switch oligo sequence that provides a handle for 10x compatibility by annealing of 5P 10x gel bead oligos during first-strand cDNA synthesis, in addition to a 3' Illumina NGS sequencing handle (Nextera read 2). The 10x 5P kit was used with specific protocol modifications (as outlined in Online Methods) to capture oligo-derived tags and mRNA-derived cDNA. Only oligo capture is shown here (step 1). After separation of the large and small fractions, following cDNA amplification with additive primer (step 2), the low molecular weight fraction was amplified with 10x Genomics SI-PCR oligo and a Nextera P7 oligos (step 3) to create a sequencing library compatible with Illumina instruments (step 4). The high molecular weight cDNA fraction was processed according to manufacturer's instructions. Tetramer tags and TCR cDNAs from the same cell will share the same cell barcode and can be associated. (B) Bulk amplification of all PE-tetramer barcodes contained in library 2. (C) Comparative staining of DMF5 Jurkat T cells with a single (non-barcoded) PE-tetramer prepared by TAPBPR exchange of the MART-1 peptide (left panel), versus an equal concentration of the full barcoded tetramer library 2, comprising of 34 epitopes, including MART-1 (right panel). Gating strategies are outlined in Figure 11. Data show are representative of triplicate, independent staining and bulk sequencing experiments.

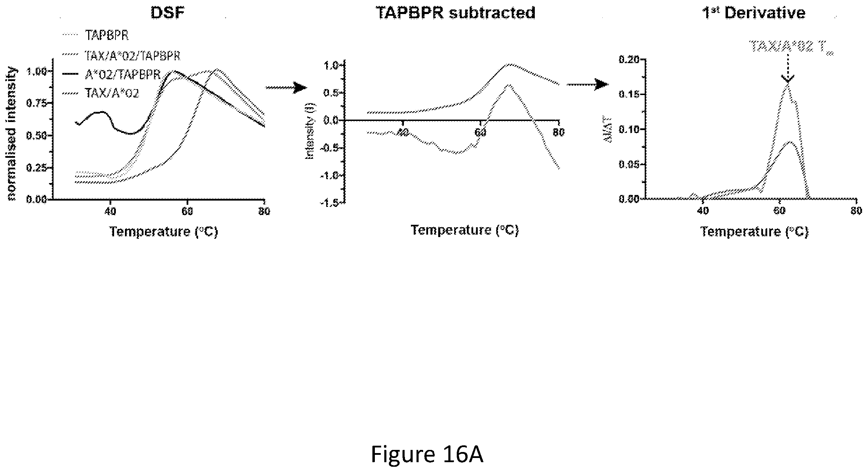

Figures 16A-16C depict high-throughput validation of peptide loading on HLAA*02:01/TAPBPR complexes using differential scanning Fluorimetry (DSF). (a) Derivation of melting temperatures for TAPBPR exchanged TAX/HLA-A*02:01 from conventional DSF data. The DSF trace of TAPBPR alone (left, orange line) was subtracted from that of TAPBPR exchanged HLA-A*02:01 (red line) to obtain the subtracted curve (center, red line). The Tₘ can be extracted by taking the first derivative (right, red line). As a reference the DSF trace of TAX/HLA-A*02:01 is processed in the manner but without removing the TAPBPR trace (blue line). (b) (upper panel) nanoDSF traces showing a negative inflection point for TAPBPR at a Tₘ of 52.5 °C, and a positive inflection point for TAX/HLA-A*02:01 at a Tₘ of 66.5 °C. (lower panel) nanoDSF traces of HLA-A*02:01/TAPBPR loaded with different peptides from the library showing a consistent negative inflection point corresponding to the TAPBPR Tₘ, and a positive inflection point corresponding to the Tₘ values of different pMHC molecules. (c) Correlation between Tₘ values of different pHLA-A*02:01 molecules, measured by DSF, and _{EC50} values of peptide binding on empty HLA-A*02:01/TAPBPR complexes, measured by Bio-Layer Interferometry. EC₅₀ error bars were estimated from 3 independent experiments per peptide. (d) Correlation between Tₘ values of pMHC molecules prepared using photo-exchange and TAPBPR-mediated peptide exchange. (e) Correlation between Tₘ values of TAPBPR exchanged pMHC molecules measured by conventional DSF as shown in (a), and by nanoDSF (b). Data shown is representative of triplicate, independent experiments.

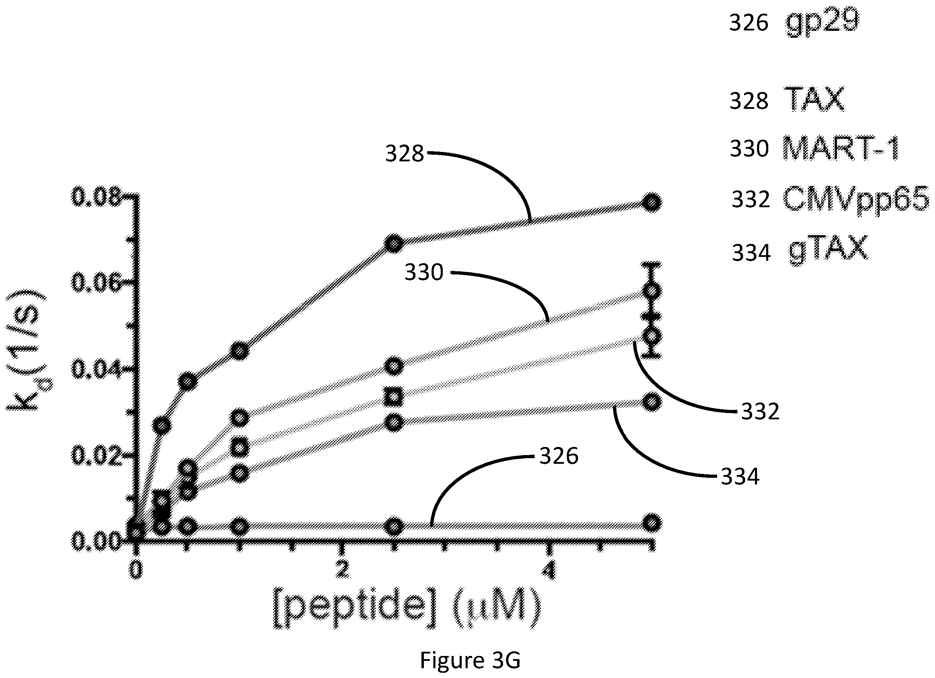

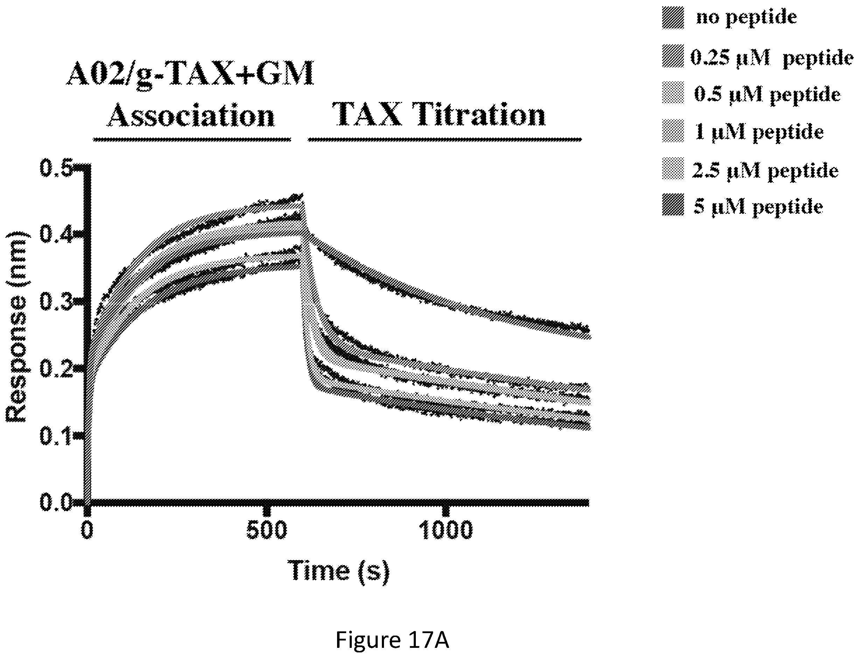

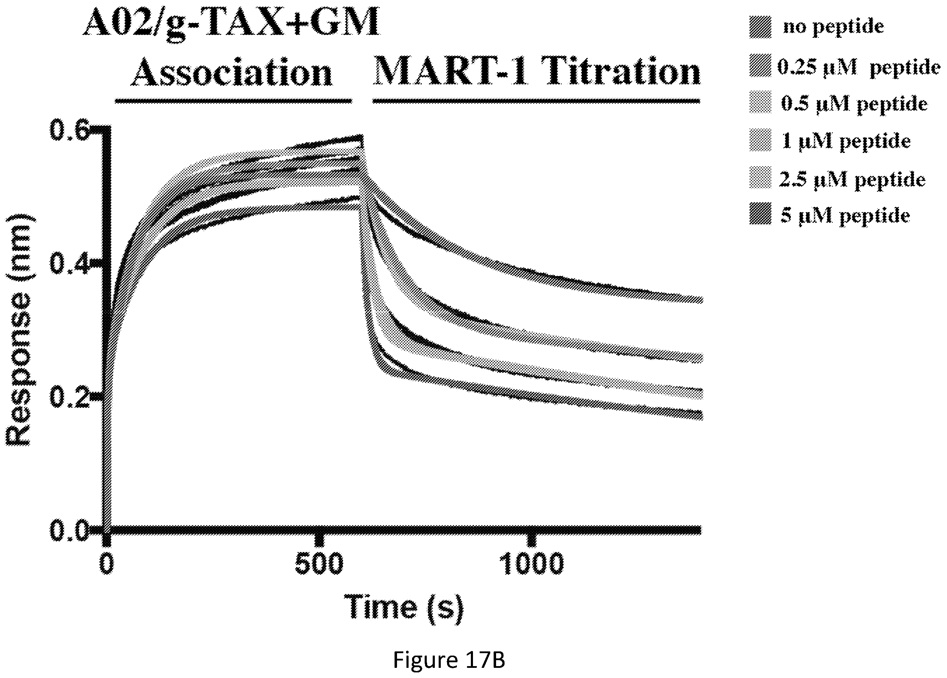

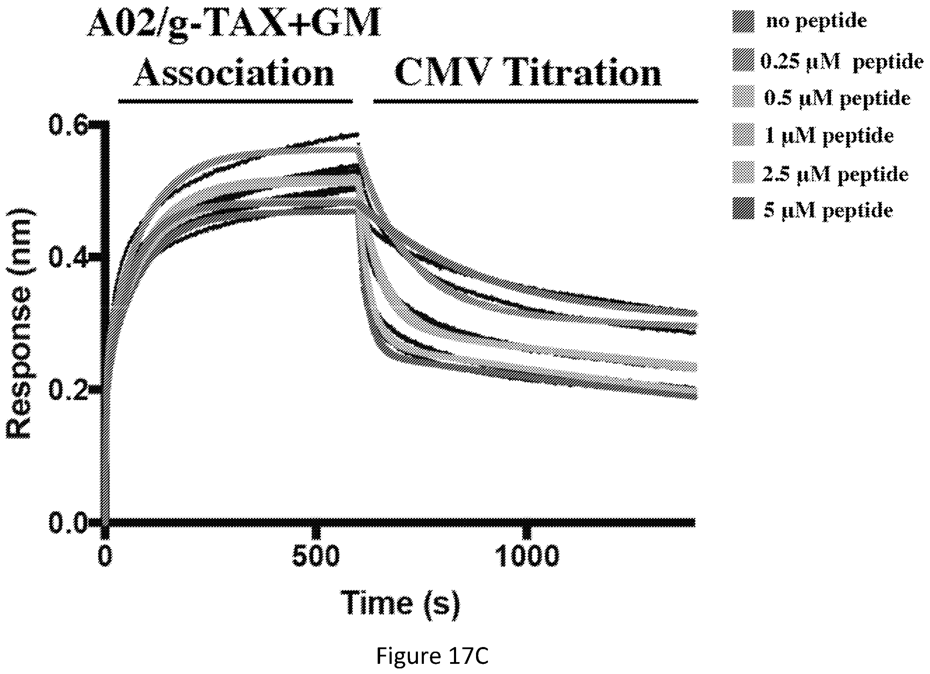

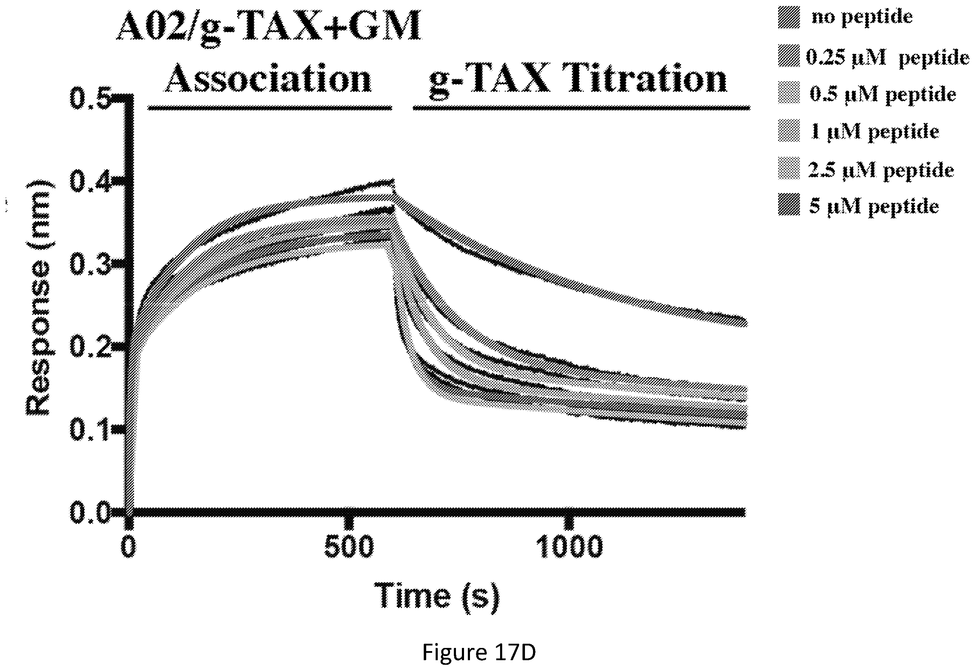

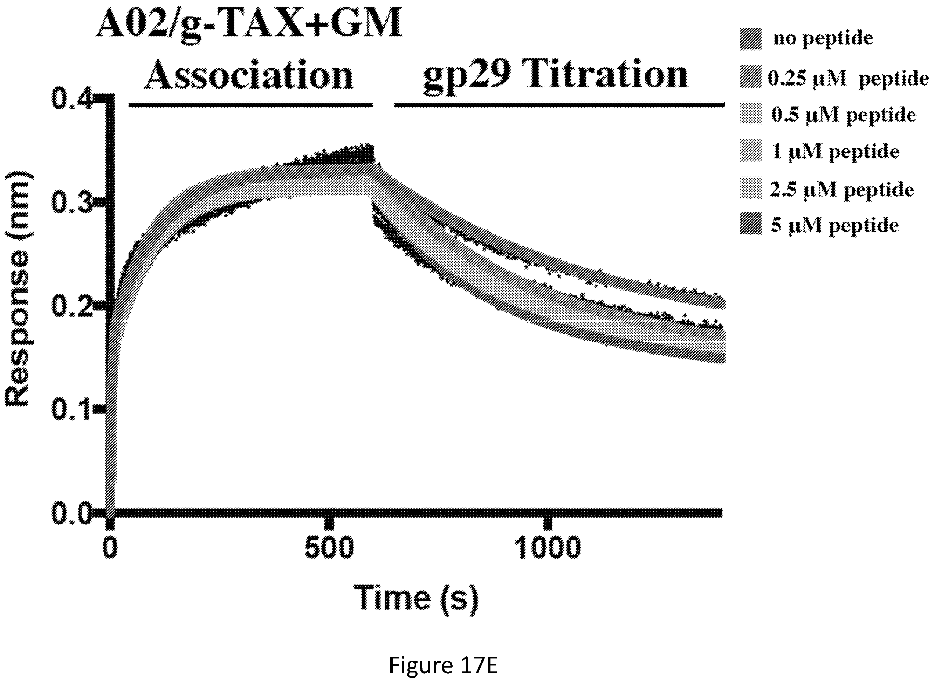

Figure 17 depicts measurement of peptide-induced HLA-A*02:01 dissociation from TAPBPR using Bio-layer Interferometry. Binding and dissociation of HLA-A*02:01 from immobilized TAPBPR on a tip surface. The association of a peptide deficient HLA-A*02:01 to TAPBPR was achieved by the addition of 10 mM GM dipeptide. The dissociation of HLAA*02:01 was measured in the presence of buffer supplemented with increasing concentrations of the indicated peptide. Dissociation data was corrected using a reference biosensor of immobilized TAPBPR (not bound to HLA-A*02:01), in buffer. Data shown are representative of 2 independent experiments. Raw data points are shown in black. All series were fit locally using a 2:1 model with a R² of 0.99 or greater, shown as colored curves. The derived pseudo first-order dissociation rate constants, k_{d}, are plotted as a function of peptide concentration in Figure 3g. Data shown is representative of triplicate, independent experiments.
Figures 18A and 18B provides a flow cytometry gating strategy. (A) Gating strategy used to sort tetramer positive cells expanded *in vitro* with NB epitope pulsed DCs. Mixed cell cultures of pulsed DCs and PBMCs were stained using both PE/APC versions of tetramer library 1, and sorted on a flow cytometer by gating on live cells, then on tetramer positive cells. ( B) Gating strategy used to sort CD8-enriched splenocytes stained with PE/APC versions of library 2. Cells were sorted by SSC-A and FSC-A to select for lymphocytes, then filtered for single cells using FSC-H and then live cells with LIVE/DEAD-Aqua. From this, CD8+ T cells were gated based on CD8 and CD3 staining and then tetramer positive cells were collected for sequencing experiments. Due to low observed staining on the APC fluorophore channel for both samples shown in (a,b) >99% of collected cells correspond to the PE-tetramer positive fractions.

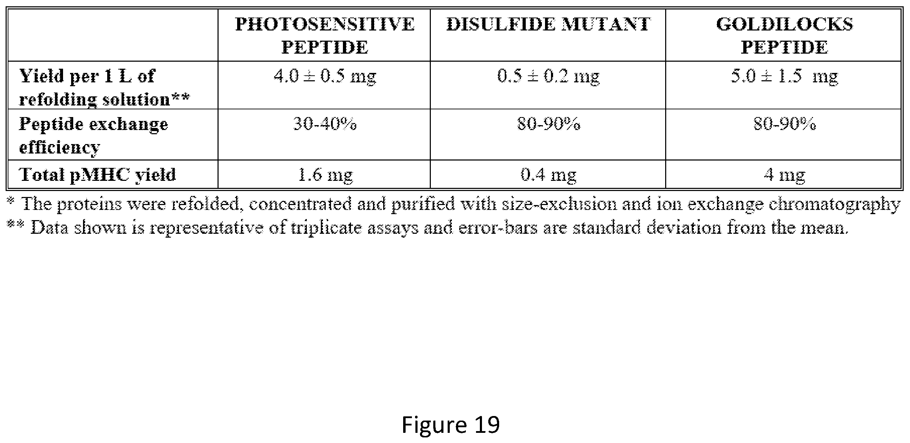

Figure 19 is a table summarizing a comparison of refolding and peptide exchange efficiencies for three different peptide exchange methods: a) photosensitive peptide (Toebes et al., Nat Med 12:246-251 (2006)); b) disulfide mutant (Saini et al., Sci Immunol 4, doi:10.1126/sciimmunol.aau9039 (2019)); and c) goldilocks peptide.

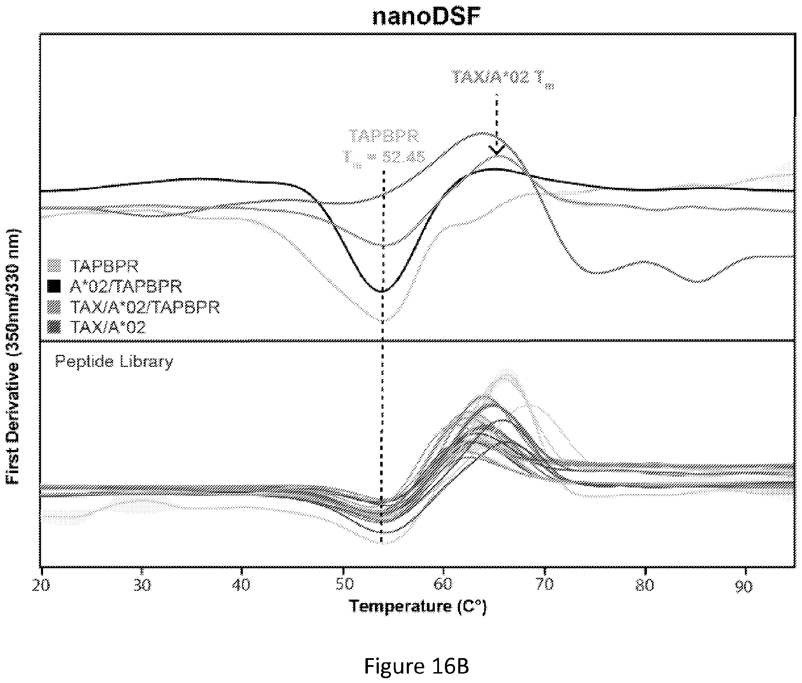

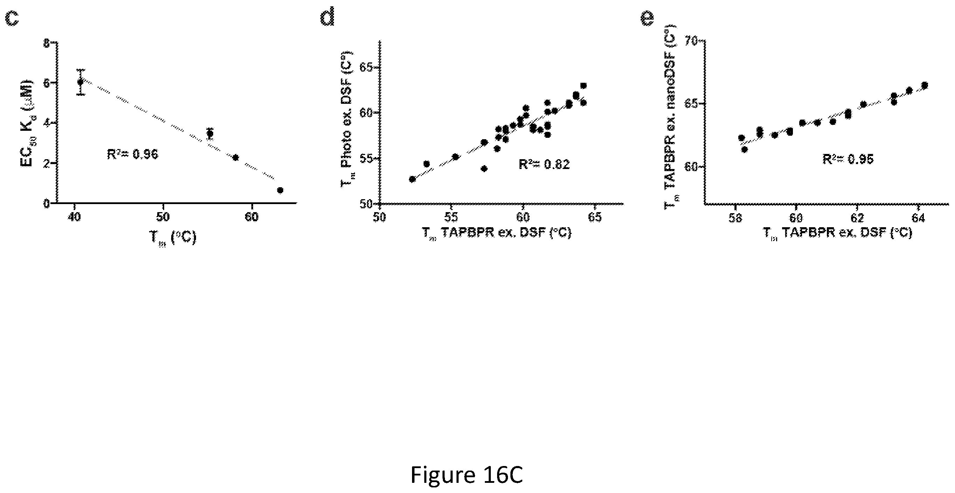

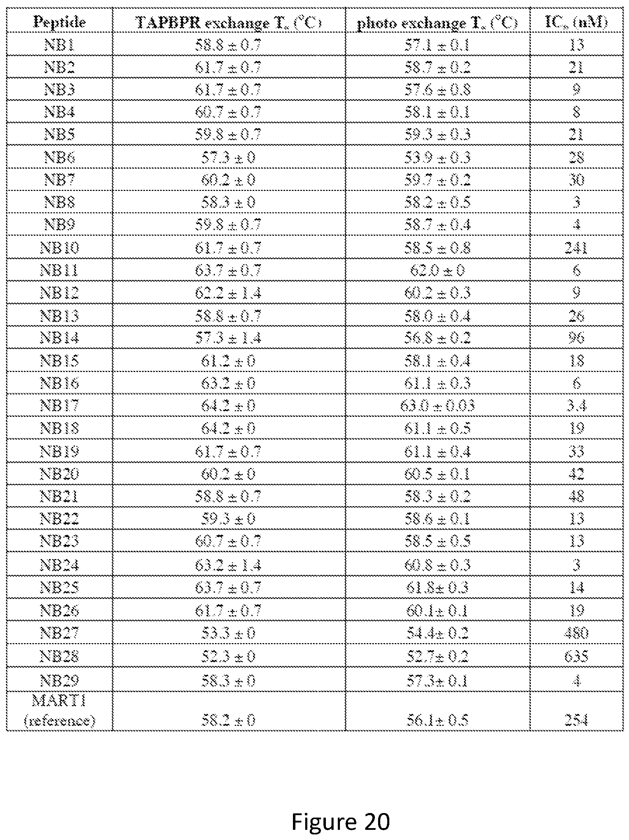

Figure 20 is a table that shows pMHC Tₘ values of Neuroblastoma neoepitopes included in Library 1. Conventional DSF was performed on pHLA- A*02:01 samples prepared by either TAPBPR-mediated peptide exchange (column 2), or exchange using UV irradiation of a photo-sensitive conditional peptide ligand (column 3), as outlined in Online Methods. A 20-fold molar excess of free peptide was used to promote exchange during a 1 hr incubation at room temperature, for all experiments. DSF profiles were analyzed as shown in Figure 16. All measurements were performed in PBS buffer. Errors represent the standard deviation of 3 replicates, individually analyzed. IC₅₀ values were obtained from NetMHCpan-4.0 (Jurtz et al., J. Immunol. 199:3360-3368 (2017)).

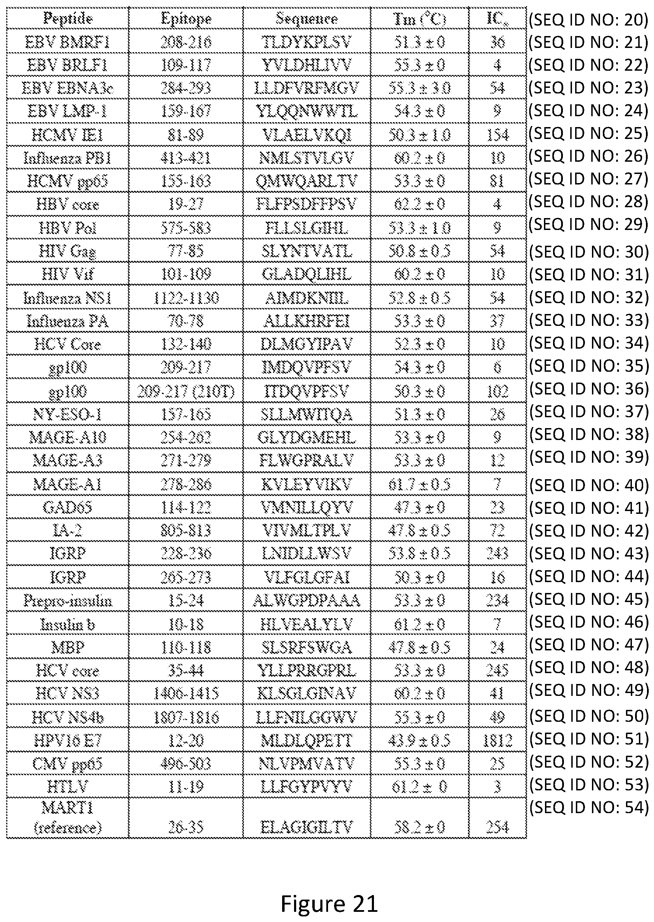

Figure 21 is a table that shows pMHC Tₘ values of viral, tumor, autoimmune epitopes included in Library 2. Conventional DSF was performed on HLA-A*02:01/TAPBPR complexes following 1 hr incubation with each listed peptide. A 20-fold molar excess of free peptide was used to promote exchange during a 1 hr incubation at room temperature, for all experiments. DSF profiles were analyzed as shown in Figure 16. Measurements were performed in PBS buffer supplemented with 0.5% DMSO to increase peptide solubility. Errors represent the standard deviation of 3 replicates, individually analyzed. IC₅₀ values were obtained from NetMHCpan-4.0 (Jurtz et al., J. Immunol. 199:3360-3368 (2017)).

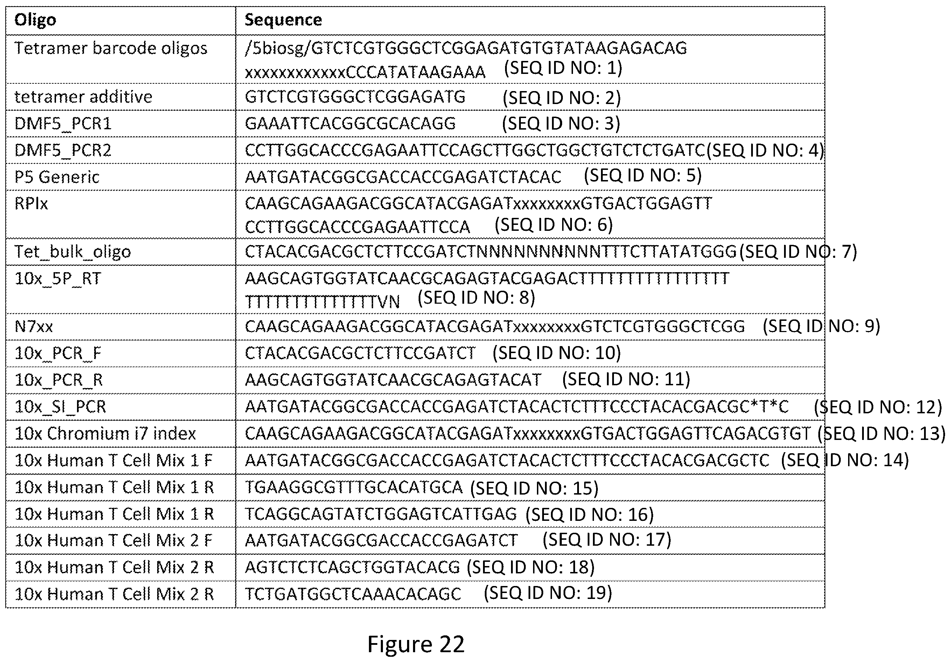

Figure 22 is a table summarizing the primer sequences used for ECCITE-seq analysis described herein.

### DETAILED DESCRIPTION

### A. Overview

High-throughput screening strategies using large arrays of peptide-MHC class I multimers can allow for the large scale identification of antigen specific T cells and/or antigens that bind to particular MHC class Is (*e.g*., tumor antigens). Identification of such antigen specific T cells and/or antigens can in turn lead to the understanding of the pathogenesis of particular diseases, as well as the development of novel therapies.

Such high-throughput screens, however, are limited by the ability to produce large collections of peptide-MHC class I multimers. The instability of peptide-deficient MHC class I molecules, for example, makes large scale production of peptide-MHC class I multimers using such MHC class I molecules difficult. While conditional ligands have been used for large scale production of peptide-MHC class I multimers, such conditional ligands are limited due to sample aggregation, precipitation during the photolysis/peptide exchange step, and, in some cases, instability of the conditional ligand leading to high background levels of exchange. As such, there remains a need for new methods of making peptide-MHC class I multimer complex libraries.

Provided herein are stable peptide-receptive MHC-I complex compositions and methods of making such compositions. Such peptide-receptive MHC-I complexes can be used for production of large collections of peptide-MHC class I multimer complexes (*e.g*., up to 10,000 different specificities), which can then in turn be used in high throughput screens for immune profiling towards disease diagnosis and for the development of new therapies. The peptide receptive MHC-I complexes provided herein are advantageously stable and can be stored for long periods of time prior to their use in making peptide-MHC class I multimer complexes.

### B. Peptide-Receptive MHC-I Complexes

In one aspect, provided herein is a composition that includes a plurality of peptide receptive MHC-I complexes and a plurality of chaperones. The peptide receptive MHC-I complex is not part of the invention. Such compositions are advantageously stable and can be stored for long periods of time in solution prior to use, for example, in making peptide-MHC class I multimer complexes, as described herein. In other aspects, the compositions include peptide receptive MHC-I complexes, but the composition has been subjected to a process that removes free chaperones, such as a dialysis process. In some embodiments, such peptide receptive complexes are substantially free of chaperones.

As used herein, an "MHC class I," "Major Histocompatibility Complex class I," "MHC-I" and the like all refer to a member of one of two primary classes of major histocompatibility complex (MHC) molecules (the other being MHC class II) that are found on the cell surface of all nucleated cells in the bodies of jawed vertebrates. MHC class I molecules function to display peptide fragments of antigen to cytotoxic T cells; resulting in an immediate response from the immune system against a particular antigen displayed with the help of an MHC class I molecule.

MHC class I molecules are heterodimers that consist of two polypeptide chains, α (heavy chain) and β2-microglobulin (light chain). The two chains are linked noncovalently via interaction of light chain and the domain of the heavy chain and floor of the α1/α2 domain. In humans, only the heavy chain is polymorphic and encoded by a HLA gene, while the light chain is species-invariant and encoded by the Beta-2 microglobulin gene. The domain is plasma membrane-spanning and interacts with the CD8 co-receptor of T-cells. The α3-CD8 interaction holds the MHC I molecule in place while the T cell receptor (TCR) on the surface of the cytotoxic T cell binds its syngeneic MHC-I ligand (or matched, in the sense that both the TCR and MHC-I are encoded in the same germline), and checks the coupled peptide for antigenicity. The α1 and α2 domains of the heavy chain fold to make up a groove for peptides to bind. MHC class I molecules bind peptides that, in most cases, are 8-10 amino acid in length.

In mice, MHC class I is called the "H-2 complex" or "H-2" and include the H-2D, H-2K and H-2L subclasses. In humans, MHC class I molecules include the highly polymorphic human leukocyte antigens HLA-A, HLA-B, HLA-C, the less polymorphic HLA-E, HLA-F, HLA-G, HLA-K and HLA-L, and the non-polymorphic MR1 and CD1 proteins. Each human leukocyte antigen (e.g., HLA-A) includes multiple alleles. For example, HLA-A includes over 2,430 known alleles. In some embodiments, the purified peptide receptive MHC-I complex includes an HLA-A. In certain embodiments, the purified peptide receptive MHC-I complexes includes an HLA-B. In other embodiments, the purified peptide receptive MHC-I complex includes an HLA-C. In an exemplary embodiment, the purified peptide receptive MHC-I complex includes an HLA-A02 allele. In other embodiments, the peptide receptive MHC-I complex includes a H-2. In certain embodiments, the H-2 is an H-2D, H-2K or H-2L. In exemplary embodiments, the H-2 is H-2D^{d} or H-2L^{d}.

Peptide receptive MHC-I complexes are created from placeholder peptide-MHC-I complexes (p*MHC-I) through the action of a chaperone. As described herein, placeholder peptide-MHC-I complexes (p*MHC-Is) are formed by incubating a plurality of MHC class I heavy chains, a plurality of β2-microglobulins; and a plurality of placeholder peptides under conditions that favor the formation of the p*MHC-I. In some embodiments, the MHCI-heavy chains are HLA-A*24:02 or HLA-A*68:02. In some embodiments, wherein the heavy chain is HLA-A*24:02 the placeholder peptide has the sequence YPLFGWCF. In some embodiments, wherein the heavy chain is HLA-A*24:02 the placeholder peptide has the sequence LFGPVYV. The chaperone can be any suitable chaperone. In some embodiments, the chaperone is the Tapasin Binding Protein Related (TAPBPR) chaperone. In an exemplary embodiment, the peptide receptive MHC-I complex includes a TAPBPR chaperone and an HLA-A MHC class-I molecule. In further embodiments, the HLA-A is HLA-A02.

In one aspect provided herein is a composition that includes a plurality of placeholder peptide-MHC-I complexes (p*MHC-Is), wherein the plurality of p*MHC-Is each include an MHC class I heavy chain, a β2-microglobulin and a placeholder polypeptide. In some embodiments, the MCI-heavy chains are HLA-A*24:02 or HLA-A*68:02. In some embodiments, wherein the heavy chain is HLA-A*24:02 the placeholder peptide has the sequence YPLFGWCF. In some embodiments, wherein the heavy chain is HLA-A*24:02 the placeholder peptide has the sequence LFGPVYV.

In some embodiments, the peptide receptive MHC-I complexes are contacted with the chaperone at a molar ratio of 1:1 of chaperone to p*MHC-I. In some embodiments, a chaperone such as TAPBPR can act catalytically upon p*MHC-I to yield a peptide receptive MHC-I complex. This means that the reaction that creates a peptide receptive MHC-I complex from a p*MHC-I can be performed even when the molar ratio of chaperone to p*MHC-I is less than 1:1. The molar ratio can be any such ratio of chaperone to p*MHC-I including a ratio less than 1:2, less than 1:10, less than 1:50, less than 1:100, less than 1:500, less than 1:1000, less than 1:5000, or less than 1:10,000 provided that there is sufficient chaperone present to convert at least 50%, at least 60%, at least 70%, at least 75%, at least 80%, at least 85%, at least 90%, at least 95%, or 100% of the p*MHC-I to peptide receptive MHC-I complexes.'

In one aspect provided herein is a composition that includes a plurality of peptide receptive MHC class I complexes, wherein the plurality of p peptide receptive MHC class I complexes each include an MHC class I heavy chain, and a β2-microglobulin. In some embodiments, the MCI-heavy chains are HLA-A*24:02 or HLA-A*68:02.

In some embodiments, the purified peptide receptive MHC class I described herein further includes a chaperone (i.e., a MHC class I/chaperone complex). In some embodiments, the chaperone is Tapasin Binding Protein Related (TAPBPR). In an exemplary embodiment, the peptide-receptive MHC class I/chaperone complex includes a TAPBPR chaperone and an HLA-A MHC class-I molecule. In some embodiments, the HLA-A is HLA-A02. In other embodiments, the HLA-A is HLA-A*24:02 or HLA-A*68:02.

Peptide receptive MHC-I complexes compositions provided herein are advantageously highly stable and soluble. In some embodiments, the peptide receptive MHC-I complexes can be stored at concentrations of up to 50, 100, 150, 200, 250, 300, 350, or 400 µM in solution without precipitation at 4°C. In certain embodiments, the peptide receptive MHC-I complexes are completely soluble and remain peptide receptive in solution at a concentration of up to 400 µM at 4°C for up to 1 month, 2 months, 3 months, 4 months, 5 months, 6 months, 7 months, 8 months, 9 months, 10 months, 11 months, or a year. The stability of subject peptide MHC class I complexes (*e.g*., peptide receptive MHC-I complexes) can be measured, for example, using differential scanning fluorimetry techniques. In certain embodiments, the peptide receptive MHC-I complexes are stored at -80 °C. In particular, embodiments, the peptide receptive MHC-I complexes are lyophilized.

In another aspect, provided herein is a method of making the subject peptide receptive MHC-I complexes. To make such complexes, an MHC class I heavy chain, an MHC class I light chain and a placeholder peptide are first incubated under conditions wherein the MHC class I heavy chain, the MHC class I light chain and the placeholder peptide form a placeholder peptide-MHC class I (p*MHC-I ) complex. The p*MHC-I complexes are then contacted with a dipeptide and chaperone and then changed into peptide receptive MHC-I complexes that can accept a peptide of interest.

In the first step of making the placeholder peptide-MHC class I (p*MHC-I ) complex, MHC class I heavy chains, MHC class I light chains and placeholder peptides are incubated under conditions wherein the MHC class I heavy chain, the MHC class I light chain and the placeholder peptide refold to form a placeholder peptide-MHC class I (p*MHC-I ) complex. MHC class I heavy chains and light chains can be obtained using any method known to one of skill in the art. For example, nucleic acids encoding known MHC class I heavy chains and light chains can be integrated into one or more expression vectors, that are in turn transformed into a suitable host for expression (*e.g*., E. coli). MHC class I heavy chains and light chains produced by such host cells can then be isolated and purified for use with the subject methods. In some embodiments, the MCI-heavy chains are HLA-A*24:02 or HLA-A*68:02. In some embodiments, wherein the heavy chain is HLA-A*24:02 the placeholder peptide has the sequence YPLFGWCF. In some embodiments, wherein the heavy chain is HLA-A*24:02 the placeholder peptide has the sequence LFGPVYV.

In some embodiments, the heavy chain, light chain and placeholder peptide are incubated in a refolding buffer that favors the formation of the p*MHC-I complex. In an exemplary embodiment, the refolding buffer includes arginine-HCI, EDTA, reduced oxidized L-glutathione, and Tris base. The heavy chain and light chain can be present at any ratio that favors the formation of the p*MHC-I complex. In certain embodiments, the MHC class I heavy chains and light chains are incubated at about a 1:1, 1:2, 1:3, 1:4, 1:5 ratio of heavy chain to light chain. In an exemplary embodiment, the MHC class I heavy chains and light chains are incubated at a ratio of 1:3 in the presence of the placeholder polypeptide. The MHC class I heavy chain and light chains can be incubated with the placeholder peptide in the refolding buffer for at least 12 hours, 18 hours, 24 hours, 2 days, 3 days, 4 days, 5 days, 6 days or a week to obtain properly folded p*MHC-I complexes. In some embodiments, the MHC class I heavy chain, light chain and placeholder peptides are incubated for 1 to 4 days. In certain embodiments, this first step is carried out at approximately 3 °C to 10 °C. In an exemplary embodiment, the refolding of the MHC class I heavy chain and light chains with the placeholder peptide is carried out at 4 °C.

Suitable placeholder peptides that can be used in this first step include, for example, modified versions of peptides known to bind to the particular MHC class I allele included in the peptide-receptive MHC class I complex. Such placeholder polypeptides have been modified to have a lower binding affinity to the particular MHC class I molecule compared to the known polypeptide. Peptides known to bind various MHC-class Is can be found, for example, at the MHCBN database (http://crdd.osdd.net/raghava/mhcbn/). In some embodiments, the placeholder polypeptide is a N-terminal truncated version of a known polypeptide that binds to the MHC class I of interest (*e.g*., an HLA-A02). For example, a known peptide that binds to HLA-A02 is the HTLV-1 epitope TAX₁₁₋₁₉, having the amino acid sequence LLFGYPVYV. As such, a suitable placeholder peptide that can be used in making subject peptide receptive MHC-I complexes has the amino acid sequence LFGYPVYV. In some embodiments, wherein the MHC class I is HLA-A02, the placeholder peptide is one of the following sequences: LFGYPVYV (gTAX), Ac-LLFGYPVYV (N-terminally acetylated TAX ), or ILFGYPVYV (first residue is a D-leucine). In some embodiments, where the MHC class I is mouse H-2D^{d}, the placeholder peptide has the sequence GPGRAFVTI.(gP18-I10). In certain embodiments, where the MHC class I is mouse H-2L^{d}, the placeholder peptide is QLSPFPFDL (QL9). In other embodiments, where the MHC class I is HLA-A*24:02, the placeholder peptide is YPLTFGWCF. In some embodiments, where the MHC class I is HLA-A*68:02, the placeholder peptide is LFGYPVYV. In one aspect, provided herein is a composition that includes a p*MHC-I complex, wherein the MHC class I is HLA-A*24:02 and the placeholder peptide is YPLTFGWCF. In another aspect, provided herein is a composition that includes a p*MHC-I complex, wherein the MHC class I is HLA-A*68:02 and the placeholder peptide is LFGYPVYV.

Placeholder peptides are typically at least 8 amino acids long. In some embodiments, the placeholder polypeptide is at least 8, 9, 10, 11, 12 or 13 amino acids long. In particular embodiments, the polypeptide is 8, 9, 10, 11, 12 or 13 amino acids long.

In the second step, the p*MHC-I complex is incubated in the presence of a dipeptide and chaperone. In the presence of the dipeptide and chaperone, the placeholder peptide is displaced from the MHC class I and the chaperone binds the MHC class I to form the peptide receptive MHC-I complexes. In some embodiments, the chaperone acts catalytically on the p*MHC-I complex, changing it to a peptide receptive MHC-I capable of accepting a peptide of interest. As discussed above, the peptide receptive MHC-I complex is stable and can be stored for long periods of time prior to loading with one or more peptides of interest and formation of multimers (*e.g*., tetramers).

In some embodiments, the chaperone is a Tapasin Binding Protein Related (TAPBPR). TAPBPR protein includes a signal sequence, three extracellular domains comprising a unique membrane distal domain, an IgSF (immunoglobulin superfamily) V domain and an IgC1 domain, a transmembrane domain, and a cytoplasmic region. *See, e.g.,* Boyle et al., PNAS 110 (9) 3465-3470 (2013). TAPBPR can be made by any method known in the art, including those described in Morozov et al., http://www.pnas.org/content/113/8/E1006. In certain embodiments, the chaperone is Tapasin. In some embodiments, the chaperone (*e.g*., TAPBR) is incubated with the p*MHC-I complex and dipeptide at ratio of 1:1 chaperone to p*MHC-I complex. In an exemplary embodiment, the dipeptide is glycyl-methionine or glycyl-phenylalanine.

The incubation of the p*MHC-I, chaperone and dipeptide can be done for at least 1 hour, 2 hours, 3 hours, 4 hours, 5 hours and 6 hours. Following incubation, the resulting peptide receptive MHC-I complexes can be isolated by size exclusion chromatography (SEC) and confirmed using any technique known in the art, including, for example, liquid chromatography-mass spectrometry techniques.

In some embodiments, the p*MHC-I, chaperone and dipeptide are incubated at equimolar quantities of p*MHC-I and chaperone (e.g., TAPBR). In certain embodiments described below, the p*MHC-I and chaperone are incubated at a p*MHC-I : chaperone molar concentration of at least 10:1. In some embodiments, the p*MHC-I and chaperone are incubated at a p*MHC-I : chaperone molar concentration of at least 10:1, 20:1, 30:1, 40:1, 50:1, 60:1, 70:1, 80:1, 90:1, 100:1, 500:1, or 1,000:1.

In some embodiments, the MHC class I of the p*MHC-I is an HLA-A. In an exemplary embodiment, the p*MHC-I includes an MHC class I that is HLA-A*24:02 or HLA-A*68:02.

In some embodiments, the MHC class I is a variant MHC class I that includes one or more mutations that reduces MHC class I binding affinity for TAPBR chaperone. Such a mutation advantageously facilitates subsequent peptide loading and/or peptide-MHC class I multimer complex production as described below. In some embodiments, the variant MHC class I includes one or more mutations in the domain of the heavy chain that reduces MHC class I binding affinity for TAPBR chaperone.

In certain embodiments, either or both of the MHC class I heavy chain and β2-microglobulin molecules in the peptide receptive MHC-I composition are biotinylated. In an exemplary embodiment, the MHC-I heavy chain or light chain is biotinylated using a C-terminal BirA tag with any method known to one of skill in the art, including use of a biotin ligase. In an exemplary embodiment, the biotinylation occurs after the purification of the p*MHC-I complex. Biotinylation of peptide receptive MHC-I complex monomers allows for the attachment of such monomers to backbones (*e.g*., streptavidin or dextran) to form multimers (e.g., peptide receptive MHC-I complex tetramers) that can be used in various methods described herein.

### C. Peptide-MHC Class I Complexes

The subject peptide receptive MHC-I complexes can be subsequently loaded with a peptide of interest. In this step, the purified peptide receptive MHC-I complex is incubated in the presence of the peptide of interest. Without being bound by any particular theory of operation, it is believed that when present in molar excess of the peptide receptive MHC-I complexes, the peptide of interest is loaded onto the MHC class I, thereby forming a peptide-MHC class I complex that includes the peptide of interest (pMHC-I). The resulting peptide-MHC class I (pMHC-I) complexes can further be used to form multimers, for example, tetramers. Uses for such multimers (*e.g.*, the identification of antigen specific T cells) are further described herein.

In some embodiments, the peptide of interest is incubated at a molar excess of at least 2, 5, 10, 15, 20, 25, 30, 35, 40, 45, 50, 75 or 100 times to the peptide receptive MHC-I complex. In an exemplary embodiment, the peptide of interest is incubated at a molar excess of 50 times to the peptide receptive MHC-I complex. Such incubations can be carried out at room temperature for at least 30 minutes, 1 hour, 2 hours, 5, hours, 10 hours, 15 hours, 20 hours or a day.

Peptides of interest are typically 8 to 13 amino acids in length. In some embodiments, the peptide of interest is an antigen. In an exemplary embodiment, the peptide of interest is a tumor antigen. Tumor antigen peptides in the context of the MHC class I and multimer complexes described herein can be useful, for example, in the identification of T cells reactive to the antigen of interest.

Purification of the formed peptide-MHC class I (pMHC-I) complexes from the released chaperone can be carried out using size exclusion chromatography (SEC). The newly formed pMHC-I can be confirmed using any technique known in the art, including, for example, liquid chromatography-mass spectrometry techniques.

In some embodiments, the MHC class I of the peptide-MHC class I complexes is an HLA-A. In an exemplary embodiment, the HLA-A is HLA-A*24:02 or HLA-A*68:02. In one embodiment, the pMHC-I is biotinylated. In certain embodiments, the HLA includes an amino acid substitution in the domain of the heavy chain. Such a mutation advantageously reduces HLA binding affinity for TAPBR chaperone, thereby facilitating subsequent peptide loading and/or peptide-MHC class I multimer complex production as described below.

### D. Peptide-MHC Class I Multimer Complexes

Peptide-MHC class I (pMHC-I) complexes made using the subject methods described herein can undergo further multimerization to form multimers that include two or more of the pMHC-Is (i.e., pMHC-I multimers.) In certain embodiments, the multimers include 2, 3, 4, 5, 6, 7, 8, 9 or 10 pMHC-I molecules.

In some embodiments, pMHC-I multimers can be produced by attachment of biotinylated pMHC class I to a backbone (*e.g*., a streptavidin, avidin or dextran backbone), thereby forming a pMHC-I multimer. In an exemplary embodiment, the biotinylation occurs following the purification of p*MHC-I monomers and prior to the formation of peptide receptive MHC-I complexes. In some embodiments wherein large scale production of pMHC-I multimer libraries is desired, aliquots of the peptide receptive MHC-I complexes are incubated with various peptides of interest and allowed to undergo peptide loading. The resulting pMHC-class I complexes are then multimerized in the presence of a suitable multimer backbone to form pMHC-class I multimers (*e.g*., tetramers). In some embodiments, the backbone is a streptavidin backbone. In certain embodiments, the backbone is an avidin backbone. In other embodiments, the backbone is a dextran backbone.

In other embodiments, peptide receptive MHC-I complexes are biotinylated and then attached to a backbone (*e.g*., a streptavidin, avidin or dextran backbone), thereby forming peptide receptive MHC-I complex multimers (*e.g.*, tetramers). Such peptide receptive MHC-I complex multimers can be used for the large scale production of pMHC-I multimers comprising one or more peptides of interest by contacting the peptide receptive MHC-I complex multimers with the one or more peptides of interest. For example, in one embodiment, aliquots of the peptide receptive MHC-I complex multimers are contacted with different peptides of interest, thereby forming a library of pMHC-I multimers. After loading of the pMHC-I multimers with peptides of interest, the resulting loaded pMHC-I multimers can be washed to remove any free chaperones, labels (e.g., nucleic acid barcodes) and/or peptides of interest. Following such a washing step, the exchanged pMHC-I multimers can be stored (e.g., 4 °C for several weeks) or used immediately. In some embodiments, the free chaperones, labels and/or peptides of interest are removed by spin column dialysis.

In certain embodiments, the placeholder peptide-MHC class I (p*MHC-I ) complexes are biotinylated and the biotinylated p*MHC-Is are incubated in the presence of chaperones, dipeptides and multimer backbones (e.g., streptavidin-PE) under conditions wherein peptide receptive MHC-I complex multimers (e.g., tetramers) are formed. Such peptide receptive MHC-I complex multimers (e.g., tetramers) can be loaded with peptides of interest (e.g., antigen peptides) as described above.

In some embodiments, the placeholder peptide-MHC class I (p*MHC-I ) complexes are incubated in the presence of chaperones, dipeptides and multimer backbones using a "stoichiometric" approach with equimolar quantities of p*MHC-I and chaperone (e.g., TAPBR).

In certain embodiments, a "catalytic" approach is taken wherein biotinylated p*MHC-Is are incubated in the presence of chaperones, dipeptides, multimer backbones (e.g., streptavidin-PE) and peptides of interest under conditions wherein peptide-MHC class I (pMHC-I) multimers (e.g., tetramers) are formed. In such embodiments, the p*MHC-I and chaperone are incubated at a p*MHC-I: chaperone molar concentration of at least 10:1, 20:1, 30:1, 40:1, 50:1, 60:1, 70:1, 80:1, 90:1, 100:1, 500:1, or 1,000:1. In exemplary embodiments, the p*MHC-I and chaperone are incubated at a p*MHC-I: chaperone molar concentration of 100:1. In such embodiments, molecular tags (e.g., DNA barcodes) can also be added in the incubation to form wherein peptide-MHC class I (pMHC-I) multimers (e.g., tetramers) with tagged backbones to facilitate tracking and detection as explained below.

In some embodiments, the pMHC-I multimer is a dimer. In some embodiments, the pMHC-I multimer is a trimer. In preferred embodiments, the pMHC-I multimer is a tetramer. In one embodiment, the multimer is a dextramer. Dextramers include ten pMHC-I complexes attached to a dextran backbone. Dextramers allow for the detection, isolation, and quantification of antigen specific T-cell populations due to an improved signal-to-noise ratio not present in prior generations of multimers. *See, e.g.,* Bakker and Schumacher, Current Opinion in Immunology 17(4): 428-433 (2005); and Davis et al., Nature Reviews Immunology 11:551-558 (2011).

In some embodiments, the backbone is conjugated with a detectable label (*e.g.,* a fluorophore or a radiolabel) that allow the multimer to be detected in various applications. In certain embodiments, the detectable label is as fluorophore. *See, e.g.,* Nepom et al., J Immunol 188 (6) 2477-2482 (2012). In one embodiment, the detectable label is a radiolabel. In certain embodiments, the backbone includes a barcode (*e.g*., a nucleic acid barcode) that allows the MHC class I multimer to be used in large scale high throughput processes. *See, e.g.,* Bentzen et al., Nature Biotechnology 34(1): 1037-1045 (2016). In an exemplary embodiment, unique barcodes are used for each of the different peptides of interest included in the pMHC-I multimers, thereby allowing for the tracking, sorting and identification of particular pMHC-I multimers in high throughput applications. In particular embodiments, each barcode includes a unique nucleotide sequence.

In some embodiments, the pMHC-I multimer complex is coupled to a toxin (e.g., saporin). Such pMHC-I multimer conjugates can be used to modulate or deplete specific T cell populations. *See, e.g.,* Maile et al., J. Immunol. 167: 3708-3714 (2001); and Yuan et al., Blood 104: 2397-2402 (2004).

### E. Peptide-MHC Class I Multimer Libraries

The methods provided herein allow for the large scale production of stable peptide receptive MHC-I complexes that can in turn be used to produce pMHC-I multimer libraries that include a plurality different peptides of interest for high throughput applications.

In one aspect provided herein, peptide receptive MHC-I complex multimers (*e.g.*, tetramers) are used to form a peptide-MHC class I (pMHC-I) multimer library that includes pMHC-I multimers having different peptides of interest. Such pMHC-I multimers can be made by contacting aliquots of peptide receptive MHC-I complex multimers with different peptides of interest. In some embodiments, the peptides of interest are different peptides from an antigen of interest. In certain embodiments, the antigen of interest is a tumor antigen.

In some embodiments, pMHC-Is that include the same peptide of interest are attached to backbones to form pMHC-I multimers and the step is performed for a plurality of pMHC-Is that include a library of different peptides of interest. The resulting pMHC-I multimers (*e.g*., tetramers) are subsequently pooled to form the pMHC-I multimer library.

In some embodiments, each of the pMHC-I multimer in the library have the same MHC class I allele. In certain embodiments, the MHC class I is HLA-A. In an exemplary embodiment, the HLA-A is HLA-A*24:02 or HLA-A*68:02.

In some embodiments, the library includes pMHC-I multimers with different MHC class I alleles. In some embodiments, the library includes pMHC-I tetramers.

In certain embodiments, the pMHC-I multimer library includes over 10, 20, 30, 40, 50, 60, 70, 80, 90, 100, 200, 250, 300, 400, 500, 600, 700, 800, 900, 1,000, 1 x 10⁴, 1 x 10⁵, 1 x 10⁶, 1 x 10⁷, or 1 x 10⁸ different peptides. In some embodiments, each pMHC-I multimer in the library includes a detectable marker. In some embodiments, each pMHC-I multimer in the library includes a nucleic acid bar code or a fluorophore that is used to identify peptide of interest included in the pMHC-I multimer, wherein each barcode or fluorophore corresponds to a different peptide of interest. In some embodiments, each of the pMHC-I multimer in the library includes the same detectable label.

### F. Methods of Use

In certain embodiments, the pMHC-I multimer is a pMHC-I tetramer. MHC class I tetramers provided herein can be used to study pathogen immunity, for the development of vaccines, in the evaluation of antitumor response, in allergy monitoring and desensitization studies, and in autoimmunity. *See, e.g.,* Nepom et al., J Immunol 188 (6) 2477-2482 (2012); and Davis et al., Nature Reviews Immunology 11:551-558 (2011).

In some embodiments, the pMHC-I multimers are used to characterize T cell (*e.g*., CD8 T cell) responses to a vaccine, including, but not limited to influenza, yellow fever, tuberculosis, and HIV/SIV vaccines. In an exemplary embodiment, the vaccine is a cancer vaccine. In particular embodiments, the cancer vaccine is melanoma or chronic myeloid leukemia. In such embodiments, a sample (*e.g*., a blood sample) of a vaccinated patient is contacted with one or more of the subject pMHC-I multimers that include one or more peptide of interests derived from the vaccine to identify and monitor antigen specific T cells that are produced in response to the vaccine.

Peptide-MHC class I multimers provided herein can also be used to isolate and enrich particular antigen specific T cells for therapeutic use. *See, e.g.,* Cobbold et al., J. Exp. Med. 202: 379-386 (2006); and Davis et al., Nature Reviews Immunology 11:551-558 (2011). In this particular application, patient samples are contacted with sortable pMHC-I multimers that include a peptide antigen of interest and a label that allows for sorting (*e.g*., a fluorophore or nucleic acid label). Antigen specific T cells that bind the pMHC-I multimer are subsequently isolated and purified, for example, using flow cytometry or similar cell sorting and identification techniques.

In certain embodiments, the peptide-MHC class I multimers provided herein are used for epitope mapping. In this method, a plurality of peptide-MHC class I multimers that include different peptides derived from an antigen of interest (*e.g*., a tumor antigen) are contacted with a sample from a subject. Antigen specific T cells are detected and the corresponding epitope peptide sequences are identified any technique known in the art, include, for example, flow cytometry and cell sorting techniques. *See, e.g.,* Bentzen et al., Nat Biotechnol. 34(10):1037-1045 (2016).

In some embodiments, the peptide-MHC class I multimers provided herein are used to determine a T cell profile of one or more subjects. In such an embodiment, a sample from a subject is contacted with a library of pMHC-I multimers that include a library of peptide of interest and a detectable label. Identification of antigen specific T cells that bind particular peptides of interest presented in the context of the pMHC-I multimers is achieved using the detectable label. The methods described herein allow for the large scale production of pMHC-I multimer libraries that can in turn be used for high throughput T cell profiling.

In another aspect, the pMHC-I multimers are used therapeutically for the targeted elimination of particular antigen specific T cells in a subject. In one embodiment, the pMHC-I multimers are conjugated to a cytotoxic agent or a toxin. When administered to a subject, the pMHC-I multimer conjugates attach to and facilitate the elimination of particular antigen specific T cells.

Peptide-MHC class I multimers used in the methods described herein can be tracked and detected using any suitable techniques including, but not limited to, techniques utilizing detectable labels and nucleic acid barcodes that allow identification of particular peptide-MHC class I multimers. In addition, T cells of interest isolated in such methods can also be identified using similar techniques.

T cells of interest that interact with pMHC-I multimers can be isolated using any suitable technique including, for example, flow cytometry techniques. Isolated T cells and corresponding peptide-MHC class I multimers can then be characterized using any suitable method, for example, the ECCITE-seq method as explained below ((https://www.nature.com/articles/s41592-019-0392-0) in conjunction with 10X Genomics 5P V(D)J kit (https://support.10xgenomics.com/single-cell-vdj/software/vdj-and-gene-expression/latest/overview). This method incorporates a cellular barcode into cDNA generated from both tetramer oligos and TCR mRNA, thus the pairing of cellular barcodes can connect TCR sequences and other mRNAs with pMHC-I multimers specificities.

### EXAMPLES

### Introduction

T cells recognize foreign or aberrant antigens presented by MHC-I expressing cells through the T cell receptor (TCR) and is the first critical step towards establishment of protective immunity against viruses and tumors (Germain & Margulies, Annu Rev Immunol 11:403-450 (1993)). Fluorescently tagged, multivalent MHC class-I reagents (multimers) displaying individual peptides of interest have revolutionized detection of antigen specific T cells (Altman et al., Science 274:94-96 (1996)). Staining with multimers followed by flow cytometry is routinely used to interrogate T cell responses, to characterize antigen-specific TCR repertoires and to identify immunodominant clones (Glanville et al., Nature 547:94 (2017); DeWitt et al., Elife 7:e38358 (2018); and Dash et al., Nature 547:89 (2017)). However, polyclonal repertoires are estimated to contain 10⁵-10⁸ TCRs of distinct antigen specificities (Arstila et al., Science 286:958-961 (1999)). Preparing libraries of properly conformed peptide/MHC-I (pMHC-I) molecules displaying an array of peptide epitopes to probe such repertoires remains a significant challenge, due to the inherent instability of empty (i.e. peptide deficient) MHC-I molecules. To circumvent the problem of unstable peptide deficient MHC-I molecules, conditional MHC class I ligands are used (Toebes et al., Nat Med 12:246-251 (2006)). Conditional ligands, bound to the MHC-I, can be cleaved by exposure to UV light (Bakker et al., Proceedings of the National Academy of Sciences 105:3825-3830 (2008)), or to increased temperature (Luimstra et al., J Exp Med 215, 1493-1504, doi:10.1084/jem.20180156 (2018)). Upon cleavage and in the presence of a peptide of interest, a net exchange occurs where the cleaved conditional ligand dissociates and the peptide of interest associates with the MHC-I, thereby forming the desired pMHC-I complex. Such conditional ligands, however, also have limitations. The use of photo-cleavable peptides necessitates a more elaborate protein purification protocol, and may lead to increased aggregation and sample loss during the peptide exchange step. Dipeptides, which compete with the C-terminus of bound peptides to promote exchange (Saini et al., Pro. Natl Acad. Sci. USA 112:202-207 (2015)), and the stabilization of empty MHC-I molecules using an engineered disulfide bond (Saini et al., Sci Immunol 4, doi:10.1126/sciimmunol.aau9039 (2019)) have been recently proposed as alternatives to catalyze peptide exchange under physiological conditions.

TAPasin Binding Protein Related (TAPBPR) is a chaperone protein homologue of Tapasin involved in the quality control of pMHC-I molecules (Boyle et al., Proc Natl Acad Sci U S A 110:3465-3470 (2013)). TAPBPR associates with MHC-I molecules to edit the repertoire of displayed peptides at the cell surface (Hermann et al., Elife 4:e09617 (2015)). In a similar manner to Tapasin (Chen & Bouvier, The EMBO Journal 26:1681-1690 (2007)), TAPBPR binds several MHC-I alleles *in vitro* to promote the exchange of low- for high-affinity peptides (Morozov et al., Pro. Natl. Acad. Sci. 113:E1006-E1015 (2016)). Using solution NMR, a detailed characterization of the TAPBPR peptide exchange cycle for the murine H-2D^{d} molecule was recently performed (McShan et al., Nat. Chem. Biol. 14:811 (2018)). This work revealed a critical role for N-terminal peptide interactions with the MHC-I peptide-binding groove, which allosterically regulates TAPBPR release from the pMHC-I. Peptide binding is therefore negatively coupled to chaperone release and, conversely, the affinity of incoming peptides for the MHC-I groove is decreased by 100-fold in the presence of TAPBPR molecule (McShan et al., Nature Chemical Biology 14:811 (2018)), due to a widening of the MHC-I groove, as directly observed in X-ray structures of MHC-I/TAPBPR complexes (Jiang et al., Science 358, 1064-1068 (2017); and Thomas & Tampé, Science 358, 1060-1064 (2017)). In follow up work (McShan et al., Proc. Natl Acad. Sci. USA 116:25602-25613 (2019)), the molecular determinants which confer specificity of interactions between TAPBPR and different class-I MHC alleles have been elucidated. This work revealed an approx. 5% "minor" conformation with a widened peptide-binding groove and altered dynamics at the α₃/β₂m interface exhibited by some MHC alleles, which enables binding to TAPBPR, consistently with reports that polymorphisms within the F-pocket of the groove relate to recognition by Tapasin (Rizvi et al., J Immunol 192, 4967-4976 (2014)) and TAPBPR (Ilca et al., Cell Rep 29:1621-1632 (2019)).

These mechanistic insights were leveraged to design conditional ligands for the production of peptide deficient MHC-I/TAPBPR complexes for multiple murine and human MHC-I allotypes, independent of photo-cleavable peptides. Empty MHC-I/TAPBPR complexes are stable for months, can be readily multimerized and loaded with peptides of interest in a high-throughput manner. Focusing on a common human allele, HLA-A*02:01, the capability of the system was extended by incorporating multi-modal cellular indexing technology (ECCITE-seq) (Mimitou et al., Nat Methods 16:409-412 (2019); and Stoeckius et al., Nat Methods 14:865 (2017)). The resulting library of barcoded, TAPBPR exchanged tetramers can be directly applied in a multiplexed analysis of numerous antigen-specificities simultaneously, enabling the identification of TCR V(D)J sequences together with other T cell transcriptional markers of interest in a single cell format (Figure 1). The use of a protein chaperone to accomplish peptide exchange, a task previously addressed using UV-sensitive synthetic peptide ligands (Bakker et al., Proc. Natl Acad. Sci. USA 105:3825-3830 (2008)), *in vitro* translation systems (Zhang et al., Nat Biotechnol, doi:10.1038/nbt.4282 (2018)) or disulfide-linked MHC molecules ((Saini et al., Sci Immunol 4, doi:10.1126/sciimmunol.aau9039 (2019)), leads to clear gains in terms of ease of use, efficiency and sensitivity. Moreover, the indexing design allows a convenient workflow that is fully compatible with a commercially available kit, thereby allowing TCR specificities to be interrogated easily at large scale. Given the central role of T cell responses, this work is of immediate practical relevance to experimental immunologists and cancer biologists, with clear biomedical ramifications toward the development of T cell-based diagnostics and autologous therapies.

### RESULTS

### Isolation of empty MHC-I/TAPBPR complexes using placeholder peptides

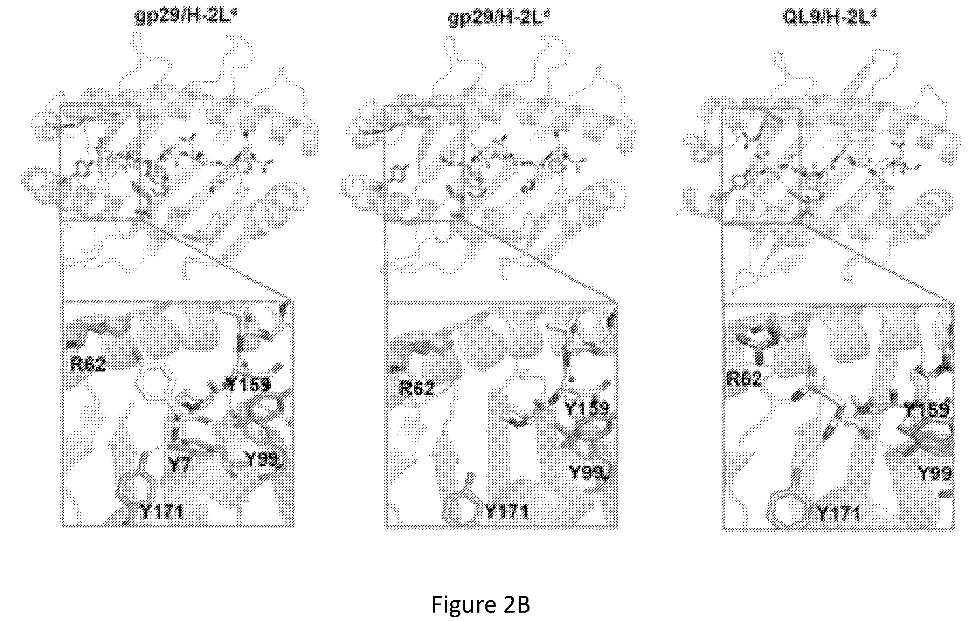

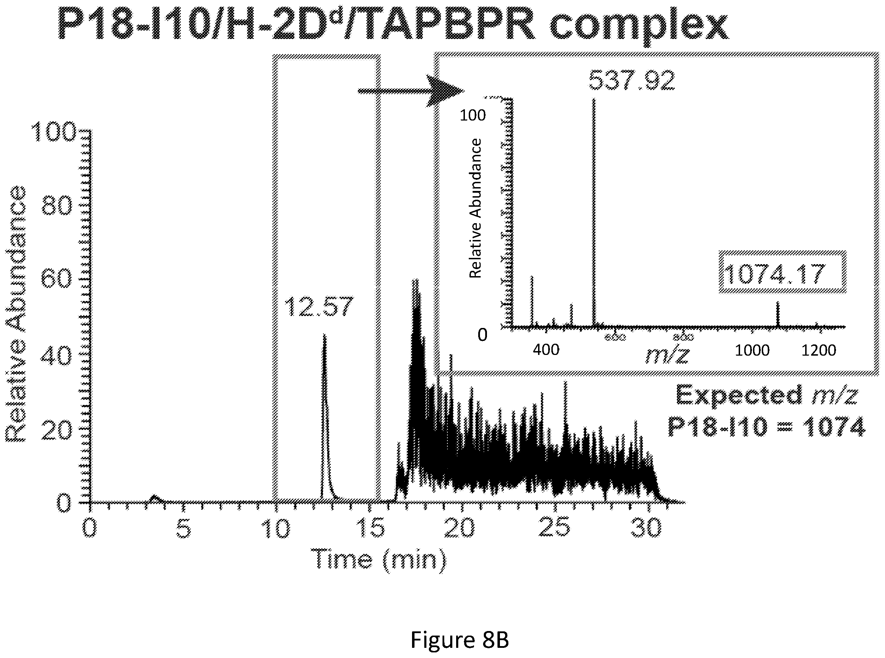

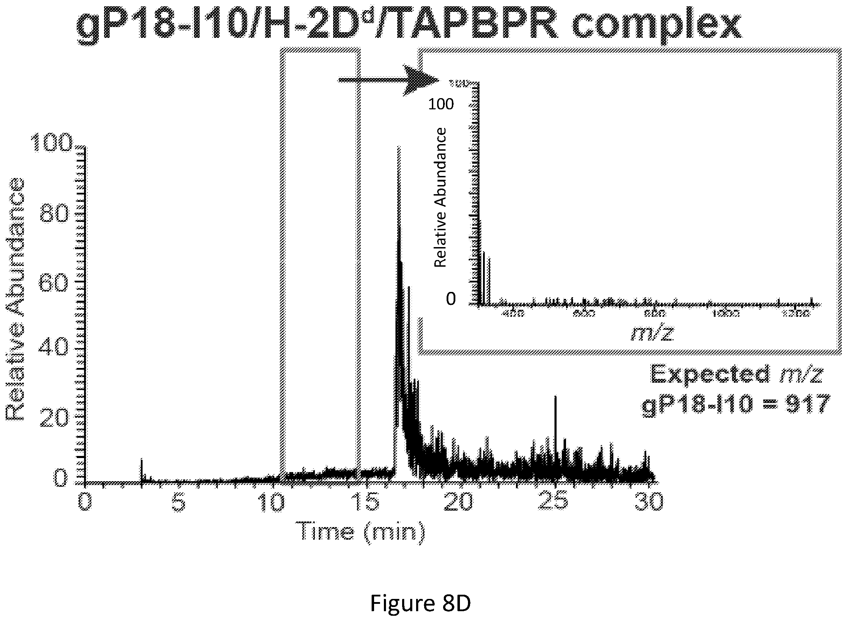

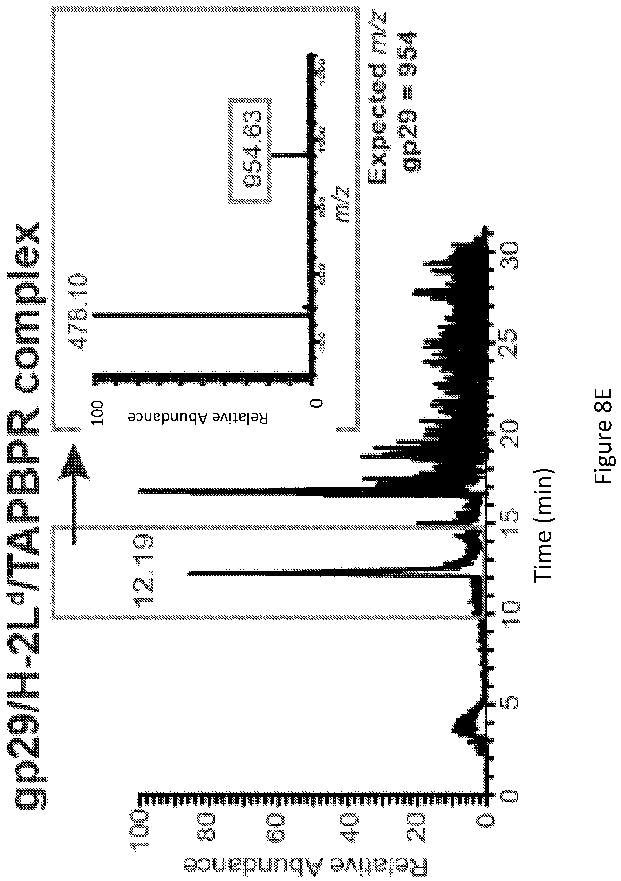

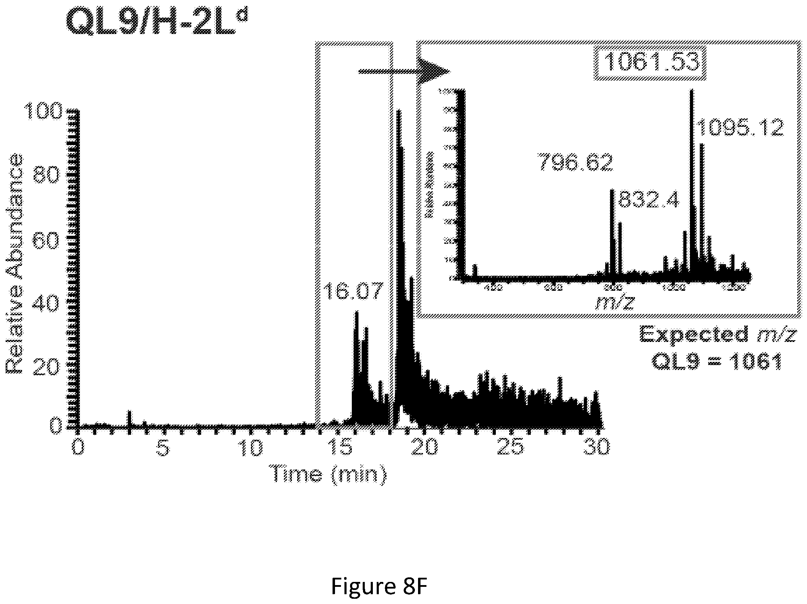

To circumvent the need for photo-cleavable ligands, previously used to demonstrate high-affinity TAPBPR binding to empty MHC-I molecules (Morozov et al., Pro. Natl. Acad. Sci. 113:E1006-E1015 (2016)), the use of destabilizing placeholder peptides was explored. Recently, a destabilizing N-terminally truncated mutant of the P18-I10 peptide _GPGRAFVTI (gP18-I10, Figure 2a) has been described (McShan et al., Nat. Chem. Biol. 14:811 (2018)). gP18-I10 showed a high affinity for a free H-2D^{d} groove during *in vitro* refolding, but dissociated in the presence of TAPBPR to generate stable, empty H-2D^{d}/TAPBR complexes (Figures 8a and d). In contrast, full length P18-I10 remained captured in the groove of the H-2D^{d}/TAPBPR complex (Figures 8a and b). The previously reported 100-fold increase in the peptide off-rate for gP18-I10 relative to full-length P18-I10 peptide (McShan et al., Nat. Chem. Biol. 14:811 (2018)) resulted from the loss of specific polar contacts in the H-2D^{d} A-pocket (Figure 2a), as further reflected in predicted IC₅₀ values (24 µM gP18-I10 vs 23 nM for P18-I10). Therefore, this destabilizing peptide has been termed a goldilocks peptide (gP18-I10). Extending the same concept to a different murine MHC-I molecule, H-2L^{d}, an N-terminal truncation of the high-affinity p29 nonamer _PNVNIHNF (IC₅₀ of 16.5 µM) was tested. However, the resulting 8mer peptide remained bound to the H-2L^{d}/TAPBPR complex (Figures 8a and e, Figure 2b), indicating that truncation of the extreme N-terminal residue cannot be used as a general rule to generate goldilocks peptides (Figure 8e). As p29 fits the typical H-2L^{d} binding motif of xPxx[NA]xx[FLM], QLSPFPFDL (QL9), a predicted low-affinity peptide (IC₅₀ of 9.27 µM) with non-canonical Leu and Phe residues at position 2 and 5, respectively, was explored. Using QL9 as a placeholder peptide, empty H-2L^{d}/TAPBPR complexes were obtained by further adding 10 mM Gly-Phe dipeptide (GF), which promotes peptide release by directly competing for interactions in the F-pocket of the peptide-binding groove (Saini et al., Pro. Natl Acad. Sci. USA 112:202-207 (2015)) (Figures 8f-g, Figure 2b). Taken together, these results establish the principle that destabilization of peptide interactions at both ends of the groove, through a range of approaches, can be used to generate peptide-deficient MHC-I/TAPBPR complexes.

### TAPBPR-mediated peptide loading on disease relevant HLA alleles

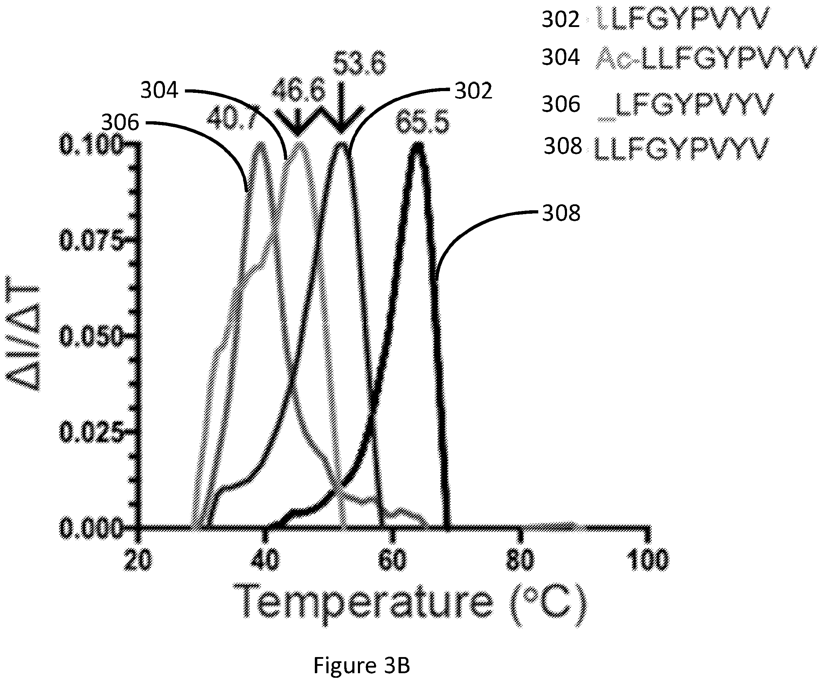

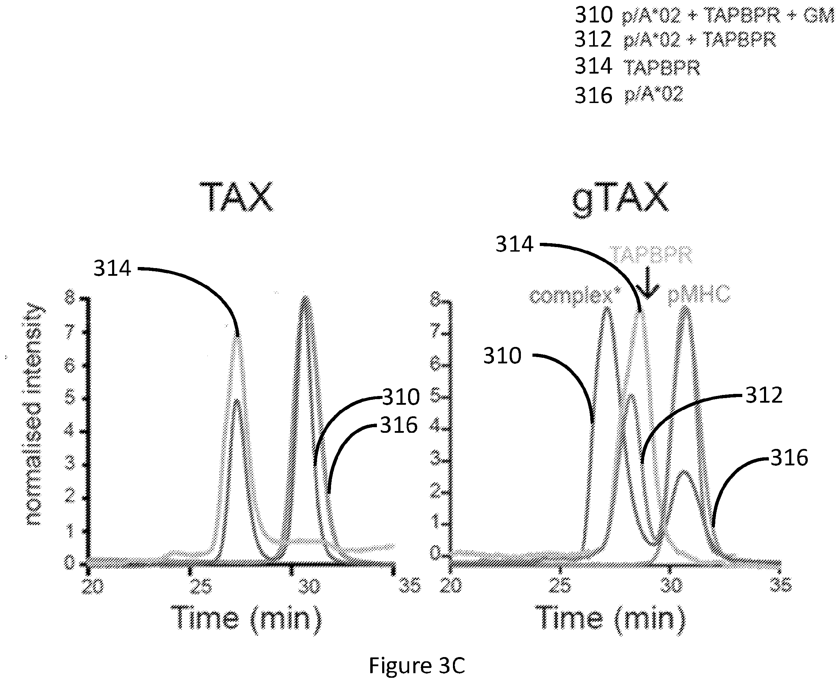

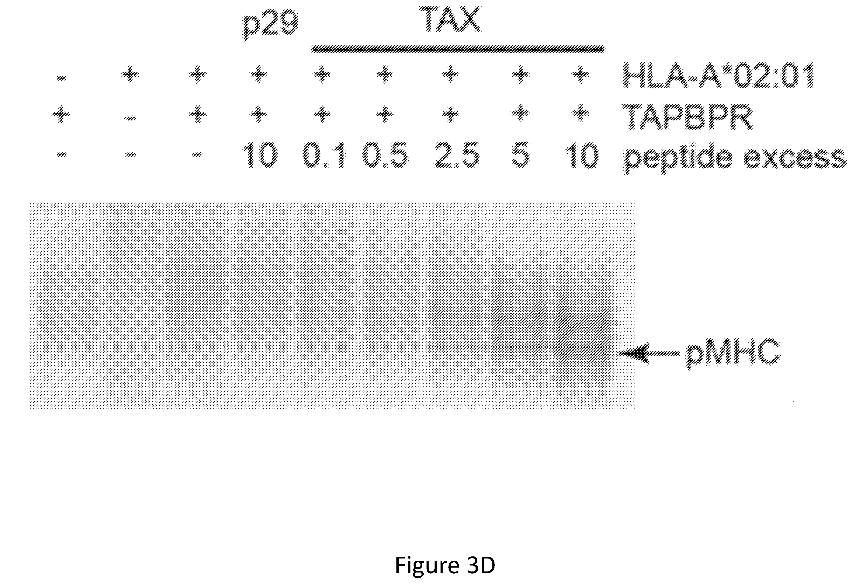

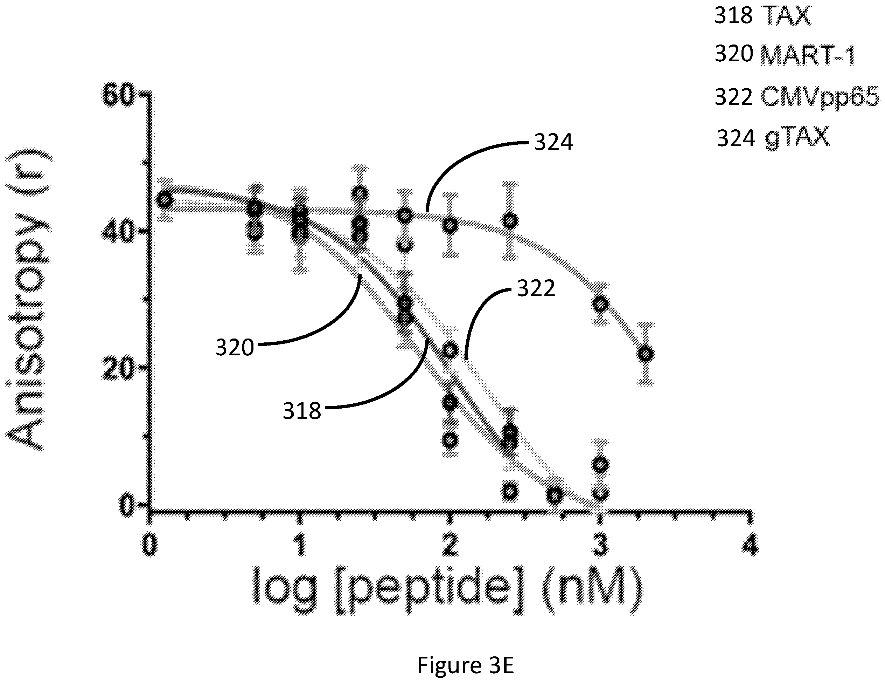

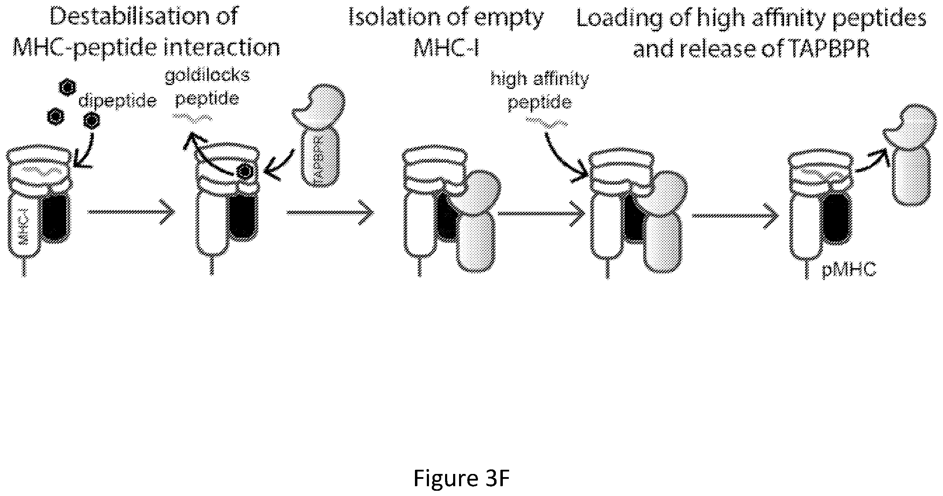

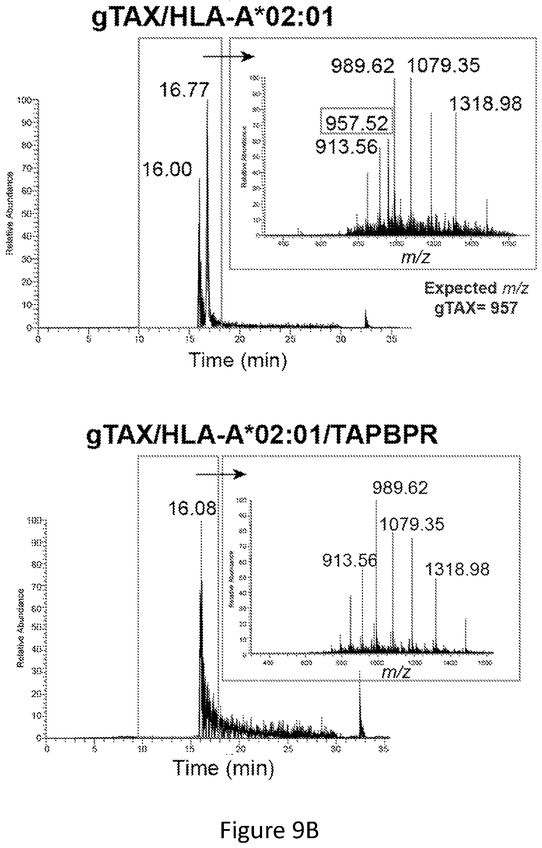

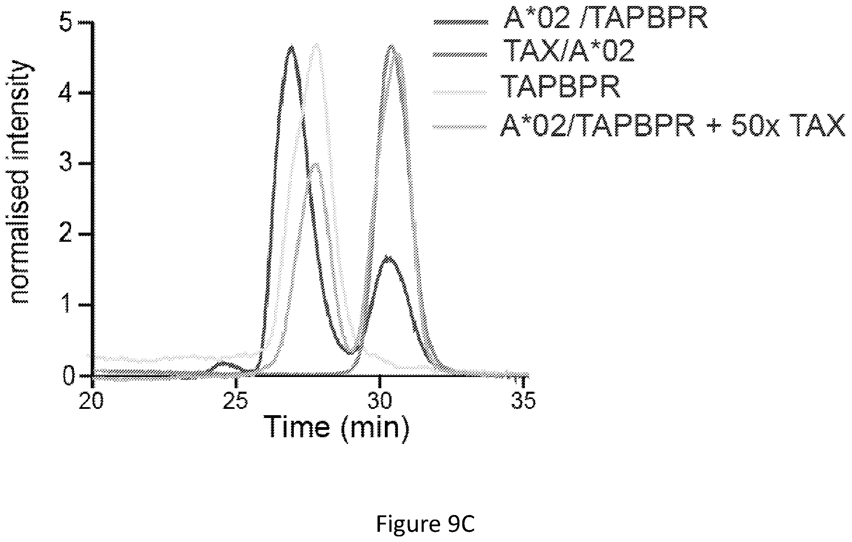

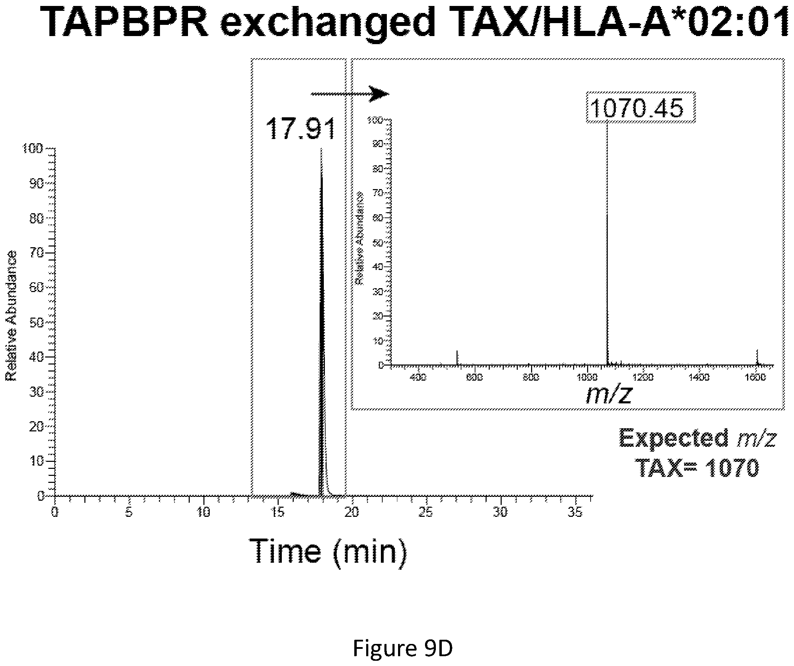

To extend these findings using murine MHC-I alleles towards a high-throughput method for the production of pMHC tetramer libraries for human alleles, focus was placed on the common allele HLA-A*02:01 which displays a wide range of immunodominant viral and tumor epitopes, rendering the study of HLA-A*02:01 restricted responses highly relevant (González-Galarza et al., Nucleic acids Res. 43:D784-D788 (2014)). Guided by the existing TAX/HLA-A*02:01 crystal structures (Khan et al., J. Immunol. 164:6398-6405 (2000)), a number of variants of the LLFGYPVYV (TAX) peptide were designed by progressively reducing N-terminal polar contacts with MHC-I groove residues while maintaining the anchor positions 2 and 8 (x[LM]xxxxx[ILV]) (Figure 3a). Comparison of thermal stabilities (*T*ₘ) of HLA-A*02:01 refolded using N-terminal variants of TAX revealed a progressive reduction in *T*ₘ as a result of destabilization of the peptide complex upon loss of N-terminal polar contacts (Figure 3b). Both gTAX/HLA-A*02:01 and Ac-LLFGYPVYV/HLA-A*02:01, which gave the lowest *T*ₘ values at 40 °C and 46 °C due to loss of polar contacts and steric clashes between the N-terminal acetyl and sidechains of groove residues (Figure 3a), respectively, promoted the formation of peptide-deficient MHC-I/TAPBPR complexes in the presence of 10 mM GM dipeptide (Figures 3c, f and 9a) as shown by liquid chromatography-mass spectrometry (LC-MS) (Figure 9b). In contrast, the variant ILFGYPVYV, where the N-terminal L-Leu was substituted by the D-Leu enantiomer (I) leading to suboptimal packing interactions in the A-pocket (Figure 3a) and consequently a reduced *T*ₘ value of 54 °C (Figure 3b), did not promote the formation of a stable complex with TAPBPR. The stability of empty HLA-A*02:01/TAPBPR complexes was examined by SDS-PAGE gel, to find that they remained intact for up to 6 months at -80 °C, without compromising their performance in peptide exchange reactions. Incubation with 10-fold molar excess of high-affinity TAX peptide induced dissociation of the complex, as observed both by native gel and size-exclusion chromatography (SEC) assays (Figures 3d and 9c), with the loaded peptide detectable by LC-MS (Figure 9d). Accordingly, high-affinity peptides (TAX, CMVpp65 or MART-1) could be readily loaded into the empty complex, out competing fluorescently labelled TAMRA-TAX for HLA-A*02:01 binding (Figure 3e). Using Bio-layer interferometry, the TAPBPR dissociation rate was measured from HLA-A*02:01, which showed a significant increase in the presence of high-affinity peptides (TAX, CMVpp65 or MART-1), compared to an irrelevant peptide, used as a negative control (p29) (Fig. 3g).

Using the TAX/HLA-A*02:01 system as a benchmark, it was determined that the overall yield of pMHC molecules prepared by TAPBPR exchange is approx. 2.5 times higher relative to the use of a photo-cleavable ligand (Bakker et al., Proc. Natl Acad. Sci. 105:3825-3830 (2008)), and approx. 10 times higher relative to the use of an empty HLA-A*02:01 molecule with an engineered disulfide bond (Saini et al., Sci Immunol 4, doi:10.1126/sciimmunol.aau9039 (2019)) (Figure 20). An additional benefit of these method is that the MHC-I/TAPBPR complexes are stable for several weeks at 4 °C, while their preparation and handling does not require dark conditions, as is the case for MHC molecules refolded with photo-cleavable ligands (Bakker et al., Proc. Natl Acad. Sci. 105:3825-3830 (2008)). Using a similar approach, suitable goldilocks peptides were designed for the disease-relevant HLA-A*24:02 and HLA-A*68:02 alleles (Figure 4a), and demonstrated exchange of the goldilocks for high affinity peptides in the presence of TAPBPR by native gel (Figure 4b) and by differential scanning fluorimetry experiments (Figure 4c). Several additional classical HLA alleles (llca, et al., Cell Rep 29:1621-1632 (2019)) are likely amenable to TAPBPR-mediated peptide exchange using these method.

### Loading high affinity peptides using catalytic amounts of TAPBPR

In some applications, purification of empty, stoichiometric MHC-I/TAPBPR complexes can be a limiting step. On the other hand, refolded goldilocks/MHC complexes can be aliquoted and stored at - 80°C for several months. To evaluate whether exchange of goldilocks for high affinity peptides can occur when TAPBPR is offered in sub-stoichiometric concentrations, a native gel electrophoresis assay was used. This allowed for the monitoring of the formation of different pMHC species upon overnight incubation in the presence of 10-fold molar excess of different peptides and varying molar ratios of TAPBPR (Figure 10a). Under these conditions, complete peptide exchange on gTAX/HLA-A*02:01 was obtained using down to 1:1000 TAPBPR:MHC molar ratio, while no exchange was observed for a non-specific peptide, or in the absence of TAPBPR. In this assay the electrophoretic mobility of different pMHC molecules is dependent on the net charge of the bound peptide, which allowed for the resolving of distinct protein bands of HLA-A*02:01 loaded with peptides of disparate charges from -2 to +1 in overnight catalytic peptide exchange reactions (Figure 10b). Taken together, these results highlight the use of TAPBPR as a peptide exchange catalyst, which can be advantageous for high throughput applications.

### T cell staining using TAPBPR-exchanged pMHC molecules

The performance of TAPBPR-exchanged phycoerythrin (PE)-tetramers were the compared, relative to PE-tetramers refolded *in vitro* using standard protocols(Garboczi et al., Proc. Natl Acad. Sci. USA 89:3429-3433 (1992)). Staining of a B4.2.3 TCR transgenic T cell line (Natarajan et al., Nature communications 8, 15260 (2017)) with 1µg/ml of TAPBPR-exchanged P18-110/H-2D^{d} tetramers prepared using either a stoichiometric (1:1) or catalytic (1:100) TAPBPR:MHC ratio, showed an identical population of tetramer positive cells relative to refolded pMHC-I tetramers. No staining was observed using an irrelevant motif peptide (sequence AGPARAAAL), which binds with high affinity to H-2D^{d} but is not recognized by the B4.2.3 TCR (Figure 5a, b). The staining efficiency was further quantified using tetramer titrations, which showed a staining saturation curve with an EC₅₀ of 0.16 µg/mL compared to 0.44 µg/mL for refolded TAPBPR-exchanged tetramers, respectively (Figure 5c, with gating strategy shown in Figure 11). The 2.5-fold reduced staining efficiency can be attributed to sample loss due to the formation of aggregation prone, peptide-deficient H-2D^{d} molecules during the overnight incubation with peptide. However, given that the observed EC₅₀ value remains well below the standard tetramer staining concentration of 1µg/ml, TAPBPR exchange can still be used to obtain reliable staining results with the added benefit that, in contrast to refolding, TAPBPR exchange can be performed at high-throughput for a library of peptides (see below). A similar trend was observed in MART-1/HLA-A*02:01 tetramer staining of Jurkat/MA T cells transduced with a MART-1 specific TCR, DMF5 (Figure 5a, b - middle), and in NY-ESO-1/HLA-A*02:01 tetramer staining of T cells transduced with a NY-ESO-1 specific TCR (Johnson et al., J. Immunol. 177:6548-6559 (2006)) (Figure 5a and b -bottom). Here, TAPBPR exchanged tetramers showed identical EC₅₀ values relative to refolded tetramers, highlighting the robustness of TAPBPR exchange for the preparation of human pMHC-I molecules in a high-throughput setting (Figure 5c middle and bottom). No staining was observed using tetramers prepared by exchanging the mismatched peptides, NY-ESO-1 and MART-1 on T cells expressing the DMF5 and NY-ESO-1 TCRs, respectively (Figures 5a and b).

### Monitoring peptide loading through the release of TAPBPR

Since TAPBPR has been shown to promote the release of high affinity peptides from the MHC groove *in vitro* (McShan et al., Nat. Chem. Biol. 14:811 (2018)), the persistence of TAPBPR following exchange may partially regenerate empty MHC-I molecules, thereby reducing the staining efficiency of the resulting tetramers. In a library format this may also lead to scrambling of excess peptides between MHC tetramers upon mixing, which would limit the use of molecular indices or barcodes to label tetramers according to their displayed peptides (see below). Specifically, the presence of TAPBPR induced the exchange of CMVpp65 loaded on HLA-A*02:01 tetramers for free MART-1 peptide, which can be detected by staining of DMF5+ T cells (Figure 12b). To prevent this, full removal of TAPBPR molecules was readily achieved by spin column dialysis, immediately following the tetramerization and peptide loading steps as confirmed by SDS-PAGE (Figure 1 and Figure 12a). The resulting pMHC-I tetramers did not capture free high affinity peptides, even when present at high (20x) molar excess, and peptide exchange between tetramers was undetectable by flow cytometry (Figures 12b and c).

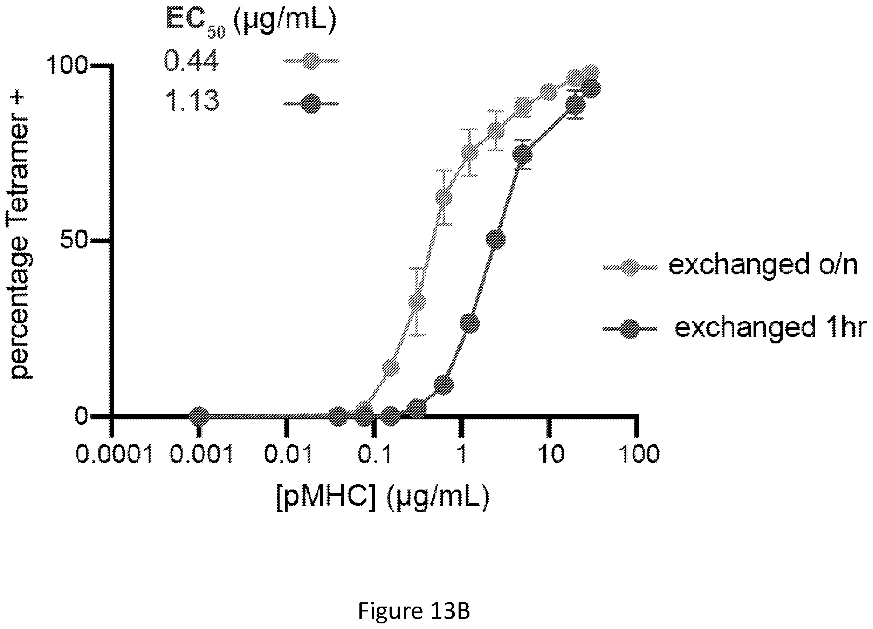

Complete binding of high affinity peptides on empty, tetramerized MHC-I/TAPBPR complexes can be monitored using a simple SDS-PAGE assay, through the disappearance of a distinct TAPBPR band following the spin column dialysis step. Here, TAPBPR is used in a similar manner to a conformation-specific antibody to label empty MHC-I molecules present after the peptide exchange reaction. Using this assay, it was found that TAPBPR remained present on H-2D^{d} tetramers following 1 h incubation with P18-I10 peptide, despite exhaustive spin column dialysis, due to the slow binding of peptide on empty H-2D^{d}/TAPBPR molecules (McShan et al., Nat. Chem. Biol. 14:811 (2018)) (Figure 13a). As a result, H-2D^{d} tetramers prepared upon incomplete loading with peptide show a 2.5 times higher EC₅₀ value relative to those obtained after complete peptide exchange, which can be readily achieved in an overnight peptide loading reaction, as confirmed by gel assay (Figure 13b). Thus, an overnight incubation with peptide is a requirement for TAPBPR-mediated exchange on the murine MHC-I molecules tested here.

### Fine-tuning interactions with TAPBPR through α3 domain mutants

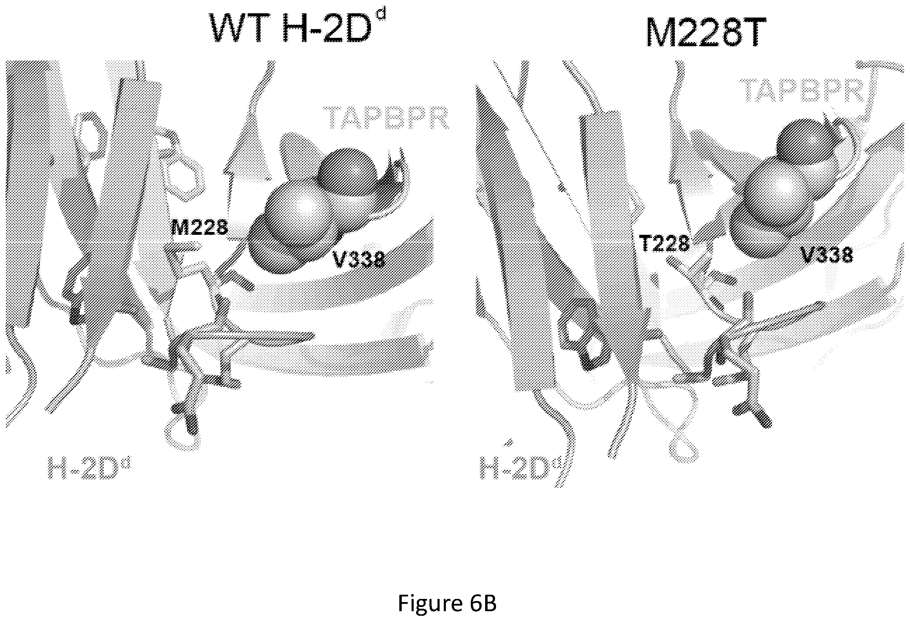

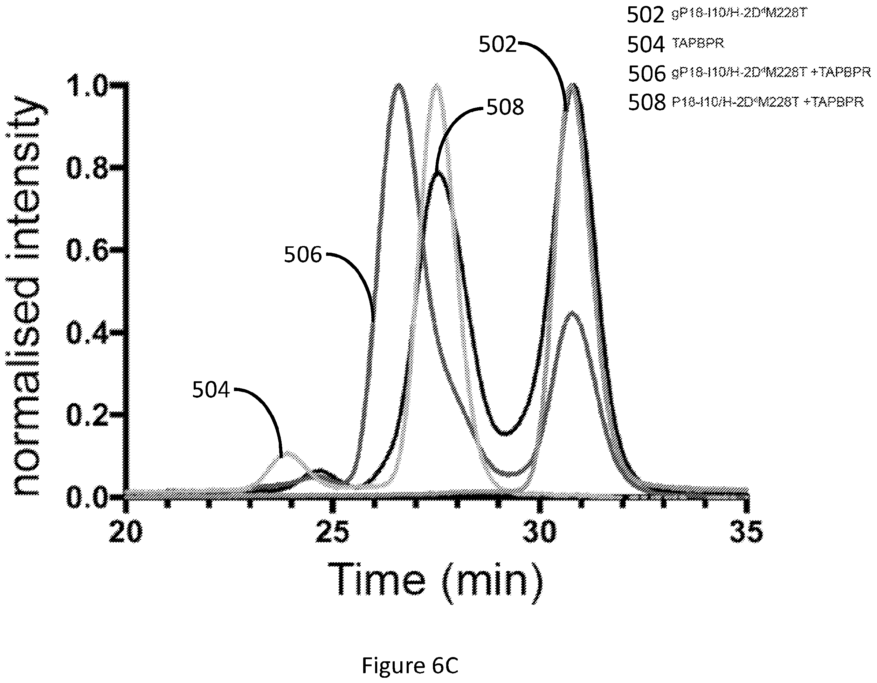

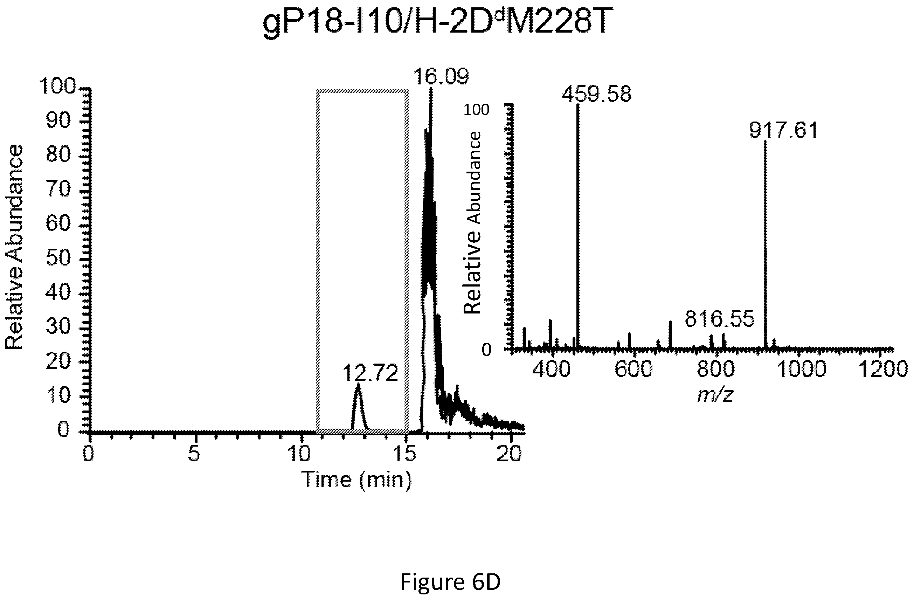

Next, studies were preformed to fine-tune TAPBPR interactions with murine MHC-I molecules previously shown to form stable complexes with TAPBPR even when bound to high affinity peptides (McShan et al., Nat. Chem. Biol. 14:811 (2018)), an effect which limits their use towards high-throughput tetramer library production due to persistent peptide exchange activity in the pooled library. Towards reducing the affinity of TAPBPR for murine molecules, designed mutations at the α₃ domain of the heavy chain were explored, which participates in direct interactions with TAPBPR, and does not contribute to the formation of the peptide binding groove. Specifically, Met 228 is located at an edge loop of the α₃ immunoglobulin fold and forms a hydrophobic contact with TAPBPR residues in the X-ray structure of the H-2D^{d} /TAPBPR complex (Jiang et al., Science 358, 1064-1068 (2017)) (Figure 6a, b). It was hypothesized that a mutation at this position from a Met, present in H-2D^{d} and H-2L^{d}, to a polar Thr residue, present in HLA-A*02:01, would lead to a reduced binding affinity of peptide-bound MHC-I molecules for TAPBPR. In contrast to the WT molecule, H-2D^{d}M228T did not bind TAPBPR when the high affinity P18-I10 peptide was present in the MHC-I groove. Notably, TAPBPR maintained binding to empty H-2D^{d}M228T upon dissociation of the goldilocks gP18-I10 peptide, to generate a peptide-receptive complex (Figure 6c, d). These results highlight the requirements of a system that is amenable to large-scale tetramer library production using an approach that entails: i) formation of a stable TAPBPR complex with an empty molecule and ii) TAPBPR dissociation upon binding of high affinity peptides to the MHC-I groove.

### Labelling individual peptide specificities with DNA "barcodes"

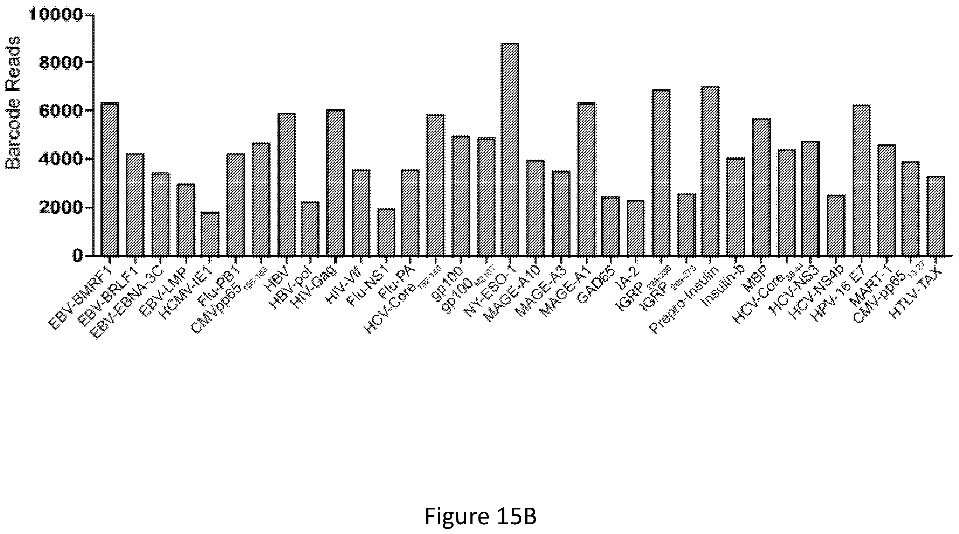

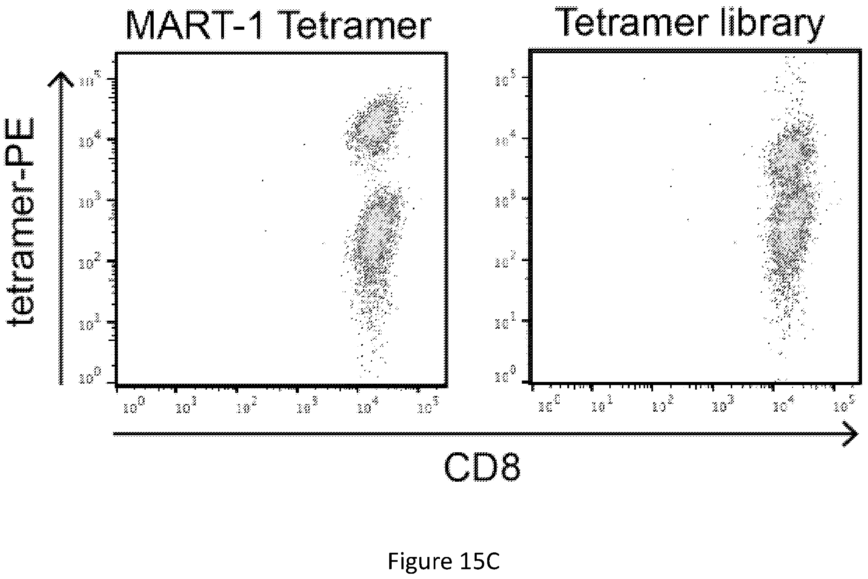

To test the above method on a high-throughput setting, two HLA-A*02:01 tetramer libraries, one containing 29 tumor epitopes identified in neuroblastoma tissues and another encompassing a range of 31 viral, neoantigen and autoimmune epitopes (Figures 21 and 22). To link pMHC specificities with TCR V(D)J sequences present in polyclonal samples fluorophore-labelled pMHC tetramers were barcoded, prepared using TAPBPR exchange, with biotinylated DNA oligonucleotides (oligos) (Bentzen et al., Nat. Biotechnol. 34:1037 (2016)) at a 1:1 molar ratio (DNA oligos:pMHC). Using tetramer staining titrations on DMF5 reporter cells, it was determined that the barcoded tetramers show a similar EC₅₀ value relative to the non-barcoded control (Figure 14). An in-house oligo design compatible with 10x Genomics gel bead oligos in the 5' V(D)J product was used(Figure 15a), adding an additional modality of cellular information to a recently described ECCITE-seq method (Mimitou et al., Nat. Methods 16:409-412 (2019)). This method incorporates a cellular barcode into cDNA generated from both tetramer oligos and cellular mRNAs, including TCR transcripts, thus connecting TCR sequences and other mRNAs with tetramer specificities. Formation of stable pMHC species upon loading of each peptide can be detected at high throughput using two complementary differential scanning Fluorimetry (DSF) assays (Figure 16). Together with the SDS-PAGE (Figures 12a and 13a) and fluorescence/Bio-layer interferometry assays (Figures 3e, g and 17), the DSF data provide an additional way to confirm binding of a candidate peptide to the empty MHC-I groove, and can be performed on a 96-well format. After exhaustive dialysis of TAPBPR, excess barcodes and peptides, all individual peptide loading reactions were pooled into a single tetramer library sample for staining. Both final libraries prepared in this manner were further validated for: (i) the presence of all barcodes, using bulk sequencing reactions (Figure 15b) and (ii) staining of Jurkat/MA cells transduced with the DMF5 receptor specific to the MART-1 reference peptide included in each library. Here, the observed signal intensities (10³-10⁴) were sufficient to distinguish the approximately 33% population of DMF5 positive cells from the negative fraction (Figure 15c).

### Probing polyclonal TCR repertoires using barcoded pMHC libraries

To confirm that the above approach has sufficient sensitivity to detect antigen-specific T cells within a heterogeneous sample, 1% DMF5 Jurkat/MA T cells were spiked into a sample of CD8-enriched PBMCs co-cultured with dendritic cells and stained with the library of 31 neoepitopes, including the MART-1 peptide. 100,000 tetramer positive cells were collected and 3,000 were sequenced. From this sparse sample, 256 cells with the highest number of DMF5 TCR reads were extracted and analyzed together with their corresponding MART-1 tetramer reads. In total, 85 cells with ≥10 MART-1 tetramer counts were recovered, 76 of which showed ≥ 10 DMF5 TCR reads (considered DMF5 positive), giving an approximate false positive rate of 10.6% (Figure 7a). Conversely, 6 DMF5 positive cells showed ≤ 10 MART-1 tetramer counts, resulting in a false negative rate of 7.9%. The low number of cells with significant tetramer barcode reads could be a function of tetramer avidity, exacerbated by high dilutions through the 10x Genomics system prior to cDNA preparation. This analysis shows that the discrimination between antigen-specific and non-specific T cells present in a mixed sample can be accomplished on the basis of tetramer barcode reads with high confidence (p-value < 0.0001 using a two-tailed Mann-Whitney test), suggesting that tetramer libraries prepared by TAPBPR-mediated exchange can be used to identify sparse populations of antigen-specific T cells within a heterogeneous sample.

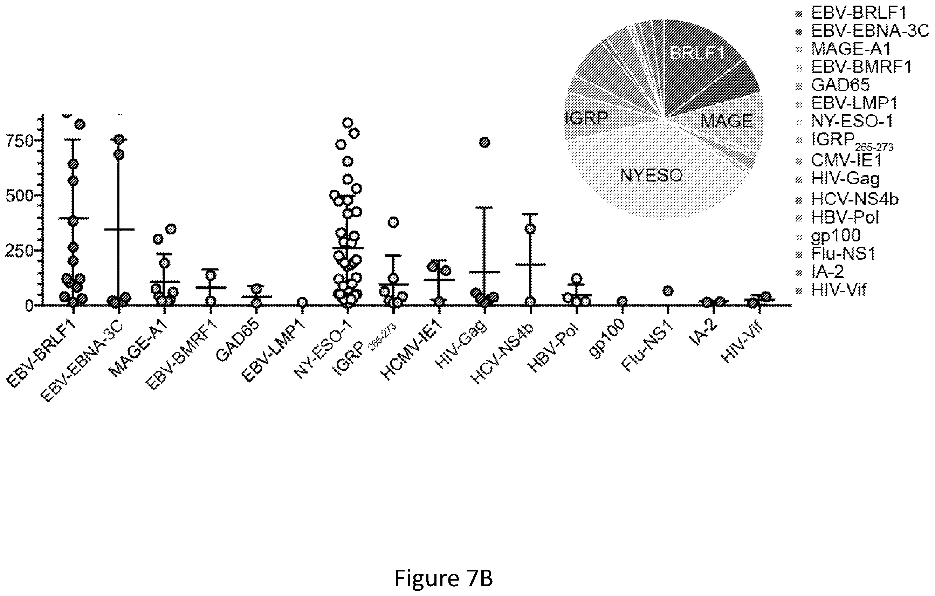

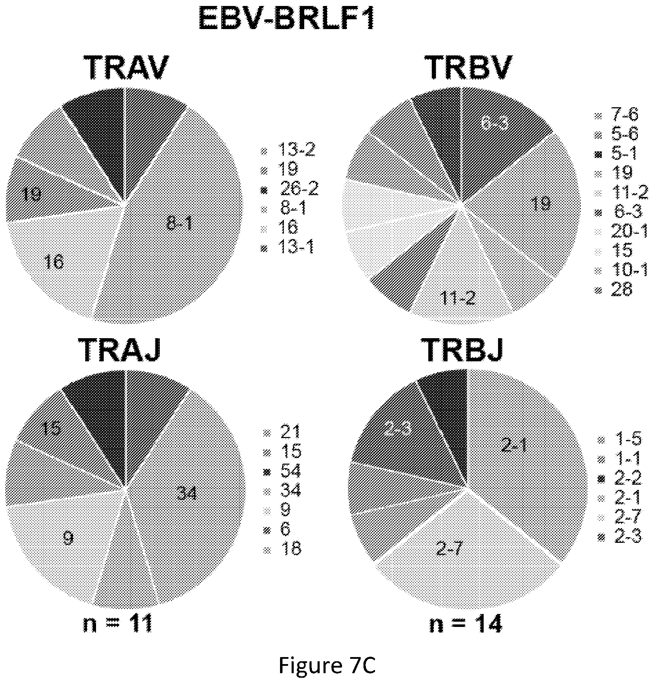

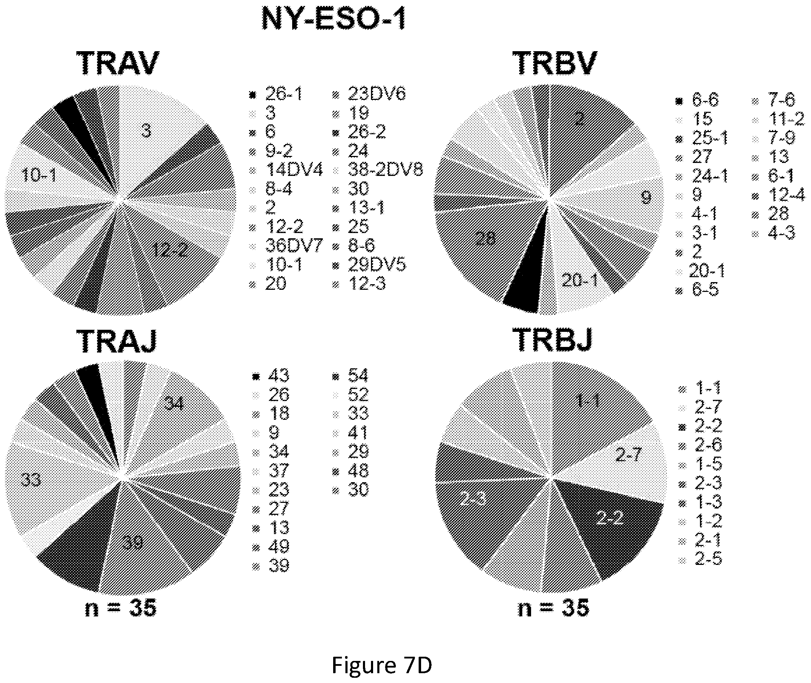

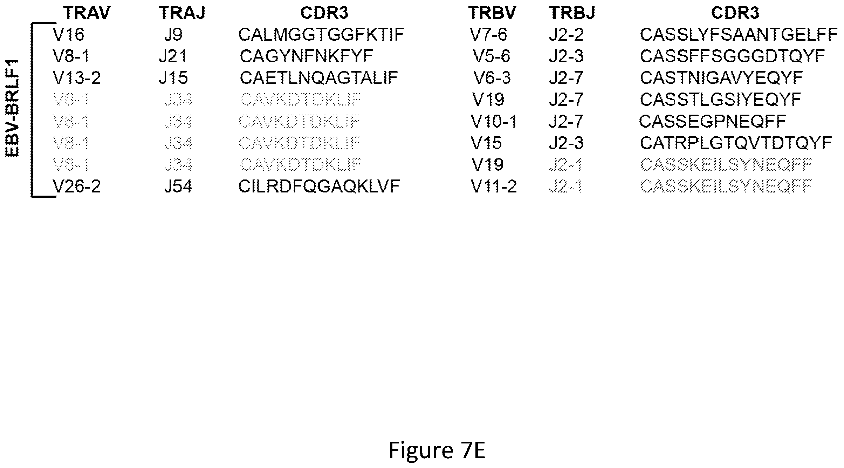

Finally, the above methodology was used to probe distinct TCR specificities present in a polyclonal repertoire of T cells. A total of 2 x 10⁶ CD8⁺ T cells isolated from the spleen of an EBV-positive donor were stained using a barcoded tetramer library consisting of 34 viral, autoimmune and tumor epitopes (Figure 22). After sorting (gating strategy shown in Figure 18), 3,000 tetramer positive cells were loaded on the 10x platform for single-cell sequencing. ECCITE-seq analysis retrieved 1,722 cell barcodes, the majority of which were associated with < 2 tetramer reads, giving a low apparent background. 110 cells were further identified as tetramer-enriched, defined as cells with >10 tetramer reads for at least one peptide specificity. 27 tetramer-enriched cells bound to >5 different peptide epitopes, 5 of which showed no bias towards a particular epitope and were excluded from subsequent analysis. Among the final set of 102 tetramer-enriched cells, a total of 16 distinct epitope specificities were identified, with an average of 200 reads per cell for each dominant tetramer (Figure 7b). Specifically, a large fraction of tetramer-enriched cells had high reactivity for the NY-ESO-1 epitope (37 cells), followed by EBV-BRLF1 (16 cells), MAGE-A1 (10 cells) and IGRP₂₆₅₋₂₇₃ (7 cells). Towards validating the significance of these results, barcodes corresponding to the EBV-BRLF1 epitope (Figure 7c) whose TCR repertoire has been previously characterized using *ad hoc* tetramers prepared using conventional refolding protocols were the focus (Trautmann et al., Eur. J. Immunol. 32:3181-3190 (2002)). Inspection of V(D)J TCR sequence reads from tetramer-enriched cells identified a clear bias towards the usage of TRAV8-1 (50%) with TRAJ34 (50%), TRBV19 (21%), TRBJ2-1 (36%) and TRBJ2-7 (29%), a finding consistent with published reports on EBV-BRLF1 specific repertoires (Koning, et al., J. Immunol. 190:931-939 (2013)). Whereas the β-chain CDR3 sequences were more variable, analysis of TRA CDR3 sequences further identified a known public α-chain CDR3 (CAVKDTDKLIF) which was previously linked to a functional TCR, specific for the EBV-BRLF1 epitope (Bentzen et al., Nature Biotechnology 34(1): 1037-1045 (2016)) (Figure 7e). This sequence was observed in half of EBV-BRLF1 tetramer-enriched cells, and was not detected in cells enriched for any other tetramer. The TCR repertoire from 37 T cells with high barcode reads corresponding to the heteroclitic NY-ESO-1 epitope (SLLMWITQA) were also analyzed. Here, the TCR α- and β-chain TCR sequences were more diverse, with some biases for TRBV28 (15%) and TRBJ2-7 (8%) usage (Figure 7d). The observed diversity in TCR CDR3 regions that recognize NY-ESO-1 tetramers are consistent with the known high cross-reactivity of this epitope (Valmori et al., Cancer Res 60:4499-4506 (2000)), and provide clues for engineering more exclusive TCRs for immunotherapy applications (Bethune et al., Proc. Natl Acad. Sci. USA 115:E10702-E10711, doi:10.1073/pnas.1810653115 (2018)) (Figure 7e). Taken together, these results corroborate the initial findings using the spiked DMF5/PBMC sample (Figure 7a), and further suggest that the barcoded pMHC libraries prepared using TAPBPR exchange are of sufficient quality and staining efficiency to identify antigen-specific TCRs present within polyclonal repertoires.

### DISCUSSION

A robust method has been outlined to isolate stable, empty MHC-I molecules at milligram quantities, that can be readily loaded with peptides of choice in a high-throughput manner. Rather than relying on chemically synthesized conditional ligands, this approach employs a molecular chaperone, TAPBPR, in an analogous manner to the antigen processing pathway used by cells to load MHC-I molecules with immunodominant peptides (Germain & Margulies, Annu Rev Immunol 11:403-450 (1993)). In combination with a simple indexing design that is compatible with the 10x Genomics system, this approach can efficiently link paired TCR V(D)J sequences and other transcription markers to their pMHC specificities in a polyclonal sample setting. The peptide exchange and barcode sequencing workflow has no theoretical upper limit with respect to library size, other than the cost of peptide and oligo synthesis, which renders it suitable for the simultaneous analysis of hundreds of epitope specificities in future experiments. In addition to expediting tetramer library preparation and the identification of novel TCR specificities, the described method can be readily extended to include the analysis of complete transcriptomes (Mimitou et al., Nat Methods 16:409-412 (2019); and Stoeckius et al., Nat Methods 14:865 (2017)), thereby providing a toehold for functional studies of TCR:pMHC recognition.

### METHODS

### Peptide sequences

All peptide sequences are given as standard single letter code. Peptides used for MHC refoldings and production of the neoepitope library were purchased from Genscript at 98% purity or as pepsets from Mimotopes as crude peptides and dissolved in 8.25% Acetonitrile, 25% DMSO, and 66.75% H₂O. Peptides containing modifications: TAMRA-TAX and GILGFVFXL (where X = 3-amino-3-(2-nitrophenyl)-propionic acid) were purchased from Biopeptik at 98% purity. ILFGYPVYV and Ac-LLFGYPVYV were synthesized in house using standard FMOC chemistry. Peptide binding affinities were predicted using netMHCpan 4.0 (Jurtz et al., J. Immunol. 199:3360-3368 (2017)).

### In vitro refolding of pMHC molecules

Plasmid DNA encoding the luminal domain of class I MHC (MHC-I) heavy chains H-2D^{d}, HLA-A*02:01, H-2L^{d} and human β2-microglobulin (hβ₂m,) were provided by the tetramer facility (Emory University), and transformed into *Escherichia coli* BL21(*DE3)* (Novagen). MHC-I proteins were expressed in Luria-Broth media, and inclusion bodies (IBs) were purified using standard protocols (Garboczi et al., Proc. Natl Acad. Sci. USA 89:3429-3433 (1992)). *In vitro* refolding of pMHC-I molecules was performed by slowly diluting a 200 mg mixture of MHC-I and hβ2m at a 1:3 molar ratio over 24 hours in refolding buffer (0.4 M L-Arginine, 100 mM Tris pH 8, 2 mM EDTA, 4.9 mM reduced glutathione, 0.57 mM oxidized glutathione) containing 10 mg of synthetic peptide purchased from Genscript at 98% purity at 4 °C. H-2D^{d} heavy chain was refolded with RGPGRAFVTI (P18-I10) derived from HIV gp120 (Li, et al., J. Mol. Biol. 283, 179-191 (1998)) or _GPGRAFVTI (gP18-I10). H-2L^{d} was refolded with _PNVNIHNF (gp29) or QLSPFPFDL (QL9) derived from oxo-2-gluterate dehydrogenase. HLA-A*02:01 was refolded with variants of LLFGYPVYV (TAX) derived from HTLV-1 including _LFGYPVYV (gTAX), N-terminally acetylated TAX (Ac-LLFGYPVYV), lLFGYPVYV where the first residue is a D-leucine or with ELAGIGILTV (MART-1) derived from Melan-A. Refolds were allowed to proceed for 96 h followed by size exclusion chromatography (SEC) using a HiLoad 16/600 Superdex 75 column (150 mM NaCl, 25mM Tris pH 8) at a flow rate of 1 mL/min, followed by anion exchange chromatography on a mono Q 5/50 GL column at 1 mL/min using a 40 minute 0-100% gradient of buffer A (50 mM NaCl, 25mM Tris pH 8) and buffer B (1M NaCl, 25mM Tris pH 8). Typical protein yields from a 1L refold were 5 to 10 mg of purified pMHC-I.

### Recombinant TAPBPR expression and purification

The luminal domain of TAPBPR was expressed using a stable *Drosophila* S2 cell line (Dr Kannan Natarajan, National Institutes of Health) induced with 1 mM CuSO₄ for 4 days and purified using affinity-based and size exclusion chromatography ¹⁷. Briefly, His₆-tagged TAPBPR was captured from the supernatant by affinity chromatography using high-density metal affinity agarose resin (ABT, Madrid). Eluted TAPBPR was further purified by size exclusion using a Superdex 200 10/300 increase column at a flow rate of 0.5 mL/min in 100 mM NaCl and 20 mM sodium phosphate pH 7.2.

### Size exclusion chromatography of MHC-I/TAPBPR complexes

SEC analysis of MHC-I/TAPBPR interaction was performed by incubating 40 µM purified pMHC-I molecules with purified TAPBPR at a 1:1 molar ratio in 100 mM NaCl, 20 mM sodium phosphate pH 7.2 for 1 h at room temperature. Complexes were resolved on an Superdex 200 10/300 increase column (GE healthcare) at a flow rate of 0.5 mL/min in 100 mM NaCl and 20 mM sodium phosphate pH 7.2 at room temperature. MHC-I/TAPBPR complexes eluted at 26.5 min. In the case of H-2L^{d} and HLA-A*02:01, 10 mM GF and GM were added respectively both to the initial incubation and to the running buffer during chromatography.

### LC-MS analysis

Peptide occupancy of SEC purified MHC-I was determined by HPLC separation on a Higgins PROTO300 C4 column (5 µm, 100 mm x 21 mm) followed by electrospray ionisation performed on a Thermo Finnigan LC/MS/MS (LQT) instrument. Peptides were identified by extracting expected m/z ions from the chromatogram and deconvoluting the resulting spectrum in MagTran.

### Preparation of photo-exchanged pMHC-I

H-2D^{d} refolded with RGPGRAFJ*TI (photo-P18-I10) and HLA-A*02:01 refolded with GILGFVFJ*L, where J* is the photo-cleavable residue 3-amino-3-(2-nitrophenyl)-propionic acid, were UV-irradiated at 365 nm for 1 h in the presence of 20-fold molar excess peptide at room temperature. Reactions were iced for 1 h then centrifugated at 14, 000 rpm for 10 min to remove aggregates. Photo-exchanged pMHC-I was then used for DSF analysis or tetramer preparation.

### Differential scanning Fluorimetry (DSF)

To measure thermal stability of pMHC-I molecules (Hellman et al., J Immunol Methods 432:95-101 (2016)), 2.5 µM of protein was mixed with 10 x Sypro Orange dye in matched buffers (20 mM sodium phosphate pH 7.2, 100 mM NaCl) in MicroAmp Fast 96 well plates (Applied Biosystems) at a final volume of 50 µL. DSF was performed using an Applied Biosystems ViiA qPCR machine with excitation and emission wavelengths at 470 nm and 569 nm respectively. Thermal stability was measured by increasing the temperature from 25 ºC to 95 ºC at a scan rate of 1 ºC/min. Melting temperatures (Tₘ) were calculated in GraphPad Prism 7 by plotting the first derivative of each melt curve and taking the peak as the Tₘ (Figure 17a). Determination of Tₘ values of TAPBPR exchanged molecules additionally required subtraction of the TAPBPR melt curve from the curve obtained for the complex, then calculating the first derivative. This procedure, on average, enhanced the Tₘ values calculated for TAPBPR exchanged pMHC-I molecules by 1.5 °C, compared to refolded and photo-exchanged pMHC-I molecules. All samples were analyzed in duplicate and the error is represented as the standard deviation of the duplicates analyzed independently.

### Bio-Layer Interferometry

In each experiment, HIS1K biosensor tips (ForteBio) were first baselined in a buffer of 20 mM sodium phosphate pH 7.2, 100 mM NaCl and then coated with 6 µg/mL of HIS-Tagged TAPBPR in a matched buffer until the response was between 0.3 nm and 0.4 nm for each tip. TAPBPR coated biosensor tips were then dipped into matched buffer supplemented with 0.02% TWEEN-20 and 0.5 mg/mL BSA for 6 minutes to block non-specific interaction and as secondary baseline step. Subsequent steps were performed in the secondary baseline buffer (20 mM sodium phosphate pH 7.2, 100 mM NaCl, 0.02% TWEEN-20, 0.5% BSA). Biosensor tips were then dipped into buffer containing 10 µM HLA-A*02:01/g-TAX and 10 mM GM dipeptide to facilitate peptide deficient MHC-I/TAPBPR formation for 10 minutes. After peptide deficient MHC-I/TAPBPR formation on the biosensor tips, they were dipped into buffer supplemented with 0-5 µM of the indicated peptides for 14 minutes to facilitate pMHC dissociation from TAPBPR. Data was processed after subtraction of the reference sensor tip data set which was coated with TAPBPR in buffer, Y-axis alignment to the secondary baseline, and an interstep correction alignment to dissociation. All data was locally fit for association and dissociation with a 2:1 (heterogeneous) binding model. Goodness of fit was determined according to R² values of above 0.99. All experiments were performed using an Octet Red 96e system and analyzed with the Octet data analysis HT v.11.1 software.

### Nano Differential Scanning Fluorimetry (nanoDSF)

In a volume of 20 µl, 1 µM peptide deficient HLA-A*02:01/TAPBPR was incubated with 20 µM of various peptides (Figure 22) in a buffer of 20 mM sodium phosphate pH 7.2, 100 mM NaCl, 0.02% TWEEN-20 for at least one hour. 10 ul of each sample was loaded on the Prometheus NT.48 instrument (NanoTemper) using the high sensitivity capillaries. NanoDSF measurements are performed using a temperature ramp rate of 1°C/min from 25 °C to 95 °C and an LED intensity of 20%. Data was analyzed using the PR Control software (NanoTemper). Melting temperatures (Tₘ values) correspond to the inflection points of the first derivative of the 330/350 nm fluorescence ratio. Experiments were performed in duplicates and the shaded areas (Figure 17b) are representative of the error.

### Native gel electrophoresis

Peptide-deficient MHC-I/TAPBPR complexes were incubated with the indicated molar ratio of relevant (TAX) or irrelevant (P18-I10) peptide for 1 h at room temperature. Samples were run at 90 V on 8 % polyacrylamide gels in 25 mM TRIS pH 8.8, 192 mM glycine, at 4ºC for 4.5 hours and developed using InstantBlue (Expedeon).

### Fluorescence anisotropy

Fluorescence anisotropy was performed using TAX peptide labeled with TAMRA dye (K_{TAMRA}LFGYPVYV) (herein called TAMRA-TAX) (Jiang et al., Science 358, 1064-1068 (2017)). Briefly, 50 nM of peptide-deficient HLA-A*02:01/TAPBPR complex in 100 mM NaCl, 20 mM sodium phosphate and 0.05 % (v) tween-20, were incubated with 0.75 nM TAMRA-TAX and graded concentrations of MART-1, CMVpp65 or unlabeled TAX peptide in total volumes of 100 µL in black 96 well assay plate (Costar 3915) for 2 hours at room temperature while Fluorescence anisotropy (r) was recorded. Fluorescence anisotropy (FA) was recorded on a Perkin Elmer Envision 2103 with an excitation filter of λₑₓ = 531 nm and an emission filter of λₑₘ = 595 nm. FA were normalized against TAMRA-TAX alone. Measurements were recorded every 30 seconds and data points represented are an average of FA values acquired following 105 minutes of incubation. All experiments are representative of at least 3 individual experiments run in triplicates. Data points were plotted and fit using a sigmoidal response curve in GraphPad Prism 7.

### Preparation of barcoded peptide exchanged tetramers

Purified, *BirA*-tagged pMHC-I molecules were biotinylated using the *BirA* biotin-protein ligase bulk reaction kit (Avidity), according to the manufacturer's instructions. Biotinylated pMHC-I was buffer exchanged into PBS pH 7.4 using Amicon Ultra centrifugal filter units with the membrane cut-off 10 kDa. The level of biotinylation was evaluated by SDS-PAGE gel-shift assay in the presence of excess streptavidin.

In the "stoichiometric" approach, equimolar quantities of MHC-I loaded with "goldilocks" peptides and TAPBPR were added to streptavidin-PE (Prozyme) (2:1 MHC-I:streptavidin molar ratio) in ten additions every ten minutes at room temperature. For tetramer barcoding, custom biotinylated DNA oligos (IDT) were used. Each tetramer was barcoded by adding 2:1 molar equivalent of DNA-barcodes relative to streptavidin and incubated for 1 hr, at room temperature. Peptide-deficient barcoded-MHC-I/TAPBPR tetramers were then exchanged with peptides of interest by adding a 10-fold molar excess of peptide to each well and incubating overnight at 4°C. Additionally, 10-fold molar excess biotin (to block any free streptavidin sites) was added and incubated for a further 1 h at room temperature. After exchange, tetramers were transferred to Amicon Ultra centrifugal filter units with the membrane cut-off 100 kDa and washed with 1000 volumes of PBS to remove TAPBPR and excess of peptide and barcodes. After washing, exchanged tetramers were pooled and stored at 4 °C for up to 3 weeks. In the "catalytic" approach, the MHC-I loaded with "goldilocks" peptides were mixed with a 1:100 molar ratio of TAPBPR and added to streptavidin-PE (Prozyme) in the same way as described for the "stoichiometric" approach. All the further procedures were exactly the same.

### Cell Culture

58 α⁻β⁻T cells expressing the B4.2.3 TCR, which recognizes P18-I10 bound to H-2D^{d}, were obtained from Dr. Kannan Natarajan (NIH). TCR β-chain deficient Jurkat-MA T cells expressing the DMF5 TCR, which recognizes Melan-A epitope MART-1 bound to HLA-A*02:01 and Jurkat-MA T cells expressing the NY-ESO-1 TCR which recognizes the NY-ESO-1 epitope bound to HLA-A*02:01 were generated as described below. All three lines were grown in DMEM supplemented with 10 % FBS, 25 mM HEPES pH 7, 2 µM β-mercaptoethanol, 2 mM L-glutamine, 100 U/mL penicillin/streptomycin and 1 x non-essential amino acids. Cells were maintained in exponential phase in a humidified incubator at 37 °C with 5 % CO₂. Splenocytes from HLA-A*02:01+ organ donors were obtained through the Human Pancreas Analysis Program (http://hpap.pmacs.upenn.edu/) (University of Pennsylvania) after informed consent by each donor's legal representative.

### Generation of DMF5 and of NY-ESO-1 T cell lines

Retrovirus for transduction of Jurkat/MA (Calogero et al., Anticancer Res 20:1793-1799 (2000)) and primary CD8 T cells was produced using Platinum-A retroviral packaging cell line. DMF5 / NY-ESO-1 cassettes were assembled using previously described CDR3 sequences and V(D)J family genes (Borbulevych et al., J Immunol 187:2453-2463 (2011)), codon optimized, synthesized, and cloned into pMP71 retroviral vector (Engels et al., Hum Gene Ther 14:1155-1168 (2003)). Jurkat/MA cells were plated in 6-well plates at 7 x 10⁵ cells/well and transfected with 2.5 mg of retroviral vector pMP71 using Lipofectamine 3000 (Life Technologies, Invitrogen). After 24 hours, medium was replaced with IMDM-10 % FBS or AIM-V-10 % FBS. Supernatants were harvested and filtered with 0.2 mM filters after 24 hours incubation and transferred to Jurkat/MA cells in 6-well plates pre-treated with 1 mL well/Retronectin (20 mg/mL in PBS, Takara Bio. Inc.,) at 1 x 10⁶ cells/well and spinoculated with 2 mL of retroviral supernatant at 800 g for 30 min at RT. After 24 hours, cells were washed and PBS, and cultured in IMDM-10% FBS. Jurkat/MA cells were stained with MART-1/ NY-ESO-1 dextramers (Immudex), and sorted for dextramer positive cells.

### PBMC/DC co-culture

Normal donor monocytes were plated on day 1 in 6-well plates at 5 x 10⁶/ well in RPMI-10 FBS supplemented with 10 ng/ml IL-4 (Peprotech) and 800 IU/ml GM-CSF (Peprotech) and incubated at 37 °C overnight. On day 2, fresh media supplemented with 10 ng/ml IL-4 and 1600 IU/ml GM-CSF was added to the monocytes and incubated at 37°C for another 48 hours. On day 4, non-adherent cells were removed and immature dendritic cells washed and pulsed with 5 uM peptide in AIM-V-10 % FBS supplemented with 10 ng/ml IL-4, 800 IU/ml GM-CSF, 10 ng/ml LPS (Sigma-Aldrich), and 100 IU/ml IFN-γ (Peprotech) at 37°C overnight. Day 1 was repeated on days 4 and 8 to generate dendritic cells for the second and third stimulations on days 8 and 12, respectively. On day 5, normal donor-matched CD8+ T cells were co-cultured with the pulsed dendritic cells in AIM-V-10 % FBS. Day 5 protocol was repeated on day 8 and day 12 using dendritic cells generated on days 4 and 8 for the second and third stimulation, respectively.

### Flow cytometry

Tetramer analysis was carried out by staining 2 x 10⁵ cells with anti-CD8α mAb (BD Biosciences) and 1 µg/mL of HLA-A02:01/MART-1, HLA-A02:01/NY-ESO-1 or 1µg/mL H-2D^{d}/P18-I10 tetramer for 30 minutes on ice, followed by two washes with 30 volumes of FACS buffer (PBS, 1% BSA, 2 mM EDTA). Live / dead gating determined by staining with propidium iodide. All flow cytometric analysis was performed using a BD LSR II instrument equipped with FACSDiva software (BD Biosciences). For cell sorting experiments, cryopreserved human splenocytes were thawed and rested in RPMI media (10 % FBS, 1 % L-glutamine, 1 % Pencillin/Streptomycin). CD8⁺ T cells were enriched by negative selection using magnetic beads according to the manufacturer's protocol (STEMCELL Technologies). Cells were then treated with dasatinib (50 nM, Sigma-Aldrich) for 30 minutes prior to staining. Afterward, 50 µL each of PE and APC versions of the tetramer library were added (final amount was 0.5 µg pMHC per tetramer) were added for 15 minutes at room temperature. Cells were washed and resuspended in BD pre-sort buffer (BD Biosciences). Cell sorting was performed on a FACS Aria FUSION (BD Biosciences). Live cells were gated based on forward and side scatter profiles and data was analyzed using FlowJo software (FlowJo, LLC). For EC₅₀ determination, tetramer concentrations were calculated based on total amount of pMHC-I at the time of exchange. Titrations were performed on the appropriate cell line in duplicate in two independent experiments. The percentage of tetramer+ T cells was measured relative to the staining achieved at the highest concentration tested within each experiment. EC₅₀ values were calculated by fitting a Boltzmann sigmoidal function to the data GraphPad Prism 7.

### ECCITE-seq

Post sorting, samples were prepped for the 10X Genomics 5P V(D)J kit workflow, and processed according to the ECCITE-seq protocol (Mimitou et al., Nat Methods 16:409-412 (2019)), with these modifications:
1) For cDNA amplification, 1ul of 0.2uM tetramer additive **(GTCTCGTGGGCTCGGAGATG)** was spiked into the reaction.
2) Post cDNA PCR, a 0.6x SPRI cleanup was performed, resulting in the larger cDNA fragments being retained on the beads, and the tetramer tags in the supernatant. After separation of the two fractions and elution from the beads, a portion of the cDNA was used to perform TCR α/β amplification and library prep, as described in the 10X genomics protocol.
3) A separate portion of the cDNA elution was used to perform a DMF5 receptor specific enrichment, using a hemi-nested PCR strategy akin to that used for the TCRα/β enrichment. All PCRs were performed using 2X KAPA Hifi Master Mix. Primers for PCRs: PCR - DMF5_PCR1 (GAAATTCACGGCGCACAGG) with SI-PCR primer (10x). PCR2 - DMF5_PCR2 (CCTTGGCACCCGAGAATTCCAGCTTGGCTGGCTGTCTCTGATC) and P5_generic (AATGATACGGCGACCACCGAGATCTACAC). PCR3 (to add P7 end and sample index) - RPI-x primer ("x" nucleotides comprise a user-defined index) CAAGCAGAAGACGGCATACGAGATxxxxxxxxGTGACTGGAGTTCCTTGGCACCCGAGAATTCCA and P5_generic.
4) The supernatant of the 0.6X SPRI purification in step 2 above was purified with 2 rounds of 2x SPRI. First, 1.4x SPRI was added to the supernatant to bring up the volume to 2x, followed by two rounds of 80% ethanol washes. After eluting in water, an additional 2x SPRI cleanup was performed. Post second cleanup, the tetramer tags were converted to a sequenceable library by PCR with SI-PCR and N7XX ("x" nucleotides comprise a user-defined index) CAAGCAGAAGACGGCATACGAGATxxxxxxxxGTCTCGTGGGCTCGG.

### Sequencing and Analysis

Individual tetramers were pooled in one library sample prior to sequencing. Samples were sequenced on a Miseq using a v2 300 cycle kit (151 cycles R1, 8 cycles I1, 151 cycles read 2). Post sequencing, TCR fastq files were pooled together for each sample, then analyzed using cellranger vdj 3.0.0 against the GRCh38 reference genome (v2.0.0, as provided by the 10X website). To identify the DMF5 receptor, CITE-seq-Count version 1.4.1 was used to search for the DMF5 specific tag, using default parameters (hamming distance set to 5). CITE-seq-Count version 1.4.1 was used for tetramers, using all default parameters, with the exception of hamming distance set to 1, and a whitelist to search for only cells with TCR found by 10X.

### TCR Repertoire Analysis

All analysis was performed using the PE-tetramer barcodes alone. Cells with ≥10 tetramer reads were clustered into pMHC specificity groups based on the tetramer barcode read. Cells with multiple tetramers with >10 reads were clustered based on the most frequent tetramer read (≥ 50 % of total tetramer reads for that T cell). All TCR sequences identified (partial or complete) were used in global VJ usage analysis. In cases where multiple TCRs were read, only TCR sequences with the highest true reads were used (generally representative of ≥ 90 % of TCR reads for that T cell). Known receptors and CDRs were queried and identified using VDJdb and literature searches.

## Claims

1. A method of making a plurality of peptide receptive MHC-I complexes each of which can accept a peptide of interest, each peptide receptive MHC-I complex comprising an MHC class I heavy chain and an β2-microglobulin, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides have the sequence YPLFGWCF or LFGPVYV, under conditions wherein the plurality of MHC class I heavy chains, the β2-microglobulins and the plurality placeholder peptides form a plurality of placeholder peptide-MHC class I (p*MHC-I ) complexes; and
b) contacting the p*MHC-I complexes with a plurality of dipeptides and chaperones, thereby creating the plurality of peptide receptive MHC-I complexes.

2. The method of claim 1, wherein all or substantially all of the MHC class I heavy chains in the plurality of MHC-I heavy chains are HLA-A*24:02 or HLA-A*68:02 and wherein all or substantially all of the placeholder peptides in the plurality placeholder peptides have the sequence YPLFGWCF or LFGPVYV; optionally wherein
(a) at least a subset of the dipeptides are glycyl-methionine or glycyl-phenylalanine;
(b) substantially all of the dipeptides are glycyl-methionine and glycyl-phenylalanine;
(c) all or substantially all of the dipeptides are glycyl methionine or substantially all of the dipeptides are glycyl-phenylalanine;
(d) all or substantially all of the chaperones are TAPBPR;
(e) the amount of chaperone is sufficient to convert at least 95% of the MHC class I molecules to peptide receptive MHC-I complexes; and/or
(f) the molar ratio of chaperone to p*MHC-I is less than 1:2, less than 1:10, less than 1:50, less than 1:100, less than 1:500, or less than 1:1000.

3. The method of claim 1, wherein the molar ratio of chaperone to p*MHC-I is greater than 1:1 or where the molar ratio of chaperone to p*MHC-I is less than 1:1.

4. A method of making a plurality of peptide-MHC class I (pMHC-I) complexes, each complex comprising an MHC Class I heavy chain, a β2-microglobulin, and a peptide of interest, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides having the sequence YPLFGWCF or LFGPVYV, under conditions wherein the plurality of MHC class I heavy chains, the plurality β2-microglobulins and the plurality placeholder peptides form a plurality of placeholder peptide-MHC class I (p*MHC-I ) complexes;
b) forming a plurality of peptide receptive MHC-I complexes by contacting the plurality of p*MHC-I complexes with a plurality of dipeptides and chaperones; and
c) contacting the plurality of peptide receptive MHC-I complexes with a plurality of peptides of interest, thereby forming the plurality of pMHC-I complexes.

5. The method of claim 4, wherein all or substantially all of the MHC class I heavy chains in the plurality of MHC-I heavy chains are HLA-A*24:02 or HLA-A*68:02 and wherein all or substantially all of the placeholder peptides in the plurality placeholder peptides have the sequence YPLFGWCF or LFGPVYV; optionally wherein
(a) at least a subset of the dipeptides are glycyl-methionine or glycyl-phenylalanine;
(b) substantially all of the dipeptides are glycyl-methionine and glycyl-phenylalanine;
(c) all or substantially all of the dipeptides are glycyl methionine or substantially all of the dipeptides are glycyl-phenylalanine;
(d) all or substantially all of the chaperones are TAPBPR;
(e) the amount of chaperone is sufficient to convert at least 95% of the MHC class I molecules to peptide receptive MHC-I complexes; and/or
(f) the molar ratio of chaperone to p*MHC-I is less than 1:2, less than 1:10, less than 1:50, less than 1:100, less than 1:500, or less than 1:1000.

6. The method of claim 4, wherein
(a) the molar ratio of chaperone to p*MHC-I is greater than 1:1 or the molar ratio of chaperone to p*MHC-I is less than 1:1;
(b) all the peptides of interest in the plurality of the peptides of interest have the same sequence or at least two of the peptides of interest in the plurality of peptides of interest have different sequences; and/or
(c) the plurality of peptides of interest comprise tumor antigen peptides, viral derived peptides, bacterially derived peptides, or self-antigen derived peptides.

7. A method of making a plurality of peptide-MHC class I (pMHC-I) multimer complexes, each complex comprising an MHC class I multimer and a peptide of interest, the method comprising:
a) incubating a plurality of MHC class I heavy chains comprising one of HLA-A*24:02 and HLA-A*68:02; a plurality of β2-microglobulins; and a plurality of placeholder peptides comprising placeholder peptides having the sequence YPLFGWCF or LFGPVYV, under conditions wherein the plurality of MHC class I heavy chains, the β2-microglobulins and the plurality of placeholder peptides form a plurality of placeholder peptide-MHC class I (p*MHC-I ) complexes;
b) contacting the plurality of p*MHC-I complexes with a plurality of dipeptides and chaperones, thereby forming a plurality of peptide receptive MHC-I complexes;
c) attaching the plurality of peptide receptive MHC-I complexes to multimer backbones, thereby forming a plurality of peptide receptive MHC-I multimers; and
d) contacting the plurality of peptide receptive MHC- I multimers with a plurality of peptides of interest, thereby forming a plurality of pMHC-I multimer complexes.

8. A method of making a plurality of peptide-MHC class I (pMHC-I) multimer complexes, each complex comprising an MHC class I multimer and a peptide of interest, the method comprising:
a) providing a plurality of placeholder peptide-MHC class I (p*MHC-I) complexes, wherein at least a subset of the plurality of p*MHC-I complex comprises an MHC class I heavy chain comprising one of HLA-A*24:02 and HLA-A*68:02, an β2-microglobulin, and a placeholder peptides have the sequence YPLFGWCF or LFGPVYV;
b) contacting the plurality of p*MHC-I complexes with a plurality of chaperones, dipeptides, multimer backbones, and peptides of interest under conditions to form a plurality of peptide-MHC class I multimer complexes, thereby forming a plurality of peptide-MHC class I multimer complexes; and
c) recovering the plurality of peptide-MHC class I multimer complexes.

9. The method of claim 7, wherein all or substantially all of the MHC class I heavy chains in the p*MHC-I complexes are HLA-A*24:02 or HLA-A*68:02 and wherein all or substantially all of the placeholder peptides in the p*MHC-I complexes have the sequence YPLFGWCF or LFGPVYV.

10. The method of any one of claims 7-9, wherein
(a) at least a subset of the dipeptides are glycyl-methionine or glycyl-phenylalanine;
(b) substantially all of the dipeptides are glycyl-methionine and glycyl-phenylalanine;
(c) all or substantially all of the dipeptides are glycyl-methionine or substantially all of the dipeptides are glycyl-phenylalanine;
(d) all or substantially all of the chaperones are TAPBPR;
(e) the amount of chaperone is sufficient to convert at least 95% of the MHC class I molecules to peptide receptive MHC-I complexes; and/or
(f) the molar ratio of chaperone to p*MHC-I is less than 1:2, less than 1:10, less than 1:50, less than 1:100, less than 1:500, or less than 1:1000.

11. The method of claim 7 or 8, wherein the molar ratio of chaperone to p*MHC-I is greater than 1:1 or wherein the molar ratio of chaperone to p*MHC-I is less than 1:1.

12. The method of claim 7, wherein all the peptides of interest in the plurality of the peptides of interest have the same sequence or wherein at least two of the peptides of interest in the plurality of peptides of interest have different sequences, and optionally wherein the plurality of peptides of interest comprise tumor antigen peptides, viral derived peptides, bacterially derived peptides, or self-antigen derived peptides.

13. The method of any one of claims 7 to 12, wherein
(a) each of the MHC multimer-peptide complexes is further attached to a barcode DNA oligo;
(b) the multimer backbones are selected from streptavidin, avidin and dextran backbones;
(c) the peptide-MHC class I multimers are tetramers; and/or
(d) the p*MHC-I complexes are biotinylated, or further comprising removing free chaperones after the chaperones convert the p*MHC-I complexes to activated pMHC-I complexes and optionally removing the free chaperones by spin column dialysis.

14. A composition comprising:
a) a plurality of MHC class I heavy chains comprising one of HLA-A*24:02 and HLA-A*68:02;
b) a plurality of β2-microglobulins;
c) a plurality of placeholder peptides comprising placeholder peptides have the sequence YPLFGWCF or LFGPVYV; and
d) a plurality of chaperones.

15. The composition of claim 14, wherein all or substantially all of the MHC class I heavy chains in the plurality of MHC-I heavy chains are HLA-A*24:02 or HLA-A*68:02 and where all or substantially all of the placeholder peptides in the plurality of placeholder peptides have the sequence YPLFGWCF or LFGPVYV, or wherein the molar ratio of MHC class I heavy chains to β2-microglobulins is less than 1:3; optionally wherein all or substantially all of chaperones are TAPBPR.

16. A composition comprising a plurality of peptide receptive MHC-I complexes configured to accept a peptide of interest, wherein at least a subset of the plurality of peptide receptive MHC-I complexes comprises:
a) a plurality of MHC class I heavy chains comprising one of HLA-A*24:02 and HLA-A*68:02;
b) a plurality of β2-microglobulins; and
c) a plurality of placeholder peptides comprising placeholder peptides have the sequence YPLFGWCF or LFGPVYV.

17. The composition of claim 16, wherein the plurality of peptide receptive MHC-I complexes were contacted with a chaperone.

18. The composition of claim 17, wherein the composition was subjected to a process that removes free chaperones.

19. The composition of any one of claims 16-18, wherein all or substantially all of the MHC class I heavy chains in the plurality of MHC-I heavy chains are HLA-A*24:02 or HLA-A*68:02 and wherein all or substantially all of the placeholder peptides have the sequence YPLFGWCF or LFGPVYV; optionally wherein the composition is at least 90%, at least 95%, at least 98%, at least 99%, at least 99.9% or 100% free chaperones.

20. The composition of claim 18, wherein the process comprises dialysis, optionally wherein the process comprises spin column dialysis.

## Patentansprüche

1. Ein Verfahren zur Herstellung einer Vielzahl von Peptid-aufnehmenden MHC-I-Komplexen, von denen jedes ein Peptid von Interesse aufnehmen kann, wobei jeder Peptidaufnehmende MHC-I-Komplex eine schwere Kette von MHC Klasse I und ein β2-Mikroglobulin umfasst, das Verfahren umfassend:
a) Inkubieren einer Vielzahl von schweren Ketten von MHC Klasse I umfassend eines aus HLA-A*24:02 und HLA-A*68:02; einer Vielzahl von β2-Mikroglobulinen; und einer Vielzahl von Platzhalterpeptiden umfassend Platzhalterpeptide mit der Sequenz YPLFGWCF oder LFGPVYV, unter Bedingungen, unter denen die Vielzahl von schweren Ketten von MHC Klasse I, die β2-Mikroglobuline und die Vielzahl von Platzhalterpeptiden eine Vielzahl von Platzhalterpeptid-MHC Klasse I- (p*MHC-I-) Komplexen bilden; und
b) Kontaktieren der p*MHC-I-Komplexe mit einer Vielzahl von Dipeptiden und Chaperonen, wodurch die Vielzahl von Peptid-aufnehmenden MHC-I-Komplexen erzeugt wird.

2. Das Verfahren aus Anspruch 1, wobei alle oder im Wesentlichen alle der schweren Ketten von MHC Klasse I in der Vielzahl von schweren Ketten von MHC-I HLA-A*24:02 oder HLA-A*68:02 sind und wobei alle oder im Wesentlichen alle der Platzhalterpeptide in der Vielzahl von Platzhalterpeptiden die Sequenz YPLFGWCF oder LFGPVYV haben; optional wobei
(a) mindestens eine Teilmenge der Dipeptide Glycyl-Methionin oder Glycyl-Phenylalanin sind;
(b) im Wesentlichen alle der Dipeptide Glycyl-Methionin und Glycyl-Phenylalanin sind;
(c) alle oder im Wesentlichen alle der Dipeptide Glycyl-Methionin sind oder im Wesentlichen alle der Dipeptide Glycyl-Phenylalanin sind;
(d) alle oder im Wesentlichen alle der Chaperone TAPBPR sind;
(e) die Menge an Chaperon ausreichend ist, um mindestens 95% der MHC Klasse I-Moleküle zu Peptid-aufnehmenden MHC-I-Komplexen umzuwandeln; und/oder
(f) das molare Verhältnis von Chaperon zu p*MHC-I geringer als 1:2, geringer als 1:10, geringer als 1:50, geringer als 1:100, geringer als 1:500 oder geringer als 1:1000 ist.

3. Das Verfahren aus Anspruch 1, wobei das molare Verhältnis von Chaperon zu p*MHC-I größer als 1:1 ist oder wobei das molare Verhältnis von Chaperon zu p*MHC-I geringer als 1:1 ist.

4. Ein Verfahren zur Herstellung einer Vielzahl von Peptid-MHC Klasse I- (pMHC-I-) Komplexen, jeder Komplex umfassend eine schwere Kette von MHC Klasse I, ein β2-Mikroglobulin und ein Peptid von Interesse, das Verfahren umfassend:
a) Inkubieren einer Vielzahl von schweren Ketten von MHC Klasse I umfassend eines aus HLA-A*24:02 und HLA-A*68:02; einer Vielzahl von β2-Mikroglobulinen; und einer Vielzahl von Platzhalterpeptiden umfassend Platzhalterpeptide mit der Sequenz YPLFGWC oder LFGPVYV, unter Bedingungen, unter denen die Vielzahl von schweren Ketten von MHC Klasse I, die β2-Mikroglobuline und die Vielzahl von Platzhalterpeptiden eine Vielzahl von Platzhalterpeptid-MHC Klasse I- (p*MHC-I-) Komplexen bilden;
b) Bilden einer Vielzahl von Peptid-aufnehmenden MHC-I-Komplexen durch Kontaktieren der Vielzahl von p*MHC-I-Komplexen mit einer Vielzahl von Dipeptiden und Chaperonen; und
c) Kontaktieren der Vielzahl von Peptid-aufnehmenden MHC-I-Komplexen mit einer Vielzahl von Peptiden von Interesse, wodurch die Vielzahl von pMHC-I-Komplexen gebildet wird.

5. Das Verfahren aus Anspruch 4, wobei alle oder im Wesentlichen alle der schweren Ketten von MHC Klasse I in der Vielzahl von schweren Ketten von MHC-I HLA-A*24:02 oder HLA-A*68:02 sind und wobei alle oder im Wesentlichen alle der Platzhalterpeptide in der Vielzahl von Platzhalterpeptiden die Sequenz YPLFGWCF oder LFGPVYV haben; optional wobei
(a) mindestens eine Teilmenge der Dipeptide Glycyl-Methionin oder Glycyl-Phenylalanin sind;
(b) im Wesentlichen alle der Dipeptide Glycyl-Methionin und Glycyl-Phenylalanin sind;
(c) alle oder im Wesentlichen alle der Dipeptide Glycyl-Methionin sind oder im Wesentlichen alle der Dipeptide Glycyl-Phenylalanin sind;
(d) alle oder im Wesentlichen alle der Chaperone TAPBPR sind;
(e) die Menge an Chaperon ausreichend ist, um mindestens 95% der MHC Klasse I-Moleküle zu Peptid-aufnehmenden MHC-I-Komplexen umzuwandeln; und/oder
(f) das molare Verhältnis von Chaperon zu p*MHC-I geringer als 1:2, geringer als 1:10, geringer als 1:50, geringer als 1:100, geringer als 1:500 oder geringer als 1:1000 ist.

6. Das Verfahren aus Anspruch 4, wobei
(a) das molare Verhältnis von Chaperon zu p*MHC-I größer als 1:1 ist oder das molare Verhältnis von Chaperon zu p*MHC-I geringer als 1:1 ist;
(b) alle Peptide von Interesse in der Vielzahl von Peptiden von Interesse dieselbe Sequenz haben oder mindestens zwei der Peptide von Interesse in der Vielzahl von Peptiden von Interesse zwei verschiedene Sequenzen haben; und/oder
(c) die Vielzahl von Peptiden von Interesse Tumorantigenpeptide, Virenabgeleitete Peptide, Bakterien-abgeleitete Peptide oder Selbstantigen-abgeleitete Peptide umfassen.

7. Ein Verfahren zur Herstellung einer Vielzahl von Peptid-MHC Klasse I- (pMHC-I-) Multimerkomplexen, jeder Komplex umfassend ein MHC Klasse I-Multimer und ein Peptid von Interesse, das Verfahren umfassend:
a) Inkubieren einer Vielzahl von schweren Ketten von MHC Klasse I umfassend eines aus HLA-A*24:02 und HLA-A*68:02; einer Vielzahl von β2-Mikroglobulinen; und einer Vielzahl von Platzhalterpeptiden umfassend Platzhalterpeptide mit der Sequenz YPLFGWCF oder LFGPVYV, unter Bedingungen, unter denen die Vielzahl von schweren Ketten von MHC Klasse I, die β2-Mikroglobuline und die Vielzahl von Platzhalterpeptiden eine Vielzahl von Platzhalterpeptid-MHC Klasse I- (p*MHC-I-) Komplexen bilden;
b) Kontaktieren der Vielzahl von p*MHC-I-Komplexen mit einer Vielzahl von Dipeptiden und Chaperonen, wodurch eine Vielzahl von Peptid-aufnehmenden MHC-I-Komplexen erzeugt wird;
c) Verbinden der Vielzahl von Peptid-aufnehmenden MHC-I-Komplexen mit Multimer-Rückgraten, wobei eine Vielzahl von Peptid-aufnehmenden MHC-I-Multimeren gebildet wird; und
d) Kontaktieren der Vielzahl von Peptid-aufnehmenden MHC-I-Multimeren mit einer Vielzahl von Peptiden von Interesse, wodurch eine Vielzahl von pMHC-I-Multimerkomplexen gebildet wird.

8. Ein Verfahren zur Herstellung einer Vielzahl von Peptid-MHC Klasse I- (pMHC-I-) Multimerkomplexen, jeder Komplex umfassend ein MHC Klasse I-Multimer und ein Peptid von Interesse, das Verfahren umfassend:
a) Bereitstellen einer Vielzahl von Platzhalterpeptid-MHC Klasse I- (p*MHC-I-) Komplexen, wobei mindestens eine Teilmenge der Vielzahl von p*MHC-I-Komplexen eine schwere Kette von MHC Klasse I umfassend eines von HLA-A*24:02 und HLA-A*68:02, ein β2-Mikroglobulin und ein Platzhalterpeptid mit der Sequenz YPLFGWCF oder LFGPVYV umfasst;
b) Kontaktieren der Vielzahl von p*MHC-I-Komplexen mit einer Vielzahl von Chaperonen, Dipeptiden, Mutimer-Rückgraten und Peptiden von Interesse unter Bedingungen, um eine Vielzahl von Peptid-MHC Klasse I-Multimerkomplexen zu bilden, wodurch eine Vielzahl von Peptid-MHC Klasse I-Multimerkomplexen gebildet wird; und
c) Gewinnen der Vielzahl von Peptid-MHC Klasse I-Multimerkomplexen.

9. Das Verfahren aus Anspruch 7, wobei alle oder im Wesentlichen alle der schweren Ketten von MHC Klasse I in den p*MHC-I-Komplexen HLA-A*24:02 oder HLA-A*68:02 sind und wobei alle oder im Wesentlichen alle der Platzhalterpeptide in den p*MHC-I-Komplexen die Sequenz YPLFGWCF oder LFGPVYV haben.

10. Das Verfahren aus irgendeinem der Ansprüche 7-9, wobei
(a) mindestens eine Teilmenge der Dipeptide Glycyl-Methionin oder Glycyl-Phenylalanin sind;
(b) im Wesentlichen alle der Dipeptide Glycyl-Methionin und Glycyl-Phenylalanin sind;
(c) alle oder im Wesentlichen alle der Dipeptide Glycyl-Methionin sind oder im Wesentlichen alle der Dipeptide Glycyl-Phenylalanin sind;
(d) alle oder im Wesentlichen alle der Chaperone TAPBPR sind;
(e) die Menge an Chaperon ausreichend ist, um mindestens 95% der MHC Klasse I-Moleküle zu Peptid-aufnehmenden MHC-I-Komplexen umzuwandeln; und/oder
(f) das molare Verhältnis von Chaperon zu p*MHC-I geringer als 1:2, geringer als 1:10, geringer als 1:50, geringer als 1:100, geringer als 1:500 oder geringer als 1:1000 ist.

11. Das Verfahren aus Anspruch 7 oder 8, wobei das molare Verhältnis von Chaperon zu p*MHC-I größer als 1:1 ist oder wobei das molare Verhältnis von Chaperon zu p*MHC-I geringer als 1:1 ist.

12. Das Verfahren aus Anspruch 7, wobei alle Peptide von Interesse in der Vielzahl von Peptiden von Interesse dieselbe Sequenz haben oder mindestens zwei der Peptide von Interesse in der Vielzahl von Peptiden von Interesse zwei verschiedene Sequenzen haben, und optional wobei die Vielzahl von Peptiden von Interesse Tumorantigenpeptide, Virenabgeleitete Peptide, Bakterien-abgeleitete Peptide oder Selbstantigen-abgeleitete Peptide umfassen.

13. Das Verfahren aus irgendeinem der Ansprüche 7 bis 12, wobei
(a) jedes der MHC-Multimer-Peptid-Komplexe weiter mit einem Strichcode-DNA-Oligo verbunden ist;
(b) die Multimer-Rückgrate ausgewählt sind aus Streptavidin-, Avidin- und Dextran-Rückgraten;
(c) die Peptid-MHC Klasse I-Multimere Tetramere sind; und/oder
(d) die p*MHC-I-Komplexe biotinyliert sind, oder weiter umfassend Entfernen freier Chaperone, nachdem die Chaperone die p*MHC-I-Komplexe zu aktivierten pMHC-I-Komplexen umwandeln, und optional Entfernen der freien Chaperone durch Drehsäulendialyse.

14. Eine Zusammensetzung umfassend:
a) eine Vielzahl von schweren Ketten von MHC Klasse I umfassend eines von HLA-A*24:02 und HLA-A*68:02;
b) eine Vielzahl von β2-Mikroglobulinen;
c) eine Vielzahl von Platzhalterpeptiden umfassend Platzhalterpeptide mit der Sequenz YPLFGWCF oder LFGPVYV; und
d) eine Vielzahl von Chaperonen.

15. Die Zusammensetzung aus Anspruch 14, wobei alle oder im Wesentlichen alle der schweren Ketten von MHC Klasse I in der Vielzahl von schweren Ketten von MHC-I HLA-A*24:02 oder HLA-A*68:02 sind und wobei alle oder im Wesentlichen alle der Platzhalterpeptide in der Vielzahl von Platzhalterpeptiden die Sequenz YPLFGWCF oder LFGPVYV haben, oder wobei das molare Verhältnis von schweren Ketten von MHC Klasse I zu β2-Mikroglobulinen geringer als 1:3 ist; optional wobei alle oder im Wesentlichen alle der Chaperone TAPBPR sind.

16. Eine Zusammensetzung umfassend eine Vielzahl von Peptid-aufnehmenden MHC-I-Komplexen, die konfiguriert sind, ein Peptid von Interesse aufzunehmen, wobei mindestens eine Teilmenge der Peptid-aufnehmenden MHC-I-Komplexe Folgendes umfasst:
a) eine Vielzahl von schweren Ketten von MHC Klasse I umfassend eines von HLA-A*24:02 und HLA-A*68:02;
b) eine Vielzahl von β2-Mikroglobulinen; und
c) eine Vielzahl von Platzhalterpeptiden umfassend Platzhalterpeptide mit der Sequenz YPLFGWCF oder LFGPVYV.

17. Die Zusammensetzung aus Anspruch 16, wobei die Vielzahl von Peptid-aufnehmenden MHC-I-Komplexen mit einem Chaperon kontaktiert wurden.

18. Die Zusammensetzung aus Anspruch 17, wobei die Zusammensetzung einem Prozess unterzogen wurde, der freie Chaperone entfernt.

19. Die Zusammensetzung aus irgendeinem der Ansprüche 16-18, wobei alle oder im Wesentlichen alle der schweren Ketten von MHC Klasse I in der Vielzahl von schweren Ketten von MHC-I HLA-A*24:02 oder HLA-A*68:02 sind und wobei alle oder im Wesentlichen alle der Platzhalterpeptide in der Vielzahl von Platzhalterpeptiden die Sequenz YPLFGWCF oder LFGPVYV haben; optional wobei die Zusammensetzung mindestens 90%, mindestens 95%, mindestens 98%, mindestens 99%, mindestens 99,9% oder 100% freie Chaperone ist.

20. Die Zusammensetzung aus Anspruch 18, wobei der Prozess Dialyse umfasst, optional wobei der Prozess Drehsäulendialyse umfasst.

## Revendications

1. Procédé de fabrication d'une pluralité de complexes MHC-I récepteurs de peptide chacun pouvant accepter un peptide d'intérêt, chaque complexe MHC-I récepteur de peptide comprenant une chaîne lourde de MHC de classe I et une β2-microglobuline, le procédé comprenant :
a) l'incubation d'une pluralité de chaînes lourdes de MHC de classe I comprenant l'un parmi HLA-A*24:02 et HLA-A*68:02 ; d'une pluralité de β2-microglobulines ; et d'une pluralité de peptides substituants comprenant des peptides substituants ayant la séquence YPLFGWCF ou LFGPVYV, dans des conditions où la pluralité de chaînes lourdes de MHC de classe I, les β2-microglobulines et la pluralité de peptides substituants forment une pluralité de complexes peptide substituant-MHC de classe I (p*MHC-I) ; et
b) la mise en contact des complexes p*MHC-I avec une pluralité de dipeptides et de chaperonnes, créant ainsi la pluralité de complexes MHC-I récepteurs de peptide.

2. Procédé selon la revendication 1, dans lequel toutes ou sensiblement toutes les chaînes lourdes de MHC de classe I de la pluralité de chaînes lourdes de MHC-I sont HLA-A*24:02 ou HLA-A*68:02 et dans lequel tous ou sensiblement tous les peptides substituants dans la pluralité de peptides substituants ont la séquence YPLFGWCF ou LFGPVYV ; facultativement dans lequel
(a) au moins un sous-ensemble des dipeptides est constitué de glycyl-méthionine ou de glycyl-phénylalanine ;
(b) sensiblement tous les dipeptides sont une glycyl-méthionine et une glycyl-phénylalanine ;
(c) tous ou sensiblement tous les dipeptides sont une glycyl-méthionine ou sensiblement tous les dipeptides sont une glycyl-phénylalanine ;
(d) toutes ou sensiblement toutes les chaperonnes sont TAPBPR ;
(e) la quantité de chaperonne est suffisante pour convertir au moins 95 % des molécules de MHC de classe I en complexes MHC-I récepteurs de peptide ; et/ou
(f) le rapport molaire entre chaperonne et p*MHC-I est inférieur à 1:2, inférieur à 1:10, inférieur à 1:50, inférieur à 1:100, inférieur à 1:500, ou à inférieur à 1:1000.

3. Procédé selon la revendication 1, dans lequel le rapport molaire entre chaperonne et p*MHC-I est supérieur à 1:1 ou dans lequel le rapport molaire entre chaperonne et p*MHC-I est inférieur à 1:1.

4. Procédé de fabrication d'une pluralité de complexes peptide-MHC de classe I (pMHC-I), chaque complexe comprenant une chaîne lourde de MHC de classe I, une β2-microglobuline et un peptide d'intérêt, le procédé comprenant :
a) l'incubation d'une pluralité de chaînes lourdes de MHC de classe I comprenant l'un parmi HLA-A*24:02 et HLA-A*68:02 ; d'une pluralité de β2-microglobulines ; et d'une pluralité de peptides substituants comprenant des peptides substituants ayant la séquence YPLFGWCF ou LFGPVYV, dans des conditions où la pluralité de chaînes lourdes de MHC de classe I, la pluralité de β2-microglobulines et la pluralité de peptides substituants forment une pluralité de complexes peptide substituant-MHC de classe I (p*MHC-I) ;
b) la formation d'une pluralité de complexes MHC-I récepteurs de peptide en mettant en contact la pluralité de complexes p*MHC-I avec une pluralité de dipeptides et de chaperonnes ; et
c) la mise en contact de la pluralité de complexes MHC-I récepteurs de peptide avec une pluralité de peptides d'intérêt, formant ainsi une pluralité de complexes pMHC-I.

5. Procédé selon la revendication 4, dans lequel toutes ou sensiblement toutes les chaînes lourdes de MHC de classe I de la pluralité de chaînes lourdes de MHC-I sont HLA-A*24:02 ou HLA-A*68:02 et dans lequel tous ou sensiblement tous les peptides substituants dans la pluralité de peptides substituants ont la séquence YPLFGWCF ou LFGPVYV ; facultativement dans lequel
(a) au moins un sous-ensemble des dipeptides est constitué de glycyl-méthionine ou de glycyl-phénylalanine ;
(b) sensiblement tous les dipeptides sont une glycyl-méthionine et une glycyl-phénylalanine ;
(c) tous ou sensiblement tous les dipeptides sont une glycyl-méthionine ou sensiblement tous les dipeptides sont une glycyl-phénylalanine ;
(d) toutes ou sensiblement toutes les chaperonnes sont TAPBPR ;
(e) la quantité de chaperonne est suffisante pour convertir au moins 95 % des molécules de MHC de classe I en complexes MHC-I récepteurs de peptide ; et/ou
(f) le rapport molaire entre chaperonne et p*MHC-I est inférieur à 1:2, inférieur à 1:10, inférieur à 1:50, inférieur à 1:100, inférieur à 1:500, ou à inférieur à 1:1000.

6. Procédé selon la revendication 4, dans lequel
(a) le rapport molaire entre chaperonne et p*MHC-I est supérieur à 1:1 ou le rapport molaire entre chaperonne et p*MHC-I est inférieur à 1:1 ;
(b) tous les peptides d'intérêt de la pluralité des peptides d'intérêt ont la même séquence ou au moins deux des peptides d'intérêt dans la pluralité de peptides d'intérêt ont des séquences différentes ; et/ou
(c) la pluralité de peptides d'intérêt comprend des peptides d'antigène tumoral, des peptides dérivés de virus, des peptides dérivés de bactéries ou des peptides dérivés d'auto-antigène.

7. Procédé de fabrication d'une pluralité de complexes peptide-multimère MHC de classe I (pMHC-I), chaque complexe comprenant un multimère MHC de classe I et un peptide d'intérêt, le procédé comprenant :
a) l'incubation d'une pluralité de chaînes lourdes de MHC de classe I comprenant l'un parmi HLA-A*24:02 et HLA-A*68:02 ; d'une pluralité de β2-microglobulines ; et d'une pluralité de peptides substituants comprenant des peptides substituants ayant la séquence YPLFGWCF ou LFGPVYV, dans des conditions où la pluralité de chaînes lourdes de MHC de classe I, les β2-microglobulines et la pluralité de peptides substituants forment une pluralité de complexes peptide substituant-MHC de classe I (p*MHC-I) ;
b) la mise en contact de la pluralité de complexes p*MHC-I avec une pluralité de dipeptides et de chaperonnes, formant ainsi une pluralité de complexes MHC-I récepteurs de peptide ;
c) la fixation de la pluralité de complexes MHC-I récepteurs de peptide à des squelettes de multimère, formant ainsi une pluralité de multimères MHC-I récepteurs de peptide ; et
d) la mise en contact de la pluralité de multimères MHC-I récepteurs de peptide avec une pluralité de peptides d'intérêt, formant ainsi une pluralité de complexes multimère pMHC-I.

8. Procédé de fabrication d'une pluralité de complexes peptide-multimère MHC de classe I (pMHC-I), chaque complexe comprenant un multimère MHC de classe I et un peptide d'intérêt, le procédé comprenant :
a) la fourniture d'une pluralité de complexes peptide substituant-MHC de classe I (p*MHC-I), dans lequel au moins un sous-ensemble de la pluralité de complexes p*MHC-I comprend une chaîne lourde de MHC de classe I comprenant l'un parmi HLA-A*24:02 et HLA-A*68:02, une microglobuline β2, et un peptide substituant ayant la séquence YPLFGWCF ou LFGPVYV ;
b) la mise en contact de la pluralité de complexes p*MHC-I avec une pluralité de chaperonnes, de dipeptides, de squelettes de multimère et de peptides d'intérêt dans des conditions pour former une pluralité de complexes peptide-multimère MHC de classe I, formant ainsi une pluralité de complexes peptide-multimère MHC de classe I ; et
c) la récupération de la pluralité de complexes peptide-multimère MHC de classe I.

9. Procédé selon la revendication 7, dans lequel toutes ou sensiblement toutes les chaînes lourdes de MHC de classe I dans les complexes p*MHC-I sont HLA-A*24:02 ou HLA-A*68:02 et dans lequel tous ou sensiblement tous les peptides substituants dans les complexes p*MHC-I ont la séquence YPLFGWCF ou LFGPVYV.

10. Procédé selon l'une quelconque des revendications 7 à 9, dans lequel
(a) au moins un sous-ensemble des dipeptides est constitué de glycyl-méthionine ou de glycyl-phénylalanine ;
(b) sensiblement tous les dipeptides sont une glycyl-méthionine et une glycyl-phénylalanine ;
(c) tous ou sensiblement tous les dipeptides sont une glycyl-méthionine ou sensiblement tous les dipeptides sont une glycyl-phénylalanine ;
(d) toutes ou sensiblement toutes les chaperonnes sont TAPBPR ;
(e) la quantité de chaperonne est suffisante pour convertir au moins 95 % des molécules de MHC de classe I en complexes MHC-I récepteurs de peptide ; et/ou
(f) le rapport molaire entre chaperonne et p*MHC-I est inférieur à 1:2, inférieur à 1:10, inférieur à 1:50, inférieur à 1:100, inférieur à 1:500, ou à inférieur à 1:1000.

11. Procédé selon la revendication 7 ou 8, dans lequel le rapport molaire entre chaperonne et p*MHC-I est supérieur à 1:1 ou dans lequel le rapport molaire entre chaperonne et p*MHC-I est inférieur à 1:1.

12. Procédé selon la revendication 7, dans lequel tous les peptides d'intérêt de la pluralité des peptides d'intérêt ont la même séquence ou dans lequel au moins deux des peptides d'intérêt de la pluralité de peptides d'intérêt ont des séquences différentes, et, de manière facultative, dans lequel la pluralité de peptides d'intérêt comprend des peptides d'antigène tumoral, des peptides dérivés de virus, des peptides dérivés de bactéries ou des peptides dérivés d'auto-antigène.

13. Procédé selon l'une quelconque des revendications 7 à 12, dans lequel
(a) chacun des complexes multimère MHC-peptide est en outre attaché à un code-barres oligo-ADN ;
(b) les squelettes de multimère sont choisis parmi des squelettes de streptavidine, avidine et dextrane ;
(c) les peptide-multimères MHC de classe I sont des tétramères ; et/ou
(d) les complexes p*MHC-I sont biotinylés, ou comprenant en outre l'élimination de chaperonnes libres après que les chaperonnes ont converti les complexes p*MHC-I en complexes pMHC-I activés et facultativement l'élimination des chaperonnes libres par dialyse sur colonne de centrifugation.

14. Composition comprenant :
a) une pluralité de chaînes lourdes de MHC de classe I comprenant l'un parmi HLA-A*24:02 et HLA-A*68:02 ;
b) une pluralité de β2-microglobulines ;
c) une pluralité de peptides substituants comprenant des peptides substituants ayant la séquence YPLFGWCF ou LFGPVYV ; et
d) une pluralité de chaperonnes.

15. Composition selon la revendication 14, dans laquelle toutes ou sensiblement toutes les chaînes lourdes de MHC de classe I de la pluralité de chaînes lourdes de MHC-I sont HLA-A*24:02 ou HLA-A*68:02 et dans laquelle tous ou sensiblement tous les peptides substituants de la pluralité de peptides substituants ont la séquence YPLFGWCF ou LFGPVYV, ou dans laquelle le rapport molaire entre des chaînes lourdes de MHC de classe I et des β2-microglobulines est inférieur à 1:3 ; facultativement dans laquelle toutes ou sensiblement toutes les chaperonnes sont TAPBPR.

16. Composition comprenant une pluralité de complexes MHC-I récepteurs de peptide configurés pour accepter un peptide d'intérêt, dans laquelle au moins un sous-ensemble de la pluralité de complexes MHC-I récepteurs de peptide comprend :
a) une pluralité de chaînes lourdes de MHC de classe I comprenant l'un parmi HLA-A*24:02 et HLA-A*68:02 ;
b) une pluralité de β2-microglobulines ; et
c) une pluralité de peptides substituants comprenant des peptides substituants ayant la séquence YPLFGWCF ou LFGPVYV.

17. Composition selon la revendication 16, dans laquelle la pluralité de complexes MHC-I récepteurs de peptide ont été mis en contact avec une chaperonne.

18. Composition selon la revendication 17, dans laquelle la composition a été soumise à un processus qui élimine des chaperonnes libres.

19. Composition selon l'une quelconque des revendications 16 à 18, dans laquelle toutes ou sensiblement toutes les chaînes lourdes de MHC de classe I de la pluralité de chaînes lourdes de MHC-I sont HLA-A*24:02 ou HLA-A*68:02 et dans laquelle tous ou sensiblement tous les peptides substituants ont la séquence YPLFGWCF ou LFGPVYV ; facultativement dans laquelle la composition est constituée d'au moins 90 %, d'au moins 95 %, d'au moins 98 %, d'au moins 99 %, d'au moins 99,9 % ou de 100 % de chaperonnes libres.

20. Composition selon la revendication 18, dans laquelle le processus comprend une dialyse, facultativement dans laquelle le processus comprend une dialyse sur colonne de centrifugation.
